# EUROPEAN PATENT APPLICATION

(11) **EP 4 714 976 A1**
(43) Date of publication of application: **25.03.2026**
(21) Application number: 24810349.1
(22) Date of filing: 20.05.2024
(51) Int. Cl.: C07K 16/28, C07K 16/30, C12N 15/09, A61K 39/00, A61K 39/395, A61P 35/00

(54) **ANTI-FGFR2 ANTIBODY AND USE THEREOF**

(30) Priority: 19.05.2023 CN 202310568415; 27.12.2023 CN 202311816681
(71) Applicant: Sunshine Lake Pharma Co., Ltd., Dongguan, Guangdong 523000 (CN)
(72) Inventor: LI, Xiang, Dongguan, Guangdong 523871 (CN); ZHANG, Hui, Dongguan, Guangdong 523871 (CN); REN, Zhiheng, Dongguan, Guangdong 523871 (CN); ZHOU, Linjun, Dongguan, Guangdong 523871 (CN); PENG, Ju, Dongguan, Guangdong 523871 (CN); LIN, Shushan, Dongguan, Guangdong 523871 (CN); LI, Zhaofeng, Dongguan, Guangdong 523871 (CN); XIAO, Lin, Dongguan, Guangdong 523871 (CN); WAN, Dan, Dongguan, Guangdong 523871 (CN); DONG, Junji, Dongguan, Guangdong 523871 (CN); XU, Yatao, Dongguan, Guangdong 523871 (CN); LI, Lijia, Dongguan, Guangdong 523871 (CN)
(74) Representative: Hoffmann Eitle
(86) International application number: PCT/CN2024/094173
(87) International publication number: WO 2024/240107

(57) **Abstract**

The present invention relates to an anti-FGFR2b antibody and application thereof. Specifically, the present invention relates to a novel anti-FGFR2b antibody, a nucleic acid encoding the antibody, a vector and a host cell for expression and production thereof, an antibody-drug conjugate, and a pharmaceutical composition comprising the antibody. Furthermore, the present invention relates to the use of the antibody in the diagnosis of an FGFR2b pathway dysregulation-related cancer, and the use of the antibody or antibody-drug conjugate in the treatment of an FGFR2b pathway dysregulation-related cancer.

## Description

### Priority Information

This application claims priority to and the benefit of patent application No. 202310568415.8 filed with the State Intellectual Property Office of China on May 19, 2023, and patent application No. 202311816681.4 filed with the State Intellectual Property Office of China on December 27, 2023, and the entire contents of each of which are incorporated herein by reference.

### FIELD OF THE INVENTION

The present invention relates to the field of biomedicine, specifically, the present invention relates to a novel anti-FGFR2 antibody and application thereof in the field of medicine.

### BACKGROUND ART

Fibroblast growth factor (FGF) plays an important role in embryonic development, tissue homeostasis and metabolism via FGF receptor signaling. In humans, 22 FGF family members have been identified, ranging in size from 17 to 34 kDa. They share similarities in their core regions and are divided into seven subfamilies based on their activity and sequence similarities. For example, the FGF1 subgroup includes the prototypical FGFs, FGF1, and FGF2; the FGF4 subgroup includes FGF4, FGF5, and FGF6; and the FGF7 subgroup includes FGF3, FGF7, FGF10, and FGF22.

FGF family members bind to only four known tyrosine kinase receptors, namely the fibroblast growth factor receptors (FGFRs). FGFR is transmembrane tyrosine kinase receptor. Similar to epidermal growth factor receptor (EGFR), abnormalities in FGFR signaling pathways have emerged as a key cause of various tumors. Overexpression and mutations of FGFRs often lead to abnormal activation of downstream signaling pathways, which are closely associated with the development and progression of various diseases.

The FGFR family includes four receptor subtypes: FGFR1, FGFR2, FGFR3 and FGFR4, as well as up to 18 fibroblast growth factor ligands (FGFs). These subtypes are part of the tyrosine kinase signaling pathway (FGF/FGFR signaling pathway) responsible for cell proliferation and differentiation. FGFR2, through alternative splicing, encodes FGFR2b (FGFR2 III b) and FGFR2c (FGFR2 III c), which have distinct expression domains and ligand specificities. FGFR2c is primarily expressed in mesenchymal cells and binds with high affinity to FGF1 and FGF2, while FGFR2b is primarily expressed in epithelial cells and can bind to FGF1, FGF7, FGF10 and FGF22. FGFR2b is highly expressed in various solid tumors and primarily contributes to tumor cell proliferation, survival, and epithelial-mesenchymal transition (EMT) through FGFR2 gene amplification and hyperactivation of this signaling pathway. It is a marker of poor prognosis in many tumors, making FGFR2b a highly promising target for solid tumors.

### SUMMARY

The present invention provides a novel anti-FGFR2b monoclonal antibody, in order to solve one of the technical problems in the related art to a certain extent.

The present invention provides a novel anti-FGFR2b monoclonal antibody, the anti-FGFR2b antibody is a chimeric antibody that can simultaneously block the binding of FGF7, FGF1 and FGF10 to FGFR2b, downregulate the tyrosine phosphorylation level of FGFR2b protein, and thus inhibit tumor growth and proliferation.

Specifically,
In the first aspect of the present invention, the present invention provides an antibody or an antigen-binding fragment thereof that binds to FGFR2, wherein the antibody comprises a heavy chain variable region and a light chain variable region; the heavy chain variable region comprises: VH-CDR1, VH-CDR2 and VH-CDR3,
wherein, the VH-CDR1 has an amino acid sequence shown in SEQ ID NO: 58 or SEQ ID NO: 59,
X1YNX2H (SEQ ID NO: 58),
X1YYX2H (SEQ ID NO: 59),
wherein, X1 is selected from T, N, D, S, F, I, Y or P, and X2 is selected from I, M, L or V, the VH-CDR2 has an amino acid sequence shown in SEQ ID NO: 60 or SEQ ID NO: 61, AIYPDNGDTX4YNX5X6FX7X8 (SEQ ID NO: 60),
LIYPX3NGDTSYNQKFX7X8 (SEQ ID NO: 61),
wherein, X3 is selected from D or E, X4 is selected from S or F, X5 is selected from Q or R, X6 is selected from K or R, X7 is selected from K, R or T, and X8 is selected from V, D, G or A,
the VH-CDR3 has an amino acid sequence shown in SEQ ID NO: 62 or SEQ ID NO: 26, GX9X10X11Y (SEQ ID NO: 62),
AFTY (SEQ ID NO: 26),
wherein, X9 is selected from D or G, X10 is selected from F or L, X11 is selected from A or D, and
the light chain variable region comprises: VL-CDR1, VL-CDR2 and VL-CDR3,
wherein, the VL-CDR1 has an amino acid sequence shown in SEQ ID NO: 35, SEQ ID NO: 37 or SEQ ID NO: 126,
X12ASX13X14X15X16X17X18X19X20 (SEQ ID NO: 126),
wherein, X12 is selected from G, R or K, X13 is selected from E, Q or G, X14 is selected from N or D, X15 is selected from I, L or V, X16 is selected from Y, S, G, N or H, X17 is selected from G, S, T or N, X18 is selected from A, N, Y or D, X19 is selected from L or V, X20 is selected from N, A or S,
QATQDIVKNLN (SEQ ID NO: 37),
KASQSVDYDGDSYMN (SEQ ID NO: 35),
the VL-CDR2 has an amino acid sequence shown in SEQ ID NO: 45, SEQ ID NO: 127 or SEQ ID NO: 128,
SASYRX21 (SEQ ID NO: 127),
wherein, X21 is selected from S or T,
GISNRFS (SEQ ID NO: 45),
X22AX23X24LAX25 (SEQ ID NO: 128),
wherein, X22 is selected from G, A, N or Y, X23 is selected from T or K, X24 is selected from T, N or E, and X25 is selected from D or E,
the VL-CDR3 has an amino acid sequence shown in SEQ ID NO: 129 or SEQ ID NO: 130, QX26X27X28X29TPX30T (SEQ ID NO: 129),
wherein, X26 is selected from N, H or Q, X27 is selected from V, F or H, X28 is selected from L, W or Y, X29 is selected from S, N or G, and X30 is selected from Y or F,
LQX31X32X33X34PX35T (SEQ ID NO: 130),
wherein, X31 is selected from G or F, X32 is selected from T or Y, X33 is selected from H or E, X34 is selected from Q or F, and X35 is selected from Y or L.

According to the embodiments of the present invention, the VH-CDR1 of the present invention has an amino acid sequence shown in SEQ ID NO: 58,
X1YNX2H (SEQ ID NO: 58),
wherein, X1 is selected from F, Y or P, and X2 is selected from I, M or V,
the VH-CDR2 has an amino acid sequence shown in SEQ ID NO: 61,
LIYPX3NGDTSYNQKFX7X8 (SEQ ID NO: 61),
wherein, X3 is selected from D, X7 is selected from K, X8 is selected from G or A,
the VH-CDR3 has an amino acid sequence shown in SEQ ID NO: 62,
GX9X10X11Y (SEQ ID NO: 62),
wherein, X9 is selected from G, X10 is selected from F or L, X11 is selected from D.

According to the embodiments of the present invention, the VL-CDR1 of the present invention has an amino acid sequence shown in SEQ ID NO: 37 or SEQ ID NO: 126,
QATQDIVKNLN (SEQ ID NO: 37),
X12ASX13X14X15X16X17X18X19X20 (SEQ ID NO: 126),
wherein, X12 is selected from G, R or K, X13 is selected from E or Q, X14 is selected from N or D, X15 is selected from I or V, X16 is selected from Y, S or N, X17 is selected from G, S or T, X18 is selected from A, N or D, X19 is selected from L or V, X20 is selected from N or A,
the VL-CDR2 has an amino acid sequence shown in SEQ ID NO: 127 or SEQ ID NO: 128, SASYRX21 (SEQ ID NO: 127),
wherein, X21 is selected from S or T,
X22AX23X24LAX25 (SEQ ID NO: 128),
wherein, X22 is selected from G, A, N or Y, X23 is selected from T or K, X24 is selected from T, N or E, and X25 is selected from D or E,
the VL-CDR3 has an amino acid sequence shown in SEQ ID NO: 129 or SEQ ID NO: 130, QX26X27X28X29TPX30T (SEQ ID NO: 129),
wherein, X26 is selected from N, H or Q, X27 is selected from V, F or H, X28 is selected from L, W or Y, X29 is selected from S or N, and X30 is selected from Y or F,
LQX31X32X33X34PX35T (SEQ ID NO: 130),
wherein, X31 is selected from F, X32 is selected from Y, X33 is selected from E, X34 is selected from F, and X35 is selected from L.

According to the embodiments of the present invention, the antibody of the present invention comprises:
(a) the VH-CDR1 has an amino acid sequence shown in SEQ ID NO: 2 or SEQ ID NO: 3,
   NYYIH (SEQ ID NO: 2),
   DYYIH (SEQ ID NO: 3),
   the VH-CDR2 has an amino acid sequence shown in SEQ ID NO: 15, SEQ ID NO: 16 or SEQ ID NO: 149,
   AIYPDNGDTSYNQRFRV (SEQ ID NO: 15),
   AIYPDNGDTSYNQKFKV (SEQ ID NO: 16),
   AIYPDNGDTSYNQRFKV (SEQ ID NO: 149),
   the VH-CDR3 has an amino acid sequence shown in SEQ ID NO: 26,
   AFTY (SEQ ID NO: 26), or
(b) wherein, the VH-CDR1 has an amino acid sequence shown in any one of SEQ ID NO: 4-13,
   TYNMH (SEQ ID NO: 4),
   SYNLH (SEQ ID NO: 5),
   FYNVH (SEQ ID NO: 6),
   IYNIH (SEQ ID NO: 7),
   FYNIH (SEQ ID NO: 8),
   YYNMH (SEQ ID NO: 9),
   PYNIH (SEQ ID NO: 10),
   TYNIH (SEQ ID NO: 11),
   FYNMH (SEQ ID NO: 12),
   YYNVH (SEQ ID NO: 13),
   the VH-CDR2 has an amino acid sequence shown in any one of SEQ ID NO: 17-22,
   AIYPDNGDTFYNRKFKD (SEQ ID NO: 17),
   LIYPENGDTSYNQKFKG (SEQ ID NO: 18),
   LIYPDNGDTSYNQKFKA (SEQ ID NO: 19),
   LIYPDNGDTSYNQKFKG (SEQ ID NO: 20),
   LIYPENGDTSYNQKFKD (SEQ ID NO: 21),
   LIYPDNGDTSYNQKFTG (SEQ ID NO: 22),
   the VH-CDR3 has an amino acid sequence shown in any one of SEQ ID NO: 23-25,
   GDFAY (SEQ ID NO: 23),
   GGFDY (SEQ ID NO: 24),
   GGLDY (SEQ ID NO: 25).

According to the embodiments of the present invention, in the present invention,
the VL-CDR1 has an amino acid sequence shown in any one of SEQ ID NO: 28-38,
the VL-CDR2 has an amino acid sequence shown in any one of SEQ ID NO: 39-46,
the VL-CDR3 has an amino acid sequence shown in any one of SEQ ID NO: 48-57.

According to the embodiments of the present invention, in the present invention,
the VH-CDR1 has an amino acid sequence shown in any one of SEQ ID NO: 2-13, or an amino acid sequence having at least 85%, at least 90%, or at least 95% identity to any one of SEQ ID NO: 2-13,
the VH-CDR2 has an amino acid sequence shown in any one of SEQ ID NO: 15-22, or an amino acid sequence having at least 85%, at least 90%, or at least 95% identity to any one of SEQ ID NO: 15-22,
the VH-CDR3 has an amino acid sequence shown in any one of SEQ ID NO: 23-26, or an amino acid sequence having at least 85%, at least 90%, or at least 95% identity to any one of SEQ ID NO: 23-26,
   and
the VL-CDR1 has an amino acid sequence shown in any one of SEQ ID NO: 28-38, or an amino acid sequence having at least 85%, at least 90%, or at least 95% identity to any one of SEQ ID NO: 28-38,
the VL-CDR2 has an amino acid sequence shown in any one of SEQ ID NO: 39-46, or an amino acid sequence having at least 85%, at least 90%, or at least 95% identity to any one of SEQ ID NO: 39-46,
the VL-CDR3 has an amino acid sequence shown in any one of SEQ ID NO: 48-57, or an amino acid sequence having at least 85%, at least 90%, or at least 95% identity to any one of SEQ ID NO: 48-57.

According to the embodiments of the present invention, the antibody of the present invention comprises
a heavy chain variable region comprising VH-CDR1, VH-CDR2 and VH-CDR3 shown in SEQ ID NO: 2, 15 and 26, or an amino acid sequence having at least 85%, at least 90% or at least 95% identity to SEQ ID NO: 2, 15 and 26, respectively; or
a heavy chain variable region comprising VH-CDR1, VH-CDR2 and VH-CDR3 shown in SEQ ID NO: 2, 149 and 26, or an amino acid sequence having at least 85%, at least 90% or at least 95% identity to SEQ ID NO: 2, 149 and 26, respectively; or
a heavy chain variable region comprising VH-CDR1, VH-CDR2 and VH-CDR3 shown in SEQ ID NO: 3, 16 and 26, or an amino acid sequence having at least 85%, at least 90% or at least 95% identity to SEQ ID NO: 3, 16 and 26, respectively; or
a heavy chain variable region comprising VH-CDR1, VH-CDR2 and VH-CDR3 shown in SEQ ID NO: 7, 19 and 24, or an amino acid sequence having at least 85%, at least 90% or at least 95% identity to SEQ ID NO: 7, 19 and 24, respectively; or
a heavy chain variable region comprising VH-CDR1, VH-CDR2 and VH-CDR3 shown in SEQ ID NO: 4, 17 and 23, or an amino acid sequence having at least 85%, at least 90% or at least 95% identity to SEQ ID NO: 4, 17 and 23, respectively; or
a heavy chain variable region comprising VH-CDR1, VH-CDR2 and VH-CDR3 shown in SEQ ID NO: 5, 18 and 24, or an amino acid sequence having at least 85%, at least 90% or at least 95% identity to SEQ ID NO: 5, 18 and 24, respectively; or
a heavy chain variable region comprising VH-CDR1, VH-CDR2 and VH-CDR3 shown in SEQ ID NO: 6, 19 and 24, or an amino acid sequence having at least 85%, at least 90% or at least 95% identity to SEQ ID NO: 6, 19 and 24, respectively; or
a heavy chain variable region comprising VH-CDR1, VH-CDR2 and VH-CDR3 shown in SEQ ID NO: 8, 19 and 24, or an amino acid sequence having at least 85%, at least 90% or at least 95% identity to SEQ ID NO: 8, 19 and 24, respectively; or
a heavy chain variable region comprising VH-CDR1, VH-CDR2 and VH-CDR3 shown in SEQ ID NO: 9, 20 and 24, or an amino acid sequence having at least 85%, at least 90% or at least 95% identity to SEQ ID NO: 9, 20 and 24, respectively; or
a heavy chain variable region comprising VH-CDR1, VH-CDR2 and VH-CDR3 shown in SEQ ID NO: 10, 19 and 24, or an amino acid sequence having at least 85%, at least 90% or at least 95% identity to SEQ ID NO: 10, 19 and 24, respectively; or
a heavy chain variable region comprising VH-CDR1, VH-CDR2 and VH-CDR3 shown in SEQ ID NO: 5, 18 and 24, or an amino acid sequence having at least 85%, at least 90% or at least 95% identity to SEQ ID NO: 5, 18 and 24, respectively; or
a heavy chain variable region comprising VH-CDR1, VH-CDR2 and VH-CDR3 shown in SEQ ID NO: 5, 21 and 24, or an amino acid sequence having at least 85%, at least 90% or at least 95% identity to SEQ ID NO: 5, 21 and 24, respectively; or
a heavy chain variable region comprising VH-CDR1, VH-CDR2 and VH-CDR3 shown in SEQ ID NO: 11, 19 and 24, or an amino acid sequence having at least 85%, at least 90% or at least 95% identity to SEQ ID NO: 11, 19 and 24, respectively; or
a heavy chain variable region comprising VH-CDR1, VH-CDR2 and VH-CDR3 shown in SEQ ID NO: 9, 20 and 25, or an amino acid sequence having at least 85%, at least 90% or at least 95% identity to SEQ ID NO: 9, 20 and 25, respectively; or
a heavy chain variable region comprising VH-CDR1, VH-CDR2 and VH-CDR3 shown in SEQ ID NO: 12, 20 and 25, or an amino acid sequence having at least 85%, at least 90% or at least 95% identity to SEQ ID NO: 12, 20 and 25, respectively; or
a heavy chain variable region comprising VH-CDR1, VH-CDR2 and VH-CDR3 shown in SEQ ID NO: 13, 22 and 25, or an amino acid sequence having at least 85%, at least 90% or at least 95% identity to SEQ ID NO: 13, 22 and 25, respectively.

According to the embodiments of the present invention, the antibody of the present invention comprises
a light chain variable region comprising VL-CDR1, VL-CDR2 and VL-CDR3 shown in SEQ ID NO: 28, 40 and 48, or an amino acid sequence having at least 85%, at least 90% or at least 95% identity to SEQ ID NO: 28, 40 and 48, respectively; or
a light chain variable region comprising VL-CDR1, VL-CDR2 and VL-CDR3 shown in SEQ ID NO: 27, 39 and 47, or an amino acid sequence having at least 85%, at least 90% or at least 95% identity to SEQ ID NO: 27, 39 and 47, respectively; or
a light chain variable region comprising VL-CDR1, VL-CDR2 and VL-CDR3 shown in SEQ ID NO: 32, 42 and 50, or an amino acid sequence having at least 85%, at least 90% or at least 95% identity to SEQ ID NO: 32, 42 and 50, respectively; or
a light chain variable region comprising VL-CDR1, VL-CDR2 and VL-CDR3 shown in SEQ ID NO: 30, 41 and 49, or an amino acid sequence having at least 85%, at least 90% or at least 95% identity to SEQ ID NO: 30, 41 and 49, respectively; or
a light chain variable region comprising VL-CDR1, VL-CDR2 and VL-CDR3 shown in SEQ ID NO: 31, 42 and 50, or an amino acid sequence having at least 85%, at least 90% or at least 95% identity to SEQ ID NO: 31, 42 and 50, respectively; or
a light chain variable region comprising VL-CDR1, VL-CDR2 and VL-CDR3 shown in SEQ ID NO: 32, 42 and 51, or an amino acid sequence having at least 85%, at least 90% or at least 95% identity to SEQ ID NO: 32, 42 and 51, respectively; or
a light chain variable region comprising VL-CDR1, VL-CDR2 and VL-CDR3 shown in SEQ ID NO: 33, 43 and 52, or an amino acid sequence having at least 85%, at least 90% or at least 95% identity to SEQ ID NO: 33, 43 and 52, respectively; or
a light chain variable region comprising VL-CDR1, VL-CDR2 and VL-CDR3 shown in SEQ ID NO: 32, 42 and 53, or an amino acid sequence having at least 85%, at least 90% or at least 95% identity to SEQ ID NO: 32, 42 and 53, respectively; or
a light chain variable region comprising VL-CDR1, VL-CDR2 and VL-CDR3 shown in SEQ ID NO: 34, 39 and 51, or an amino acid sequence having at least 85%, at least 90% or at least 95% identity to SEQ ID NO: 34, 39 and 51, respectively; or
a light chain variable region comprising VL-CDR1, VL-CDR2 and VL-CDR3 shown in SEQ ID NO: 30, 41 and 54, or an amino acid sequence having at least 85%, at least 90% or at least 95% identity to SEQ ID NO: 30, 41 and 54, respectively; or
a light chain variable region comprising VL-CDR1, VL-CDR2 and VL-CDR3 shown in SEQ ID NO: 32, 39 and 50, or an amino acid sequence having at least 85%, at least 90% or at least 95% identity to SEQ ID NO: 32, 39 and 50, respectively; or
a light chain variable region comprising VL-CDR1, VL-CDR2 and VL-CDR3 shown in SEQ ID NO: 30, 44 and 55, or an amino acid sequence having at least 85%, at least 90% or at least 95% identity to SEQ ID NO: 30, 44 and 55, respectively; or
a light chain variable region comprising VL-CDR1, VL-CDR2 and VL-CDR3 shown in SEQ ID NO: 35, 45 and 56, or an amino acid sequence having at least 85%, at least 90% or at least 95% identity to SEQ ID NO: 35, 45 and 56, respectively; or
a light chain variable region comprising VL-CDR1, VL-CDR2 and VL-CDR3 shown in SEQ ID NO: 36, 39 and 51, or an amino acid sequence having at least 85%, at least 90% or at least 95% identity to SEQ ID NO: 36, 39 and 51, respectively; or
a light chain variable region comprising VL-CDR1, VL-CDR2 and VL-CDR3 shown in SEQ ID NO: 37, 46 and 57, or an amino acid sequence having at least 85%, at least 90% or at least 95% identity to SEQ ID NO: 37, 46 and 57, respectively; or
a light chain variable region comprising VL-CDR1, VL-CDR2 and VL-CDR3 shown in SEQ ID NO: 38, 41 and 49, or an amino acid sequence having at least 85%, at least 90% or at least 95% identity to SEQ ID NO: 38, 41 and 49, respectively.

According to the embodiments of the present invention, the antibody of the present invention comprises
(a) a heavy chain variable region comprising VH-CDR1, VH-CDR2 and VH-CDR3 shown in SEQ ID NO: 2, 15 and 26, and a light chain variable region comprising VL-CDR1, VL-CDR2 and VL-CDR3 shown in SEQ ID NO: 28, 40 and 48; or
(b) a heavy chain variable region comprising VH-CDR1, VH-CDR2 and VH-CDR3 shown in SEQ ID NO: 3, 16 and 26, and a light chain variable region comprising VL-CDR1, VL-CDR2 and VL-CDR3 shown in SEQ ID NO: 28, 40 and 48; or
(c) a heavy chain variable region comprising VH-CDR1, VH-CDR2 and VH-CDR3 shown in SEQ ID NO: 7, 19 and 24, and a light chain variable region comprising VL-CDR1, VL-CDR2 and VL-CDR3 shown in SEQ ID NO: 32, 42 and 50; or
(d) a heavy chain variable region comprising VH-CDR1, VH-CDR2 and VH-CDR3 shown in SEQ ID NO: 4, 17 and 23, and a light chain variable region comprising VL-CDR1, VL-CDR2 and VL-CDR3 shown in SEQ ID NO: 28, 40 and 48; or
(e) a heavy chain variable region comprising VH-CDR1, VH-CDR2 and VH-CDR3 shown in SEQ ID NO: 5, 18 and 24, and a light chain variable region comprising VL-CDR1, VL-CDR2 and VL-CDR3 shown in SEQ ID NO: 30, 41 and 49; or
(f) a heavy chain variable region comprising VH-CDR1, VH-CDR2 and VH-CDR3 shown in SEQ ID NO: 6, 19 and 24, and a light chain variable region comprising VL-CDR1, VL-CDR2 and VL-CDR3 shown in SEQ ID NO: 31, 42 and 50; or
(g) a heavy chain variable region comprising VH-CDR1, VH-CDR2 and VH-CDR3 shown in SEQ ID NO: 7, 19 and 24, and a light chain variable region comprising VL-CDR1, VL-CDR2 and VL-CDR3 shown in SEQ ID NO: 32, 42 and 51; or
(h) a heavy chain variable region comprising VH-CDR1, VH-CDR2 and VH-CDR3 shown in SEQ ID NO: 8, 19 and 24, and a light chain variable region comprising VL-CDR1, VL-CDR2 and VL-CDR3 shown in SEQ ID NO: 28, 40 and 48; or
(i) a heavy chain variable region comprising VH-CDR1, VH-CDR2 and VH-CDR3 shown in SEQ ID NO: 8, 19 and 24, and a light chain variable region comprising VL-CDR1, VL-CDR2 and VL-CDR3 shown in SEQ ID NO: 33, 43 and 52; or
(j) a heavy chain variable region comprising VH-CDR1, VH-CDR2 and VH-CDR3 shown in SEQ ID NO: 9, 20 and 24, and a light chain variable region comprising VL-CDR1, VL-CDR2 and VL-CDR3 shown in SEQ ID NO: 28, 40 and 48; or
(k) a heavy chain variable region comprising VH-CDR1, VH-CDR2 and VH-CDR3 shown in SEQ ID NO: 10, 19 and 24, and a light chain variable region comprising VL-CDR1, VL-CDR2 and VL-CDR3 shown in SEQ ID NO: 32, 42 and 53; or
(l) a heavy chain variable region comprising VH-CDR1, VH-CDR2 and VH-CDR3 shown in SEQ ID NO: 5, 18 and 24, and a light chain variable region comprising VL-CDR1, VL-CDR2 and VL-CDR3 shown in SEQ ID NO: 28, 40 and 48; or
(m) a heavy chain variable region comprising VH-CDR1, VH-CDR2 and VH-CDR3 shown in SEQ ID NO: 5, 18 and 24, and a light chain variable region comprising VL-CDR1, VL-CDR2 and VL-CDR3 shown in SEQ ID NO: 28, 40 and 48; or
(n) a heavy chain variable region comprising VH-CDR1, VH-CDR2 and VH-CDR3 shown in SEQ ID NO: 5, 21 and 24, and a light chain variable region comprising VL-CDR1, VL-CDR2 and VL-CDR3 shown in SEQ ID NO: 32, 42 and 50; or
(o) a heavy chain variable region comprising VH-CDR1, VH-CDR2 and VH-CDR3 shown in SEQ ID NO: 11, 19 and 24, and a light chain variable region comprising VL-CDR1, VL-CDR2 and VL-CDR3 shown in SEQ ID NO: 34, 39 and 51; or
(p) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 28, 40 and 48; or
(q) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 30, 41 and 54; or
(r) a heavy chain variable region comprising VH-CDR1, VH-CDR2 and VH-CDR3 shown in SEQ ID NO: 12, 20 and 25, and a light chain variable region comprising VL-CDR1, VL-CDR2 and VL-CDR3 shown in SEQ ID NO: 32, 39 and 50; or
(s) a heavy chain variable region comprising VH-CDR1, VH-CDR2 and VH-CDR3 shown in SEQ ID NO: 13, 22 and 25, and a light chain variable region comprising VL-CDR1, VL-CDR2 and VL-CDR3 shown in SEQ ID NO: 28, 40 and 48; or
(t) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 28, 40 and 48; or
(u) a heavy chain variable region comprising VH-CDR1, VH-CDR2 and VH-CDR3 shown in SEQ ID NO: 12, 20 and 25, and a light chain variable region comprising VL-CDR1, VL-CDR2 and VL-CDR3 shown in SEQ ID NO: 30, 44 and 55; or
(v) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 28, 40 and 48; or
(w) a heavy chain variable region comprising VH-CDR1, VH-CDR2 and VH-CDR3 shown in SEQ ID NO: 12, 20 and 25, and a light chain variable region comprising VL-CDR1, VL-CDR2 and VL-CDR3 shown in SEQ ID NO: 35, 45 and 56; or
(x) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 36, 39 and 51; or
(y) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 37, 46 and 57; or
(z) a heavy chain variable region comprising VH-CDR1, VH-CDR2 and VH-CDR3 shown in SEQ ID NO: 9, 20 and 25, and a light chain variable region comprising VL-CDR1, VL-CDR2 and VL-CDR3 shown in SEQ ID NO: 38, 41 and 49.

According to the embodiments of the present invention, the amino acid sequences of SEQ ID NO: 1-57 of the present invention are as follows.
TYNVH (SEQ ID NO: 1).
NYYIH (SEQ ID NO: 2).
DYYIH (SEQ ID NO: 3).
TYNMH (SEQ ID NO: 4).
SYNLH (SEQ ID NO: 5).
FYNVH (SEQ ID NO: 6).
IYNIH (SEQ ID NO: 7).
FYNIH (SEQ ID NO: 8).
YYNMH (SEQ ID NO: 9).
PYNIH (SEQ ID NO: 10).
TYNIH (SEQ ID NO: 11).
FYNMH (SEQ ID NO: 12).
YYNVH (SEQ ID NO: 13).
SIYPDNGDTSYNQNFKG (SEQ ID NO: 14).
AIYPDNGDTSYNQRFRV (SEQ ID NO: 15).
AIYPDNGDTSYNQKFKV (SEQ ID NO: 16).
AIYPDNGDTFYNRKFKD (SEQ ID NO: 17).
LIYPENGDTSYNQKFKG (SEQ ID NO: 18).
LIYPDNGDTSYNQKFKA (SEQ ID NO: 19).
LIYPDNGDTSYNQKFKG (SEQ ID NO: 20).
LIYPENGDTSYNQKFKD (SEQ ID NO: 21).
LIYPDNGDTSYNQKFTG (SEQ ID NO: 22).
GDFAY (SEQ ID NO: 23).
GGFDY (SEQ ID NO: 24).
GGLDY (SEQ ID NO: 25).
AFTY (SEQ ID NO: 26).
KASQGVSNDVA (SEQ ID NO: 27).
GASENIYGALN (SEQ ID NO: 28).
GASENLYGALN (SEQ ID NO: 29).
RASENIYSNLA (SEQ ID NO: 30).
KASQDVSTAVA (SEQ ID NO: 31).
KASQNVGTNVA (SEQ ID NO: 32).
RASENIYSYLA (SEQ ID NO: 33).
KASQDVNTDVS (SEQ ID NO: 34).
KASQSVDYDGDSYMN (SEQ ID NO: 35).
KASQDVNTDVA (SEQ ID NO: 36).
QATQDIVKNLN (SEQ ID NO: 37).
RASGNIHNYLA (SEQ ID NO: 38).
SASYRYT (SEQ ID NO: 39).
GATNLAD (SEQ ID NO: 40).
NAKTLAD (SEQ ID NO: 41).
SASYRYS (SEQ ID NO: 42).
NAKTLAE (SEQ ID NO: 43).
AATNLAD (SEQ ID NO: 44).
GISNRFS (SEQ ID NO: 45).
YATELAE (SEQ ID NO: 46).
QQHSTTPYT (SEQ ID NO: 47).
QNVLSTPYT (SEQ ID NO: 48).
QHFWSTPYT (SEQ ID NO: 49).
QQHYSTPYT (SEQ ID NO: 50).
QQHYNTPYT (SEQ ID NO: 51).
QHHYGTPYT (SEQ ID NO: 52).
QQHYSTPFT (SEQ ID NO: 53).
QHFWSTPFT (SEQ ID NO: 54).
QHFWGTPYT (SEQ ID NO: 55).
LQGTHQPYT (SEQ ID NO: 56).
LQFYEFPLT (SEQ ID NO: 57).

According to the embodiments of the present invention, the CDR sequences of the present invention are defined with reference to the Kabat nomenclature system.

According to the embodiments of the present invention, the antibody of the present invention comprises at least one of a heavy chain framework region sequence and a light chain framework region sequence, and at least a part of at least one of the heavy chain framework region sequence and the light chain framework region sequence is derived from at least one of a murine antibody, a primate antibody, or a mutant thereof.

According to the embodiments of the present invention, the antibody of the present invention comprises a heavy chain variable region having an amino acid sequence with at least 85%, at least 90% or at least 95% identical to any one of SEQ ID NO: 64-89.

According to the embodiments of the present invention, the antibody of the present invention comprises a heavy chain variable region shown in any one of SEQ ID NO: 64-89.

According to the embodiments of the present invention, the antibody of the present invention comprises a light chain variable region having an amino acid sequence with at least 85%, at least 90% or at least 95% identical to any one of SEQ ID NO: 91-116.

According to the embodiments of the present invention, the antibody of the present invention comprises a light chain variable region shown in any one of SEQ ID NO: 91-116.

According to the embodiments of the present invention, specifically, the antibody of the present invention comprises
(a) a heavy chain variable region having an amino acid sequence with at least 85%, at least 90% or at least 95% identical to SEQ ID NO: 64, and a light chain variable region having an amino acid sequence with at least 85%, at least 90% or at least 95% identical to SEQ ID NO: 106, or
(b) a heavy chain variable region having an amino acid sequence with at least 85%, at least 90% or at least 95% identical to SEQ ID NO: 65, and a light chain variable region having an amino acid sequence with at least 85%, at least 90% or at least 95% identical to SEQ ID NO: 92, or
(c) a heavy chain variable region having an amino acid sequence with at least 85%, at least 90% or at least 95% identical to SEQ ID NO: 66, and a light chain variable region having an amino acid sequence with at least 85%, at least 90% or at least 95% identical to SEQ ID NO: 93, or
(d) a heavy chain variable region having an amino acid sequence with at least 85%, at least 90% or at least 95% identical to SEQ ID NO: 67, and a light chain variable region shown in SEQ ID NO: 94 or having an amino acid sequence with at least 85%, at least 90% or at least 95% identical to SEQ ID NO: 94, or
(e) a heavy chain variable region having an amino acid sequence with at least 85%, at least 90% or at least 95% identical to SEQ ID NO: 68, and a light chain variable region having an amino acid sequence with at least 85%, at least 90% or at least 95% identical to SEQ ID NO: 95, or
(f) a heavy chain variable region having an amino acid sequence with at least 85%, at least 90% or at least 95% identical to SEQ ID NO: 69, and a light chain variable region having an amino acid sequence with at least 85%, at least 90% or at least 95% identical to SEQ ID NO: 96, or
(g) a heavy chain variable region having an amino acid sequence with at least 85%, at least 90% or at least 95% identical to SEQ ID NO: 70, and a light chain variable region having an amino acid sequence with at least 85%, at least 90% or at least 95% identical to SEQ ID NO: 97, or
(h) a heavy chain variable region having an amino acid sequence with at least 85%, at least 90% or at least 95% identical to SEQ ID NO: 71, and a light chain variable region having an amino acid sequence with at least 85%, at least 90% or at least 95% identical to SEQ ID NO: 98, or
(i) a heavy chain variable region having an amino acid sequence with at least 85%, at least 90% or at least 95% identical to SEQ ID NO: 72, and a light chain variable region having an amino acid sequence with at least 85%, at least 90% or at least 95% identical to SEQ ID NO: 99, or
(j) a heavy chain variable region having an amino acid sequence with at least 85%, at least 90% or at least 95% identical to SEQ ID NO: 73, and a light chain variable region having an amino acid sequence with at least 85%, at least 90% or at least 95% identical to SEQ ID NO: 100, or
(k) a heavy chain variable region having an amino acid sequence with at least 85%, at least 90% or at least 95% identical to SEQ ID NO: 74, and a light chain variable region having an amino acid sequence with at least 85%, at least 90% or at least 95% identical to SEQ ID NO: 101, or
(l) a heavy chain variable region having an amino acid sequence with at least 85%, at least 90% or at least 95% identical to SEQ ID NO: 75, and a light chain variable region having an amino acid sequence with at least 85%, at least 90% or at least 95% identical to SEQ ID NO: 102, or
(m) a heavy chain variable region having an amino acid sequence with at least 85%, at least 90% or at least 95% identical to SEQ ID NO: 76, and a light chain variable region having an amino acid sequence with at least 85%, at least 90% or at least 95% identical to SEQ ID NO: 103, or
(n) a heavy chain variable region having an amino acid sequence with at least 85%, at least 90% or at least 95% identical to SEQ ID NO: 77, and a light chain variable region having an amino acid sequence with at least 85%, at least 90% or at least 95% identical to SEQ ID NO: 104, or
(o) a heavy chain variable region having an amino acid sequence with at least 85%, at least 90% or at least 95% identical to SEQ ID NO: 78, and a light chain variable region having an amino acid sequence with at least 85%, at least 90% or at least 95% identical to SEQ ID NO: 105, or
(p) a heavy chain variable region having an amino acid sequence with at least 85%, at least 90% or at least 95% identical to SEQ ID NO: 79, and a light chain variable region having an amino acid sequence with at least 85%, at least 90% or at least 95% identical to SEQ ID NO: 106, or
(q) a heavy chain variable region having an amino acid sequence with at least 85%, at least 90% or at least 95% identical to SEQ ID NO: 80, and a light chain variable region having an amino acid sequence with at least 85%, at least 90% or at least 95% identical to SEQ ID NO: 107, or
(r) a heavy chain variable region having an amino acid sequence with at least 85%, at least 90% or at least 95% identical to SEQ ID NO: 81, and a light chain variable region having an amino acid sequence with at least 85%, at least 90% or at least 95% identical to SEQ ID NO: 108, or
(s) a heavy chain variable region having an amino acid sequence with at least 85%, at least 90% or at least 95% identical to SEQ ID NO: 82, and a light chain variable region having an amino acid sequence with at least 85%, at least 90% or at least 95% identical to SEQ ID NO: 109, or
(t) a heavy chain variable region having an amino acid sequence with at least 85%, at least 90% or at least 95% identical to SEQ ID NO: 83, and a light chain variable region having an amino acid sequence with at least 85%, at least 90% or at least 95% identical to SEQ ID NO: 110, or
(u) a heavy chain variable region having an amino acid sequence with at least 85%, at least 90% or at least 95% identical to SEQ ID NO: 84, and a light chain variable region having an amino acid sequence with at least 85%, at least 90% or at least 95% identical to SEQ ID NO: 111, or
(v) a heavy chain variable region having an amino acid sequence with at least 85%, at least 90% or at least 95% identical to SEQ ID NO: 85, and a light chain variable region having an amino acid sequence with at least 85%, at least 90% or at least 95% identical to SEQ ID NO: 112, or
(w) a heavy chain variable region having an amino acid sequence with at least 85%, at least 90% or at least 95% identical to SEQ ID NO: 86, and a light chain variable region having an amino acid sequence with at least 85%, at least 90% or at least 95% identical to SEQ ID NO: 113, or
(x) a heavy chain variable region having an amino acid sequence with at least 85%, at least 90% or at least 95% identical to SEQ ID NO: 87, and a light chain variable region having an amino acid sequence with at least 85%, at least 90% or at least 95% identical to SEQ ID NO: 114, or
(y) a heavy chain variable region having an amino acid sequence with at least 85%, at least 90% or at least 95% identical to SEQ ID NO: 88, and a light chain variable region having an amino acid sequence with at least 85%, at least 90% or at least 95% identical to SEQ ID NO: 115, or
(z) a heavy chain variable region having an amino acid sequence with at least 85%, at least 90% or at least 95% identical to SEQ ID NO: 89, and a light chain variable region having an amino acid sequence with at least 85%, at least 90% or at least 95% identical to SEQ ID NO: 116.

According to the embodiments of the present invention, specifically, the antibody of the present invention comprises
(a) a heavy chain variable shown in SEQ ID NO: 64 and a light chain variable region shown in SEQ ID NO: 106; or
(b) a heavy chain variable shown in SEQ ID NO: 65 and a light chain variable region shown in SEQ ID NO: 92; or
(c) a heavy chain variable shown in SEQ ID NO: 66 and a light chain variable region shown in SEQ ID NO: 93; or
(d) a heavy chain variable shown in SEQ ID NO: 67 and a light chain variable region shown in SEQ ID NO: 94; or
(e) a heavy chain variable shown in SEQ ID NO: 68 and a light chain variable region shown in SEQ ID NO: 95; or
(f) a heavy chain variable shown in SEQ ID NO: 69 and a light chain variable region shown in SEQ ID NO: 96; or
(g) a heavy chain variable shown in SEQ ID NO: 70 and a light chain variable region shown in SEQ ID NO: 97; or
(h) a heavy chain variable shown in SEQ ID NO: 71 and a light chain variable region shown in SEQ ID NO: 98; or
(i) a heavy chain variable shown in SEQ ID NO: 72 and a light chain variable region shown in SEQ ID NO: 99; or
(j) a heavy chain variable shown in SEQ ID NO: 73 and a light chain variable region shown in SEQ ID NO: 100; or
(k) a heavy chain variable shown in SEQ ID NO: 74 and a light chain variable region shown in SEQ ID NO: 101; or
(l) a heavy chain variable shown in SEQ ID NO: 75 and a light chain variable region shown in SEQ ID NO: 102; or
(m) a heavy chain variable shown in SEQ ID NO: 76 and a light chain variable region shown in SEQ ID NO: 103; or
(n) a heavy chain variable shown in SEQ ID NO: 77 and a light chain variable region shown in SEQ ID NO: 104; or
(o) a heavy chain variable shown in SEQ ID NO: 78 and a light chain variable region shown in SEQ ID NO: 105; or
(p) a heavy chain variable shown in SEQ ID NO: 79 and a light chain variable region shown in SEQ ID NO: 106; or
(q) a heavy chain variable shown in SEQ ID NO: 80 and a light chain variable region shown in SEQ ID NO: 107; or
(r) a heavy chain variable shown in SEQ ID NO: 81 and a light chain variable region shown in SEQ ID NO: 108; or
(s) a heavy chain variable shown in SEQ ID NO: 82 and a light chain variable region shown in SEQ ID NO: 109; or
(t) a heavy chain variable shown in SEQ ID NO: 83 and a light chain variable region shown in SEQ ID NO: 110; or
(u) a heavy chain variable shown in SEQ ID NO: 84 and a light chain variable region shown in SEQ ID NO: 111; or
(v) a heavy chain variable shown in SEQ ID NO: 85 and a light chain variable region shown in SEQ ID NO: 112; or
(w) a heavy chain variable shown in SEQ ID NO: 86 and a light chain variable region shown in SEQ ID NO: 113; or
(x) a heavy chain variable shown in SEQ ID NO: 87 and a light chain variable region shown in SEQ ID NO: 114; or
(y) a heavy chain variable shown in SEQ ID NO: 88 and a light chain variable region shown in SEQ ID NO: 115; or
(z) a heavy chain variable shown in SEQ ID NO: 89 and a light chain variable region shown in SEQ ID NO: 116.

According to the embodiments of the present invention, a heavy chain variable region comprising VH-CDR1, VH-CDR2, and VH-CDR3 shown in SEQ ID NO: 1, 14 and 23, has the amino acid sequence shown in SEQ ID NO: 63; the corresponding a light chain variable region has the amino acid sequence shown in SEQ ID NO: 90, and the light chain variable region comprises VL-CDR1, VL-CDR2, and VL-CDR3 shown in SEQ ID NO: 27, 39 and 47.

According to an embodiment of the present invention, SEQ ID NO: 63-116 of the present invention have the amino acid sequences shown below.



(SEQ ID NO: 63).


 (SEQ ID NO: 90).
EIQLQQSGAELVKPGASVKMSCKASGYIFRNYYIHWVKRTPGQGLEWIGAIYPDNGDTS
YNQRFKVKATLTADTSSSTAYMQLSSLTSEDSAVYYCARAFTYWGQGTTLTVSS (SEQ
ID NO: 64).

DILMTQSPASLSASVGETVTIKCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
91).

QVQLQQPGAEVVKPGASVKMSCKASGYTFTDYYIHWVKQTPGQGLEWIGAIYPDN
GDTSYNQKFKVKATLTADKSSSTAYMQLSSLTSEDSAVYYCARAFTYWGQGTLVTVSA
(SEQ ID NO: 65).

DILITQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
92).

QVQLQQSGAELVKPGASVKMSCKASGYTFTIYNIHWVRQTPGQGLEWIGLIYPDNG
DTSYNQKFKAKATLTADKSSSTAYMQLSSLTSEDSAVYYCARGGFDYWGQGTSLTVSS
(SEQ ID NO: 66).

DIVMTQSQKFMSTSVGDRVSVTCKASQNVGTNVAWYQQKPGQSPKALIYSASYRYS
GVPDRFTGSGSGTDFTLTISNVQAEDLADYFCQQHYSTPYTFGGGTKLEMK (SEQ ID NO:
93).

EVQLQQSGPELVKPGASVKMSCKASGYTFATYNMHWVKQTTGQGLEWIGAIYPDN
GDTFYNRKFKDKATLTADKSSTTAYMQLSSLTSEDSAVYYCARGDFAYWGQGTLVTVSA
(SEQ ID NO: 67).

DVLMTQSPASLSASVGETVTITCGASENIYGALNWYQRKQGKSPQLLIYGATNLAD
GMSSRFSGSGSGRQYSLKISSLHPDDVATYYCQNVLSTPYTFGGGTKLEMK (SEQ ID NO:
94).

EVQLQQSGAELVKPGASVKMSCKASGYTFASYNLHWVKQTPGQGLEWIGLIYPEN
GDTSYNQKFKGKATLTADKSSSTAYIQLSSLTSEDSAVYFCARGGFDYWGQGTSLTVSS
(SEQ ID NO: 68).

DIQMIQSPASLSVSVGETVTITCRASENIYSNLAWYQQKQGKSPQLLVYNAKTLADG
VPSRFSGSGSGTQYSLKINSLQPEDFGSYYCQHFWSTPYTFGGGTKLEIK (SEQ ID NO:
95).

EVQLQQSGAELVKPGASVKMSCKASGYTFSFYNVHWVRQTPGQGLEWIGLIYPDN
GDTSYNQKFKAKATLTADKSSSAAYMQLSSLTSEDSAVYYCARGGFDYWGQGTSLTVSS
(SEQ ID NO: 69).

DIVMTQSHKFMSTSVGDRVNITCKASQDVSTAVAWYQQKPGQSPKLLIYSASYRYSG
VPDRFTGSGSGTDFTFTISSVQAEDLAVYYCQQHYSTPYTFGGGTKLEMK (SEQ ID NO:
96).

QVQLQQSGAELVKPGASVKMSCKASGYTFTIYNIHWVRQTPGQGLEWIGLIYPDNG
DTSYNQKFKAKATLTADKSSSTAYMQLSSLTSEDSAVYYCARGGFDYWGQGTTLTVSS
(SEQ ID NO: 70).

NIVMTQSQKFMSTSVGDRVSVTCKASQNVGTNVAWYQQKPGQSPKALIYSASYRYS
GVPDRFTGSGSGTDFTLTISGVQAEDLADYYCQQHYNTPYTFGGGTKLEIK (SEQ ID NO:
97).

EVQLQQSGAELVKPGASVKMSCKASGYTFSFYNIHWVRQTPGQGLEWIGLIYPDNG

DTSYNQKFKAKATLTADKSSSTAYMQLSSLTSEDSAVYYCARGGFDYWGQGTTLTVSS
(SEQ ID NO: 71).

DVLMTQSPASLSASVGETVTITCGASENIYGALNWYQRKQGKSPQLLIYGATNLAD
GMSSRFSGSGSGRQYSLKISSLHPDDVATYYCQNVLSTPYTFGGGTKLEIK (SEQ ID NO:
98).

QVQLQQSGAELVKPGASVKMSCKASGYTFSFYNIHWVRQTPGQGLEWIGLIYPDNG
DTSYNQKFKAKATLTADKSSSTAYMQLSSLTSEDSAVYYCARGGFDYWGQGTTLTVSS
(SEQ ID NO: 72).

DIQMTQSPASLSASVGETVTITCRASENIYSYLAWYQQKQGKSPQLLVYNAKTLAEG
VPSRFSGSGSGTQFSLKINSLQPEDFGSYYCQHHYGTPYTFGGGTKLEMK (SEQ ID NO:
99).

EVQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGFDYWGQGTTLTVSS
(SEQ ID NO: 73).

DIQMTQSPASLSASVGETVTITCGASENIYGALNWYQRKQGKSPQLLIYGATNLADG
MSSRFSGSGSGRQYSLKISSLHPDDVATYYCQNVLSTPYTFGGGTKLELK (SEQ ID NO:
100).

QVQLQQSGAELVKPGASVKMSCKASGYTFAPYNIHWVRQTPGQGLEWIGLIYPDN
GDTSYNQKFKAKATLTADKSSSTAYMQLSSLTSEDSAVYYCARGGFDYWGQGTSLTVSS
(SEQ ID NO: 74).

DIVMTQSQKFMSTSVGDRVSVTCKASQNVGTNVAWYQQKPGQSPKALIYSASYRYS
GVPDRFTGSGSGTDFTLTISNVQSEDLAEYFCQQHYSTPFTFGSGTKLEIK (SEQ ID NO:
101).

QIQLQQSGAELVKPGASVKMSCKASGYTFASYNLHWVKQTPGQGLEWIGLIYPENG
DTSYNQKFKGKATLTADTSSTTAYIQLSSLTSEDSAVYFCARGGFDYWGQGTSLTVSS
(SEQ ID NO: 75).

DIKMTQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
102).

QVQLQQPGAELVKPGASVKMSCKASGYTFASYNLHWVKQTPGQGLEWIGLIYPEN
GDTSYNQKFKGKATLTADKSSSTAYIQLSSLTSEDSAVYFCARGGFDYWGQGTTLTVSQ

(SEQ ID NO: 76).

DIQMTQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
103).

QLQLQQSGAELVKPGASVKMSCKASGYTFASYNLHWVKQTPGQGLEWIGLIYPEN
GDTSYNQKFKDKATLTADKSSTTAYIQLSSLASEDSAVYFCARGGFDYWGQGTTLTVSS
(SEQ ID NO: 77).

DIVMTQSQKFMSTSVGDRVSVTCKASQNVGTNVAWYQQKPGQSPKALIYSASYRYS
GVPDRFTGSGSGTDYTLTISSVQAEDLALYYCQQHYSTPYTFGGGTKLELK (SEQ ID NO:
104).

QVQLQQSGAELVKPGASVKMSCKASGYTFTTYNIHWVRQTPGQGLEWIGLIYPDN
GDTSYNQKFKAKATLTADKSSSTAYMHLSSLTSEDSAVYYCARGGFDYWGQGTTLTVSS
(SEQ ID NO: 78).

DIVMTQSHKFMSTSVGDRVSITCKASQDVNTDVSWYQQKPGQSPKLLIYSASYRYT
GVPTRFTGSGSGTDFTFTISGVQAEDLADYYCQQHYNTPYTFGGGTRLEIK (SEQ ID NO:
105).

EIQLQQSGAELVKPGASVKMSCKASGYIFRYYNMHWVKRTPGQGLEWIGLIYPDNG
DTSYNQKFKGKATLTADTSSSTAYMQLSSLTSEDSAVYYCARGGLDYWGQGTTLTVSS
(SEQ ID NO: 79).

DIQMIQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
106).

QVQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
(SEQ ID NO: 80).

DIQMTQSPASLSVSVGETVTITCRASENIYSNLAWYQQKQGKSPQLLVYNAKTLADG
VPSRFSGSGSGTQYSLKINSLQPEDFGSYYCQHFWSTPFTFGSGTKLELK (SEQ ID NO:
107).

EIQLQQSGAELVKPGASVKMSCKASGYTFSFYNMHWVKRTPGQGLEWIGLIYPDNG
DTSYNQKFKGKATLTADTSSSTAYMQLSSLTSEDSAVYYCARGGLDYWGQGTSLTVSS
(SEQ ID NO: 81).

NIVMTQSQKFMSTSVGDRVSVTCKASQNVGTNVAWYQQKPGQSPKALIYSASYRYT
GVPDRFTGSGSGTDFTFTISSVQAEDLAVYYCQQHYSTPYTFGGGTKLEIK (SEQ ID NO:
108).

QVQLQQPGAVLVKPGASVRMSCKASGYTFPYYNVHWVKRTPGQGLEWIGLIYPDN
GDTSYNQKFTGKATLTADTSSSTAYMQLSSLTSEDSAVYYCARGGLDYWGQGTTLTVSA
(SEQ ID NO: 82).

DVQITQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
109).

EIQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTTLTVSS
(SEQ ID NO: 83).

DIVLTQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIK (SEQ ID NO:
110).

EIQLQQSGAELVKPGASVKMSCKASGYTFSFYNMHWVKRTPGQGLEWIGLIYPDNG
DTSYNQKFKGKATLTADTSSSTAYMQLSSLTSEDSAVYYCARGGLDYWGQGTTLTVSS
(SEQ ID NO: 84).

DIVLTQSPASLSVSVGETVTITCRASENIYSNLAWYQQKQGKSPQLLVYAATNLADG
VPSRFSGSGSGTQYSLKINSLQSEDFGSYYCQHFWGTPYTFGGGTKLELK (SEQ ID NO:
111).

EVQLQQSGADLVKPGASVKMSCKASGYTFSYYNMHWVRRTPGQGLEWIGLIYPDN
GDTSYNQKFKGKATLTADTSSSTAYMQLSSLTSEDSAVYYCARGGLDYWGQGTTLTVSS
(SEQ ID NO: 85).

DIQMIQSPASMSASVGETVTITCGASENIYGALNWYQRKQGKSPQLLIYGATNLADG
MSSRFSGSGSGRQYSLKISSLHPDDVATYYCQNVLSTPYTFGGGTKLEMK (SEQ ID NO:
112).

QVQLQQSGAELVKPGASVKMSCKASGYTFSFYNMHWVKRTPGRGLEWIGLIYPDN
GDTSYNQKFKGKATLTADTSSSTAYMQLSSLTSEDSAVYYCARGGLDYWGQGTSLTVSS
(SEQ ID NO: 86).

EIVLTQSPASLAVSLGQRATISCKASQSVDYDGDSYMNWYQQKPGQPPKLLIYGISN

RFSGVPDRFSGSGSGTDFTLKISTIKPEDLGMYYCLQGTHQPYTFGGGTKLEIK (SEQ ID
NO: 113).

EIQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
(SEQ ID NO: 87).

NIVMTQSHKFMSTSVGDRVSITCKASQDVNTDVAWYQQKPGQSPKLLIYSASYRYT
GVPTRFTGSGSGTDFTFTISGVQAEDLADYYCQQHYNTPYTFGGGTKLELK (SEQ ID NO:
114).

QIQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
(SEQ ID NO: 88).

DIQMTQSPSSMSASLGDRITITCQATQDIVKNLNWYQQKPGKPPSFLIYYATELAEGV
PSRFSGSGSGSDYSLTISNLESEDFADYYCLQFYEFPLTFGAGTKLELK (SEQ ID NO: 115).

QVQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
(SEQ ID NO: 89).

DIVMTQSPASLSASVGETVTITCRASGNIHNYLAWYQQKQGKSPQLLVYNAKTLAD
GVPSRFSGSGSGTQYSLKINSLQPEDFGSYYCQHFWSTPYTFGGGTKLEMK (SEQ ID NO:
116).

According to the embodiments of the present invention, the antibody of the present invention comprises at least one of a heavy chain constant region and a light chain constant region, and at least a portion of at least one of the heavy chain constant region and the light chain constant region is derived from at least one of a murine antibody, a primate antibody or a mutant thereof.

According to the embodiments of the present invention, the heavy chain constant region and light chain constant region of the antibody of the present invention are both derived from human IgG antibodies or mutants thereof.

According to the embodiments of the present invention, the heavy chain constant region of the antibody of the present invention is derived from a human IgG1 or IgG4 antibody or a mutant thereof.

According to the embodiments of the present invention, the antibody light chain constant region of the present invention is derived from a human IgGκ or IgGλ constant region.

According to the embodiments of the present invention, the full-length sequence of the heavy chain constant region of the antibody of the present invention is shown in SEQ ID NO: 117.

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVDK
SRWQQGNVFSCSVMHEALHNHYTQKSLSLSPGK (SEQ ID NO: 117).

The full-length sequence of the antibody heavy chain constant region described above includes the heavy chain constant region sequence (CH1) and the Fc fragment (hinge region, CH2 and CH3).

According to the embodiments of the present invention, the full-length sequence of the light chain constant region of the antibody of the present invention is shown in SEQ ID NO: 118.


 (SEQ ID NO:
118).

According to the embodiments of the present invention, the antibody of the present invention is natural whole antibody, single-chain antibody (scFv), single-chain Fv-Fc antibody (scFv-Fc), scFv dimer and dimer of scFv-Fc antibody. Wherein, the dimer includes homodimer and heterodimer.

The single-chain antibody (scFv) described above comprises a heavy chain variable region having an amino acid sequence shown in any one of SEQ ID NO: 64-89 and a light chain variable region having an amino acid sequence shown in any one of SEQ ID NO: 91-116, wherein the C-terminus of the heavy chain variable region is connected to the N-terminus of the light chain variable region via a connecting peptide linker, or the C-terminus of the variable region is connected to the N-terminus of the heavy chain variable region via a connecting peptide linker.

It should be noted that the "connecting peptide linker" of the single-chain antibody described in the present invention is a connecting peptide used to connect the heavy chain variable region and the light chain variable region of the antibody. It can be a commonly used connecting peptide linker for preparing single-chain antibodies, or it can be a connecting peptide linker modified by scientific researchers. An example of the amino acid sequence of the linker peptide is: (GnS)m, n is a natural number of 3-5, preferably 4, and m is a natural number of 1-5, preferably 3.
GGGS (SEQ ID NO: 119).
GGGGS (SEQ ID NO: 120).
GGGGSGGGGS (SEQ ID NO: 121).
GGGGSGGGGSGGGGS (SEQ ID NO: 122).

According to the embodiments of the present invention, preferably, the single-chain antibody connecting peptide linker of the present invention has an amino acid sequence shown in SEQ ID NO: 122.

The linker peptide described in the present invention has one or more amino acid substitutions compared with SEQ ID NO: 120-122; for example, GGGGSGGGGSGDGGS (SEQ ID NO: 123).

The single-chain Fv-Fc antibody (scFv-Fc) described above comprises an scFv and an Fc fragment, wherein the Fc fragment refers to an antibody heavy chain constant region fragment comprising at least a hinge region, a CH2 domain and a CH3 domain; the scFv is as described above, and the scFv is directly connected to the Fc fragment or connected via a linker peptide. According to the embodiments of the present invention, the scFv and the Fc fragment are directly connected via the hinge region.

The Fc fragment as described above is derived from, for example, a heavy chain constant region selected from the group consisting of human IgG1, IgG2, IgG3, IgG4, IgM, IgA1, IgA2, IgD and IgE; particularly selected from, for example, a heavy chain constant region of human IgG1, IgG2, IgG3 and IgG4, more particularly selected from the heavy chain constant region of human IgG1 or IgG4; and, the Fc fragment has one or more amino acid substitutions, deletions or additions (e.g., at most 20, at most 15, at most 10, at most 5, at most 3 or at most 1 substitution, deletion or addition) compared to the native sequence from which it is derived.

An example of an Fc fragment amino acid sequence is an IgG4 wild-type Fc fragment having the amino acid sequence shown in SEQ ID NO: 124,

ESKYGPPCPSCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSSIEK

TISKAKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK (SEQ ID
NO: 124);

An IgG4 mutant Fc fragment having the amino acid sequence shown in SEQ **ID** NO: 125,
ESKYGPPCPPCPAPEAAGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQPENNYKTT PPVLDSDGSFFLYSKLTVDKSRWQEGNVFSCSVMHEALHNHYTQKSLSLSLG (SEQ **ID** NO: 125), compared with the Fc fragment of wild-type human IgG4, the mutant IgG4 Fc fragment has S10P, F16A, L17A, R191K mutations and 229K deletion mutations, and the amino acid mutation sites are numbered according to the EU numbering system.

According to the embodiments of the present invention, the Fc fragment in the single-chain Fv-Fc antibody (scFv-Fc) of the present invention is preferably an IgG4 mutant Fc fragment, that is, the Fc fragment in the single-chain Fv-Fc antibody (scFv-Fc) of the present invention has the amino acid sequence shown in SEQ **ID** NO: 125.

According to the embodiments of the present invention, in the present invention,
the antibody comprising the heavy chain variable region shown in SEQ **ID** NO: 64 and the light chain variable region shown in SEQ **ID** NO: 106 is referred to as 3C7;
the antibody comprising the heavy chain variable region shown in SEQ **ID** NO: 65 and the light chain variable region shown in SEQ **ID** NO: 92 is referred to as 3E1;
the antibody comprising the heavy chain variable region shown in SEQ **ID** NO: 66 and the light chain variable region shown in SEQ **ID** NO: 93 is referred to as 3A8;
the antibody comprising the heavy chain variable region shown in SEQ **ID** NO: 67 and the light chain variable region shown in SEQ **ID** NO: 94 is referred to as 3G10;
the antibody comprising the heavy chain variable region shown in SEQ **ID** NO: 68 and the light chain variable region shown in SEQ **ID** NO: 95 is referred to as 2B1;
the antibody comprising the heavy chain variable region shown in SEQ **ID** NO: 69 and the light chain variable region shown in SEQ **ID** NO: 96 is referred to as 3A12;
the antibody comprising the heavy chain variable region shown in SEQ **ID** NO: 70 and the light chain variable region shown in SEQ **ID** NO: 97 is referred to as 3E7;
the antibody comprising the heavy chain variable region shown in SEQ **ID** NO: 71 and the light chain variable region shown in SEQ ID NO: 98 is referred to as 3F10;
the antibody comprising the heavy chain variable region shown in SEQ ID NO: 72 and the light chain variable region shown in SEQ ID NO: 99 is referred to as 3H6;
the antibody comprising the heavy chain variable region shown in SEQ ID NO: 73 and the light chain variable region shown in SEQ ID NO: 100 is referred to as 3B7;
the antibody comprising the heavy chain variable region shown in SEQ ID NO: 74 and the light chain variable region shown in SEQ ID NO: 101 is referred to as 3F11;
the antibody comprising the heavy chain variable region shown in SEQ ID NO: 75 and the light chain variable region shown in SEQ ID NO: 102 is referred to as 1B8;
the antibody comprising the heavy chain variable region shown in SEQ ID NO: 76 and the light chain variable region shown in SEQ ID NO: 103 is referred to as 1A3;
the antibody comprising the heavy chain variable region shown in SEQ ID NO: 77 and the light chain variable region shown in SEQ ID NO: 104 is referred to as 2B2;
the antibody comprising the heavy chain variable region shown in SEQ ID NO: 78 and the light chain variable region shown in SEQ ID NO: 105 is referred to as 3A3;
the antibody comprising the heavy chain variable region shown in SEQ ID NO: 79 and the light chain variable region shown in SEQ ID NO: 106 is referred to as 3C9;
the antibody comprising the heavy chain variable region shown in SEQ ID NO: 80 and the light chain variable region shown in SEQ ID NO: 107 is referred to as 3D6;
the antibody comprising the heavy chain variable region shown in SEQ ID NO: 81 and the light chain variable region shown in SEQ ID NO: 108 is referred to as 3E2;
the antibody comprising the heavy chain variable region shown in SEQ ID NO: 82 and the light chain variable region shown in SEQ ID NO: 109 is referred to as 3E3;
the antibody comprising the heavy chain variable region shown in SEQ ID NO: 83 and the light chain variable region shown in SEQ ID NO: 110 is referred to as 3F5;
the antibody comprising the heavy chain variable region shown in SEQ ID NO: 84 and the light chain variable region shown in SEQ ID NO: 111 is referred to as 3H9;
the antibody comprising the heavy chain variable region shown in SEQ ID NO: 85 and the light chain variable region shown in SEQ ID NO: 112 is referred to as 3B4;
the antibody comprising the heavy chain variable region shown in SEQ ID NO: 86 and the light chain variable region shown in SEQ ID NO: 113 is referred to as 3D7;
the antibody comprising the heavy chain variable region shown in SEQ ID NO: 87 and the light chain variable region shown in SEQ ID NO: 114 is referred to as 1G6;
the antibody comprising the heavy chain variable region shown in SEQ ID NO: 88 and the light chain variable region shown in SEQ ID NO: 115 is referred to as 1D7;
the antibody comprising the heavy chain variable region shown in SEQ ID NO: 89 and the light chain variable region shown in SEQ ID NO: 116 is referred to as 1C10.

According to the embodiments of the present invention, in the present invention, the antibody comprising the heavy chain variable region shown in SEQ ID NO: 63 and the light chain variable region shown in SEQ ID NO: 90 is referred to as FR21.

According to the embodiments of the present invention, the antibody of the present invention is a natural complete antibody.

According to the embodiments of the present invention, the antibody of the present invention comprises a heavy chain consisting of a heavy chain variable region having an amino acid sequence with at least 85%, at least 90% or at least 95% identical to any one of SEQ ID NO: 64-89, and a heavy chain constant region.

According to the embodiments of the present invention, the antibody of the present invention comprises a heavy chain consisting of a heavy chain variable region having an amino acid sequence with at least 85%, at least 90% or at least 95% identical to any one of SEQ ID NO: 64-89, and a heavy chain constant region shown in SEQ ID NO: 117.

According to the embodiments of the present invention, the antibody of the present invention comprises a heavy chain consisting of a heavy chain variable region shown in any one of SEQ ID NO: 64-89, and a heavy chain constant region shown in SEQ ID NO: 117.

According to the embodiments of the present invention, the antibody of the present invention comprises a light chain consisting of a light chain variable region having an amino acid sequence with at least 85%, at least 90% or at least 95% identical to any one of SEQ ID NO: 91-116, and a light chain constant region.

According to the embodiments of the present invention, the antibody of the present invention comprises a light chain consisting of a light chain variable region having an amino acid sequence with at least 85%, at least 90% or at least 95% identical to any one of SEQ ID NO: 91-116, and a light chain constant region shown in SEQ ID NO: 118.

According to the embodiments of the present invention, the antibody of the present invention comprises a light chain consisting of a light chain variable region shown in any one of SEQ ID NO: 91-116, and a light chain constant region shown in SEQ ID NO: 118.

According to the embodiments of the present invention, the antibody of the present invention comprises
(a) a heavy chain consisting of a heavy chain variable region having an amino acid sequence with at least 85%, at least 90% or at least 95% identical to SEQ ID NO: 64, and a heavy chain constant region. And a light chain consisting of a light chain variable region having an amino acid sequence with at least 85%, at least 90% or at least 95% identical to SEQ ID NO: 106, and a light chain constant region; or
(b) a heavy chain consisting of a heavy chain variable region having an amino acid sequence with at least 85%, at least 90% or at least 95% identical to SEQ ID NO: 65, and a heavy chain constant region. And a light chain consisting of a light chain variable region having an amino acid sequence with at least 85%, at least 90% or at least 95% identical to SEQ ID NO: 92, and a light chain constant region; or
(c) a heavy chain consisting of a heavy chain variable region having an amino acid sequence with at least 85%, at least 90% or at least 95% identical to SEQ ID NO: 66, and a heavy chain constant region. And a light chain consisting of a light chain variable region having an amino acid sequence with at least 85%, at least 90% or at least 95% identical to SEQ ID NO: 93, and a light chain constant region; or
(d) a heavy chain consisting of a heavy chain variable region having an amino acid sequence with at least 85%, at least 90% or at least 95% identical to SEQ ID NO: 67, and a heavy chain constant region. And a light chain consisting of a light chain variable region having an amino acid sequence with at least 85%, at least 90% or at least 95% identical to SEQ ID NO: 94, and a light chain constant region; or
(e) a heavy chain consisting of a heavy chain variable region having an amino acid sequence with at least 85%, at least 90% or at least 95% identical to SEQ ID NO: 68, and a heavy chain constant region. And a light chain consisting of a light chain variable region having an amino acid sequence with at least 85%, at least 90% or at least 95% identical to SEQ ID NO: 95, and a light chain constant region; or
(f) a heavy chain consisting of a heavy chain variable region having an amino acid sequence with at least 85%, at least 90% or at least 95% identical to SEQ ID NO: 69, and a heavy chain constant region. And a light chain consisting of a light chain variable region having an amino acid sequence with at least 85%, at least 90% or at least 95% identical to SEQ ID NO: 96, and a light chain constant region; or
(g) a heavy chain consisting of a heavy chain variable region having an amino acid sequence with at least 85%, at least 90% or at least 95% identical to SEQ ID NO: 70, and a heavy chain constant region. And a light chain consisting of a light chain variable region having an amino acid sequence with at least 85%, at least 90% or at least 95% identical to SEQ ID NO: 97, and a light chain constant region; or
(h) a heavy chain consisting of a heavy chain variable region having an amino acid sequence with at least 85%, at least 90% or at least 95% identical to SEQ ID NO: 71, and a heavy chain constant region. And a light chain consisting of a light chain variable region having an amino acid sequence with at least 85%, at least 90% or at least 95% identical to SEQ ID NO: 98, and a light chain constant region; or
(i) a heavy chain consisting of a heavy chain variable region having an amino acid sequence with at least 85%, at least 90% or at least 95% identical to SEQ ID NO: 72, and a heavy chain constant region. And a light chain consisting of a light chain variable region having an amino acid sequence with at least 85%, at least 90% or at least 95% identical to SEQ ID NO: 99, and a light chain constant region; or
(j) a heavy chain consisting of a heavy chain variable region having an amino acid sequence with at least 85%, at least 90% or at least 95% identical to SEQ ID NO: 73, and a heavy chain constant region. And a light chain consisting of a light chain variable region having an amino acid sequence with at least 85%, at least 90% or at least 95% identical to SEQ ID NO: 100, and a light chain constant region; or
(k) a heavy chain consisting of a heavy chain variable region having an amino acid sequence with at least 85%, at least 90% or at least 95% identical to SEQ ID NO: 74, and a heavy chain constant region. And a light chain consisting of a light chain variable region having an amino acid sequence with at least 85%, at least 90% or at least 95% identical to SEQ ID NO: 101, and a light chain constant region; or
(l) a heavy chain consisting of a heavy chain variable region having an amino acid sequence with at least 85%, at least 90% or at least 95% identical to SEQ ID NO: 75, and a heavy chain constant region. And a light chain consisting of a light chain variable region having an amino acid sequence with at least 85%, at least 90% or at least 95% identical to SEQ ID NO: 102, and a light chain constant region; or
(m) a heavy chain consisting of a heavy chain variable region having an amino acid sequence with at least 85%, at least 90% or at least 95% identical to SEQ ID NO: 76, and a heavy chain constant region. And a light chain consisting of a light chain variable region having an amino acid sequence with at least 85%, at least 90% or at least 95% identical to SEQ ID NO: 103, and a light chain constant region; or
(n) a heavy chain consisting of a heavy chain variable region having an amino acid sequence with at least 85%, at least 90% or at least 95% identical to SEQ ID NO: 77, and a heavy chain constant region. And a light chain consisting of a light chain variable region having an amino acid sequence with at least 85%, at least 90% or at least 95% identical to SEQ ID NO: 104, and a light chain constant region; or
(o) a heavy chain consisting of a heavy chain variable region having an amino acid sequence with at least 85%, at least 90% or at least 95% identical to SEQ ID NO: 78, and a heavy chain constant region. And a light chain consisting of a light chain variable region having an amino acid sequence with at least 85%, at least 90% or at least 95% identical to SEQ ID NO: 105, and a light chain constant region; or
(p) a heavy chain consisting of a heavy chain variable region having an amino acid sequence with at least 85%, at least 90% or at least 95% identical to SEQ ID NO: 79, and a heavy chain constant region. And a light chain consisting of a light chain variable region having an amino acid sequence with at least 85%, at least 90% or at least 95% identical to SEQ ID NO: 106, and a light chain constant region; or
(q) a heavy chain consisting of a heavy chain variable region having an amino acid sequence with at least 85%, at least 90% or at least 95% identical to SEQ ID NO: 80, and a heavy chain constant region. And a light chain consisting of a light chain variable region having an amino acid sequence with at least 85%, at least 90% or at least 95% identical to SEQ ID NO: 107, and a light chain constant region; or
(r) a heavy chain consisting of a heavy chain variable region having an amino acid sequence with at least 85%, at least 90% or at least 95% identical to SEQ ID NO: 81, and a heavy chain constant region. And a light chain consisting of a light chain variable region having an amino acid sequence with at least 85%, at least 90% or at least 95% identical to SEQ ID NO: 108, and a light chain constant region; or
(s) a heavy chain consisting of a heavy chain variable region having an amino acid sequence with at least 85%, at least 90% or at least 95% identical to SEQ ID NO: 82, and a heavy chain constant region. And a light chain consisting of a light chain variable region having an amino acid sequence with at least 85%, at least 90% or at least 95% identical to SEQ ID NO: 109, and a light chain constant region; or
(t) a heavy chain consisting of a heavy chain variable region having an amino acid sequence with at least 85%, at least 90% or at least 95% identical to SEQ ID NO: 83, and a heavy chain constant region. And a light chain consisting of a light chain variable region having an amino acid sequence with at least 85%, at least 90% or at least 95% identical to SEQ ID NO: 110, and a light chain constant region; or
(u) a heavy chain consisting of a heavy chain variable region having an amino acid sequence with at least 85%, at least 90% or at least 95% identical to SEQ ID NO: 84, and a heavy chain constant region. And a light chain consisting of a light chain variable region having an amino acid sequence with at least 85%, at least 90% or at least 95% identical to SEQ ID NO: 111, and a light chain constant region; or
(v) a heavy chain consisting of a heavy chain variable region having an amino acid sequence with at least 85%, at least 90% or at least 95% identical to SEQ ID NO: 85, and a heavy chain constant region. And a light chain consisting of a light chain variable region having an amino acid sequence with at least 85%, at least 90% or at least 95% identical to SEQ ID NO: 112, and a light chain constant region; or
(w) a heavy chain consisting of a heavy chain variable region having an amino acid sequence with at least 85%, at least 90% or at least 95% identical to SEQ ID NO: 86, and a heavy chain constant region. And a light chain consisting of a light chain variable region having an amino acid sequence with at least 85%, at least 90% or at least 95% identical to SEQ ID NO: 113, and a light chain constant region; or
(x) a heavy chain consisting of a heavy chain variable region having an amino acid sequence with at least 85%, at least 90% or at least 95% identical to SEQ ID NO: 87, and a heavy chain constant region. And a light chain consisting of a light chain variable region having an amino acid sequence with at least 85%, at least 90% or at least 95% identical to SEQ ID NO: 114, and a light chain constant region; or
(y) a heavy chain consisting of a heavy chain variable region having an amino acid sequence with at least 85%, at least 90% or at least 95% identical to SEQ ID NO: 88, and a heavy chain constant region. And a light chain consisting of a light chain variable region having an amino acid sequence with at least 85%, at least 90% or at least 95% identical to SEQ ID NO: 115, and a light chain constant region; or
(z) a heavy chain consisting of a heavy chain variable region having an amino acid sequence with at least 85%, at least 90% or at least 95% identical to SEQ ID NO: 89, and a heavy chain constant region. And a light chain consisting of a light chain variable region having an amino acid sequence with at least 85%, at least 90% or at least 95% identical to SEQ ID NO: 116, and a light chain constant region.

According to the embodiments of the present invention, the antibody of the present invention comprises
(a) a heavy chain consisting of a heavy chain variable region shown in SEQ ID NO:64 and a heavy chain constant region, a light chain consisting of a light chain variable region shown in SEQ ID NO:106 and a light chain constant region; or
(b) a heavy chain consisting of a heavy chain variable region shown in SEQ ID NO:65 and a heavy chain constant region, a light chain consisting of a light chain variable region shown in SEQ ID NO:92 and a light chain constant region; or
(c) a heavy chain consisting of a heavy chain variable region shown in SEQ ID NO:66 and a heavy chain constant region, a light chain consisting of a light chain variable region shown in SEQ ID NO:93 and a light chain constant region; or
(d) a heavy chain consisting of a heavy chain variable region shown in SEQ ID NO:67 and a heavy chain constant region, a light chain consisting of a light chain variable region shown in SEQ ID NO:94 and a light chain constant region; or
(e) a heavy chain consisting of a heavy chain variable region shown in SEQ ID NO:68 and a heavy chain constant region, a light chain consisting of a light chain variable region shown in SEQ ID NO:95 and a light chain constant region; or
(f) a heavy chain consisting of a heavy chain variable region shown in SEQ ID NO:69 and a heavy chain constant region, a light chain consisting of a light chain variable region shown in SEQ ID NO:96 and a light chain constant region; or
(g) a heavy chain consisting of a heavy chain variable region shown in SEQ ID NO:70 and a heavy chain constant region, a light chain consisting of a light chain variable region shown in SEQ ID NO:97 and a light chain constant region; or
(h) a heavy chain consisting of a heavy chain variable region shown in SEQ ID NO:71 and a heavy chain constant region, a light chain consisting of a light chain variable region shown in SEQ ID NO:98 and a light chain constant region; or
(i) a heavy chain consisting of a heavy chain variable region shown in SEQ ID NO:72 and a heavy chain constant region, a light chain consisting of a light chain variable region shown in SEQ ID NO:99 and a light chain constant region; or
(j) a heavy chain consisting of a heavy chain variable region shown in SEQ ID NO:73 and a heavy chain constant region, a light chain consisting of a light chain variable region shown in SEQ ID NO:100 and a light chain constant region; or
(k) a heavy chain consisting of a heavy chain variable region shown in SEQ ID NO:74 and a heavy chain constant region, a light chain consisting of a light chain variable region shown in SEQ ID NO:101 and a light chain constant region; or
(l) a heavy chain consisting of a heavy chain variable region shown in SEQ ID NO:75 and a heavy chain constant region, a light chain consisting of a light chain variable region shown in SEQ ID NO:102 and a light chain constant region; or
(m) a heavy chain consisting of a heavy chain variable region shown in SEQ ID NO:76 and a heavy chain constant region, a light chain consisting of a light chain variable region shown in SEQ ID NO:103 and a light chain constant region; or
(n) a heavy chain consisting of a heavy chain variable region shown in SEQ ID NO:77 and a heavy chain constant region, a light chain consisting of a light chain variable region shown in SEQ ID NO:104 and a light chain constant region; or
(o) a heavy chain consisting of a heavy chain variable region shown in SEQ ID NO:78 and a heavy chain constant region, a light chain consisting of a light chain variable region shown in SEQ ID NO:105 and a light chain constant region; or
(p) a heavy chain consisting of a heavy chain variable region shown in SEQ ID NO:79 and a heavy chain constant region, a light chain consisting of a light chain variable region shown in SEQ ID NO:106 and a light chain constant region; or
(q) a heavy chain consisting of a heavy chain variable region shown in SEQ ID NO:80 and a heavy chain constant region, a light chain consisting of a light chain variable region shown in SEQ ID NO:107 and a light chain constant region; or
(r) a heavy chain consisting of a heavy chain variable region shown in SEQ ID NO:81 and a heavy chain constant region, a light chain consisting of a light chain variable region shown in SEQ ID NO:108 and a light chain constant region; or
(s) a heavy chain consisting of a heavy chain variable region shown in SEQ ID NO:82 and a heavy chain constant region, a light chain consisting of a light chain variable region shown in SEQ ID NO:109 and a light chain constant region; or
(t) a heavy chain consisting of a heavy chain variable region shown in SEQ ID NO:83 and a heavy chain constant region, a light chain consisting of a light chain variable region shown in SEQ ID NO:110 and a light chain constant region; or
(u) a heavy chain consisting of a heavy chain variable region shown in SEQ ID NO:84 and a heavy chain constant region, a light chain consisting of a light chain variable region shown in SEQ ID NO:111 and a light chain constant region; or
(v) a heavy chain consisting of a heavy chain variable region shown in SEQ ID NO:85 and a heavy chain constant region, a light chain consisting of a light chain variable region shown in SEQ ID NO:112 and a light chain constant region; or
(w) a heavy chain consisting of a heavy chain variable region shown in SEQ ID NO:86 and a heavy chain constant region, a light chain consisting of a light chain variable region shown in SEQ ID NO:113 and a light chain constant region; or
(x) a heavy chain consisting of a heavy chain variable region shown in SEQ ID NO:87 and a heavy chain constant region, a light chain consisting of a light chain variable region shown in SEQ ID NO:114 and a light chain constant region; or
(y) a heavy chain consisting of a heavy chain variable region shown in SEQ ID NO:88 and a heavy chain constant region, a light chain consisting of a light chain variable region shown in SEQ ID NO:115 and a light chain constant region; or
(z) a heavy chain consisting of a heavy chain variable region shown in SEQ ID NO:89 and a heavy chain constant region, a light chain consisting of a light chain variable region shown in SEQ ID NO:116 and a light chain constant region.

According to the embodiments of the present invention, the antibody of the present invention comprises
(a) a heavy chain consisting of a heavy chain variable region shown in SEQ ID NO: 64 and a heavy chain constant region shown in SEQ ID NO: 117, a light chain consisting of a light chain variable region shown in SEQ ID NO: 106 and a light chain constant region shown in SEQ ID NO: 118; or
(b) a heavy chain consisting of a heavy chain variable region shown in SEQ ID NO: 65 and a heavy chain constant region shown in SEQ ID NO: 117, a light chain consisting of a light chain variable region shown in SEQ ID NO: 92 and a light chain constant region shown in SEQ ID NO: 118; or
(c) a heavy chain consisting of a heavy chain variable region shown in SEQ ID NO: 66 and a heavy chain constant region shown in SEQ ID NO: 117, a light chain consisting of a light chain variable region shown in SEQ ID NO: 93 and a light chain constant region shown in SEQ ID NO: 118; or
(d) a heavy chain consisting of a heavy chain variable region shown in SEQ ID NO: 67 and a heavy chain constant region shown in SEQ ID NO: 117, a light chain consisting of a light chain variable region shown in SEQ ID NO: 94 and a light chain constant region shown in SEQ ID NO: 118; or
(e) a heavy chain consisting of a heavy chain variable region shown in SEQ ID NO: 68 and a heavy chain constant region shown in SEQ ID NO: 117, a light chain consisting of a light chain variable region shown in SEQ ID NO: 95 and a light chain constant region shown in SEQ ID NO: 118; or
(f) a heavy chain consisting of a heavy chain variable region shown in SEQ ID NO: 69 and a heavy chain constant region shown in SEQ ID NO: 117, a light chain consisting of a light chain variable region shown in SEQ ID NO: 96 and a light chain constant region shown in SEQ ID NO: 118; or
(g) a heavy chain consisting of a heavy chain variable region shown in SEQ ID NO: 70 and a heavy chain constant region shown in SEQ ID NO: 117, a light chain consisting of a light chain variable region shown in SEQ ID NO: 97 and a light chain constant region shown in SEQ ID NO: 118; or
(h) a heavy chain consisting of a heavy chain variable region shown in SEQ ID NO: 71 and a heavy chain constant region shown in SEQ ID NO: 117, a light chain consisting of a light chain variable region shown in SEQ ID NO: 98 and a light chain constant region shown in SEQ ID NO: 118; or
(i) a heavy chain consisting of a heavy chain variable region shown in SEQ ID NO: 72 and a heavy chain constant region shown in SEQ ID NO: 117, a light chain consisting of a light chain variable region shown in SEQ ID NO: 99 and a light chain constant region shown in SEQ ID NO: 118; or
(j) a heavy chain consisting of a heavy chain variable region shown in SEQ ID NO: 73 and a heavy chain constant region shown in SEQ ID NO: 117, a light chain consisting of a light chain variable region shown in SEQ ID NO: 100 and a light chain constant region shown in SEQ ID NO: 118; or
(k) a heavy chain consisting of a heavy chain variable region shown in SEQ ID NO: 74 and a heavy chain constant region shown in SEQ ID NO: 117, a light chain consisting of a light chain variable region shown in SEQ ID NO: 101 and a light chain constant region shown in SEQ ID NO: 118; or
(l) a heavy chain consisting of a heavy chain variable region shown in SEQ ID NO: 75 and a heavy chain constant region shown in SEQ ID NO: 117, a light chain consisting of a light chain variable region shown in SEQ ID NO: 102 and a light chain constant region shown in SEQ ID NO: 118; or
(m) a heavy chain consisting of a heavy chain variable region shown in SEQ ID NO: 76 and a heavy chain constant region shown in SEQ ID NO: 117, a light chain consisting of a light chain variable region shown in SEQ ID NO: 103 and a light chain constant region shown in SEQ ID NO: 118; or
(n) a heavy chain consisting of a heavy chain variable region shown in SEQ ID NO: 77 and a heavy chain constant region shown in SEQ ID NO: 117, a light chain consisting of a light chain variable region shown in SEQ ID NO: 104 and a light chain constant region shown in SEQ ID NO: 118; or
(o) a heavy chain consisting of a heavy chain variable region shown in SEQ ID NO: 78 and a heavy chain constant region shown in SEQ ID NO: 117, a light chain consisting of a light chain variable region shown in SEQ ID NO: 105 and a light chain constant region shown in SEQ ID NO: 118; or
(p) a heavy chain consisting of a heavy chain variable region shown in SEQ ID NO: 79 and a heavy chain constant region shown in SEQ ID NO: 117, a light chain consisting of a light chain variable region shown in SEQ ID NO: 106 and a light chain constant region shown in SEQ ID NO: 118; or
(q) a heavy chain consisting of a heavy chain variable region shown in SEQ ID NO: 80 and a heavy chain constant region shown in SEQ ID NO: 117, a light chain consisting of a light chain variable region shown in SEQ ID NO: 107 and a light chain constant region shown in SEQ ID NO: 118; or
(r) a heavy chain consisting of a heavy chain variable region shown in SEQ ID NO: 81 and a heavy chain constant region shown in SEQ ID NO: 117, a light chain consisting of a light chain variable region shown in SEQ ID NO: 108 and a light chain constant region shown in SEQ ID NO: 118; or
(s) a heavy chain consisting of a heavy chain variable region shown in SEQ ID NO: 82 and a heavy chain constant region shown in SEQ ID NO: 117, a light chain consisting of a light chain variable region shown in SEQ ID NO: 109 and a light chain constant region shown in SEQ ID NO: 118; or
(t) a heavy chain consisting of a heavy chain variable region shown in SEQ ID NO: 83 and a heavy chain constant region shown in SEQ ID NO: 117, a light chain consisting of a light chain variable region shown in SEQ ID NO: 110 and a light chain constant region shown in SEQ ID NO: 118; or
(u) a heavy chain consisting of a heavy chain variable region shown in SEQ ID NO: 84 and a heavy chain constant region shown in SEQ ID NO: 117, a light chain consisting of a light chain variable region shown in SEQ ID NO: 111 and a light chain constant region shown in SEQ ID NO: 118; or
(v) a heavy chain consisting of a heavy chain variable region shown in SEQ ID NO: 85 and a heavy chain constant region shown in SEQ ID NO: 117, a light chain consisting of a light chain variable region shown in SEQ ID NO: 112 and a light chain constant region shown in SEQ ID NO: 118; or
(w) a heavy chain consisting of a heavy chain variable region shown in SEQ ID NO: 86 and a heavy chain constant region shown in SEQ ID NO: 117, a light chain consisting of a light chain variable region shown in SEQ ID NO: 113 and a light chain constant region shown in SEQ ID NO: 118; or
(x) a heavy chain consisting of a heavy chain variable region shown in SEQ ID NO: 87 and a heavy chain constant region shown in SEQ ID NO: 117, a light chain consisting of a light chain variable region shown in SEQ ID NO: 114 and a light chain constant region shown in SEQ ID NO: 118; or
(y) a heavy chain consisting of a heavy chain variable region shown in SEQ ID NO: 88 and a heavy chain constant region shown in SEQ ID NO: 117, a light chain consisting of a light chain variable region shown in SEQ ID NO: 115 and a light chain constant region shown in SEQ ID NO: 118; or
(z) a heavy chain consisting of a heavy chain variable region shown in SEQ ID NO: 89 and a heavy chain constant region shown in SEQ ID NO: 117, a light chain consisting of a light chain variable region shown in SEQ ID NO: 116 and a light chain constant region shown in SEQ ID NO: 118.

According to the embodiments of the present invention, the heavy chain and light chain of the present invention have the amino acid sequences shown below.

QVQLVQSGAEVKKPGSSVKVSCKASGYIFTTYNVHWVRQAPGQGLEWIGSIYPDNG
DTSYNQNFKGRATITADKSTSTAYMELSSLRSEDTAVYYCARGDFAYWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 131).

DIQMTQSPSSLSASVGDRVTITCKASQGVSNDVAWYQQKPGKAPKLLIYSASYRYTG
VPSRFSGSGSGTDFTFTISSLQPEDIATYYCQQHSTTPYTFGQGTKLEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO: 132).

The above SEQ ID NO: 131 and SEQ ID NO: 132 are the amino acid sequences of the heavy chain and light chain of FR21.

EVQLQQSGAELVKPGASVKMSCKASGYTFSFYNVHWVRQTPGQGLEWIGLIYPDN
GDTSYNQKFKAKATLTADKSSSAAYMQLSSLTSEDSAVYYCARGGFDYWGQGTSLTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS

TYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 133).

DIVMTQSHKFMSTSVGDRVNITCKASQDVSTAVAWYQQKPGQSPKLLIYSASYRYSG
VPDRFTGSGSGTDFTFTISSVQAEDLAVYYCQQHYSTPYTFGGGTKLEMKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 134).

The above SEQ ID NO: 133 and SEQ ID NO: 134 are the amino acid sequences of the heavy chain and light chain of 3A12.

QVQLQQSGAELVKPGASVKMSCKASGYTFAPYNIHWVRQTPGQGLEWIGLIYPDN
GDTSYNQKFKAKATLTADKSSSTAYMQLSSLTSEDSAVYYCARGGFDYWGQGTSLTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 135).

DIVMTQSQKFMSTSVGDRVSVTCKASQNVGTNVAWYQQKPGQSPKALIYSASYRYS
GVPDRFTGSGSGTDFTLTISNVQSEDLAEYFCQQHYSTPFTFGSGTKLEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 136).

The above SEQ ID NO: 135 and SEQ ID NO: 136 are the amino acid sequences of the heavy chain and light chain of 3F 11.

EIQLQQSGAELVKPGASVKMSCKASGYIFRYYNMHWVKRTPGQGLEWIGLIYPDNG
DTSYNQKFKGKATLTADTSSSTAYMQLSSLTSEDSAVYYCARGGLDYWGQGTTLTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 137).

DIQMIQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 138).

The above SEQ ID NO: 137 and SEQ ID NO: 138 are the amino acid sequences of the heavy chain and light chain of 3C9.

QVQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 139).

DIQMTQSPASLSVSVGETVTITCRASENIYSNLAWYQQKQGKSPQLLVYNAKTLADG
VPSRFSGSGSGTQYSLKINSLQPEDFGSYYCQHFWSTPFTFGSGTKLELKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 140).

The above SEQ ID NO: 139 and SEQ ID NO: 140 are the amino acid sequences of the heavy chain and light chain of 3D6.

EIQLQQSGAELVKPGASVKMSCKASGYTFSFYNMHWVKRTPGQGLEWIGLIYPDNG
DTSYNQKFKGKATLTADTSSSTAYMQLSSLTSEDSAVYYCARGGLDYWGQGTSLTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 141).

NIVMTQSQKFMSTSVGDRVSVTCKASQNVGTNVAWYQQKPGQSPKALIYSASYRYT
GVPDRFTGSGSGTDFTFTISSVQAEDLAVYYCQQHYSTPYTFGGGTKLEIKRTVAAPSVFI
FPPSDEQLKSGTASVVCLLNNFYPREAKVQWKVDNALQSGNSQESVTEQDSKDSTYSLS

STLTLSKADYEKHKVYACEVTHQGLSSPVTKSFNRGEC (SEQ ID NO: 142).

The above SEQ ID NO: 141 and SEQ ID NO: 142 are the amino acid sequences of the heavy chain and light chain of 3E2.

EIQLQQSGAELVKPGASVKMSCKASGYTFSFYNMHWVKRTPGQGLEWIGLIYPDNG
DTSYNQKFKGKATLTADTSSSTAYMQLSSLTSEDSAVYYCARGGLDYWGQGTTLTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSVMHEALHNHYTQKSLSLSPGK (SEQ ID NO: 143).

DIVLTQSPASLSVSVGETVTITCRASENIYSNLAWYQQKQGKSPQLLVYAATNLADG
VPSRFSGSGSGTQYSLKINSLQSEDFGSYYCQHFWGTPYTFGGGTKLELKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 144).

The above SEQ ID NO: 143 and SEQ ID NO: 144 are the amino acid sequences of the heavy chain and light chain of 3H9.

EIQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 145).

NIVMTQSHKFMSTSVGDRVSITCKASQDVNTDVAWYQQKPGQSPKLLIYSASYRYT
GVPTRFTGSGSGTDFTFTISGVQAEDLADYYCQQHYNTPYTFGGGTKLELKRTVAAPSVF
IFPPSDEQLKSGTASVVCLLNNFYPREAKVQWKVDNALQSGNSQESVTEQDSKDSTYSLS
STLTLSKADYEKHKVYACEVTHQGLSSPVTKSFNRGEC (SEQ ID NO: 146).

The above SEQ ID NO: 145 and SEQ ID NO: 146 are the amino acid sequences of the heavy chain and light chain of 1G6.

QIQLQQSGAELVKPGASVKMSCKASGYTFRYYNMHWVKRTPGQGLEWIGLIYPDN
GDTSYNQKFKGKATLTADTSSSTAYMQLSSLTSEDSAVYYCARGGLDYWGQGTSLTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO: 147).

DIQMTQSPSSMSASLGDRITITCQATQDIVKNLNWYQQKPGKPPSFLIYYATELAEGV
PSRFSGSGSGSDYSLTISNLESEDFADYYCLQFYEFPLTFGAGTKLELKRTVAAPSVFIFPPS
DEQLKSGTASVVCLLNNFYPREAKVQWKVDNALQSGNSQESVTEQDSKDSTYSLSSTLT
LSKADYEKHKVYACEVTHQGLSSPVTKSFNRGEC (SEQ ID NO: 148).

The above SEQ ID NO: 147 and SEQ ID NO: 148 are the amino acid sequences of the heavy chain and light chain of 1D7.

EIQLQQSGAELVKPGASVKMSCKASGYIFRNYYIHWVKRTPGQGLEWIGAIYPDNG
DTSYNQRFKVKATLTADTSSSTAYMQLSSLTSEDSAVYYCARAFTYWGQGTTLTVSSAST
KGPSVFPLAPSSKSTSGGTAALGCLVKDYFPEPVTVSWNSGALTSGVHTFPAVLQSSGLYS
LSSVVTVPSSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPPSREEMTK
NQVSLTCLVKGFYPSDIAVEWESNGQPENNYKTTPPVLDSDGSFFLYSKLTVDKSRWQQG
NVFSCSVMHEALHNHYTQKSLSLSPGK (SEQ ID NO: 198)

DIQMIQSPASLSASVGETVTITCGASENIYGALNWYQRKQGKSPQLLIYGATNLADG
MSSRFSGSGSGRQYSLKISSLHPDDVATYYCQNVLSTPYTFGGGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC (SEQ ID NO: 138)

The above SEQ ID NO: 198 and SEQ ID NO: 138 are the amino acid sequences of the heavy chain and light chain of 3C7.

In the second aspect of the present invention, the present invention provides a humanized antibody or an antigen-binding fragment thereof, wherein the humanized antibody or the antigen-binding fragment thereof is derived from the chimeric antibody or the antigen-binding fragment thereof described above.

According to the embodiments of the present invention, the humanized antibody or antigen-binding fragment thereof, wherein the antibody comprises a heavy chain variable region and a light chain variable region;
the heavy chain variable region comprises: VH-CDR1, VH-CDR2 and VH-CDR3,
the VH-CDR1 has an amino acid sequence shown in SEQ ID NO: 2,
the VH-CDR2 has an amino acid sequence shown in SEQ ID NO: 149,
the VH-CDR3 has an amino acid sequence shown in SEQ ID NO: 26; and
the light chain variable region comprises: VL-CDR1, VL-CDR2 and VL-CDR3,
the VL-CDR1 has an amino acid sequence shown in X36ASENIYGALN, wherein X36 is selected from G or R,
the VL-CDR2 has an amino acid sequence shown in SEQ ID NO: 40,
the VL-CDR3 has an amino acid sequence shown in SEQ ID NO: 48.

According to the embodiments of the present invention, in the humanized antibody or antigen-binding fragment thereof, the antibody comprises a heavy chain variable region and a light chain variable region;
the heavy chain variable region comprises: VH-CDR1, VH-CDR2 and VH-CDR3,
the VH-CDR1 has an amino acid sequence shown in SEQ ID NO: 2,
the VH-CDR2 has an amino acid sequence shown in SEQ ID NO: 149,
the VH-CDR3 has an amino acid sequence shown in SEQ ID NO: 26; and
the light chain variable region comprises: VL-CDR1, VL-CDR2 and VL-CDR3,
the VL-CDR1 has an amino acid sequence shown in SEQ ID NO: 28 or SEQ ID NO: 150,
the VL-CDR2 has an amino acid sequence shown in SEQ ID NO: 40,
the VL-CDR2 has an amino acid sequence shown in SEQ ID NO: 48.

The CDR region of the 3C7 humanized antibody has the following amino acid sequence:
HCDR1: NYYIH (SEQ ID NO: 2),
HCDR2: AIYPDNGDTSYNQRFKV (SEQ ID NO: 149),
HCDR3: AFTY (SEQ ID NO: 26),
LCDR1: RASENIYGALN (SEQ ID NO: 150),
LCDR1: GASENIYGALN (SEQ ID NO: 28),
LCDR2: GATNLAD (SEQ ID NO: 40),
LCDR3: QNVLSTPYT (SEQ ID NO: 48).

According to the embodiments of the present invention, the humanized antibody or antigen-binding fragment thereof, wherein the antibody comprises a heavy chain variable region and a light chain variable region, the heavy chain variable region comprises a heavy chain framework region, and the light chain variable region comprises a light chain framework region;
the heavy chain framework region comprises: H-FR1, H-FR2, H-FR3 and H-FR4,
the H-FR1 has an amino acid sequence shown in QVQLVQSGAEVKKPGASVKVSCKASGYX37FX38, wherein X37 is selected from T or I, X38 is selected from T or R,
the H-FR2 has an amino acid sequence shown in WVX39QAPGQGLEWMG, wherein X39 is selected from R or K,
the H-FR3 has an amino acid sequence shown in SEQ ID NO: 156,
the H-FR4 has an amino acid sequence shown in SEQ ID NO: 157; and
the light chain framework region comprises: L-FR1, L-FR2, L-FR3 and L-FR4,
the L-FR1 has an amino acid sequence shown in SEQ ID NO: 158,
the L-FR2 has an amino acid sequence shown in WYQX40KPGKX41PKLLIY, wherein X40 is selected from Q or R, X41 is selected from A or S,
the L-FR3 has an amino acid sequence shown in GVPSRFSGSGSGTDX42TLTISSLQPEDX43ATYYC, wherein X42 is selected from F or Y, X43 is selected from F or V,
the L-FR4 has an amino acid sequence shown in FGGGTKX44EIK, wherein X44 is selected from V or L.

According to the embodiments of the present invention, the humanized antibody or antigen-binding fragment thereof, wherein the antibody comprises a heavy chain variable region and a light chain variable region, the heavy chain variable region comprises a heavy chain framework region, and the light chain variable region comprises a light chain framework region;
the heavy chain framework region comprises: H-FR1, H-FR2, H-FR3 and H-FR4,
the H-FR1 has an amino acid sequence shown in SEQ ID NO: 151, SEQ ID NO: 152 or SEQ ID NO: 153,
the H-FR2 has an amino acid sequence shown in SEQ ID NO: 154 or SEQ ID NO: 155,
the H-FR3 has an amino acid sequence shown in SEQ ID NO: 156,
the H-FR4 has an amino acid sequence shown in SEQ **ID** NO: 157; and
the light chain framework region comprises: L-FR1, L-FR2, L-FR3 and L-FR4,
the L-FR1 has an amino acid sequence shown in SEQ **ID** NO: 158,
the L-FR2 has an amino acid sequence shown in SEQ **ID** NO: 159, SEQ **ID** NO: 160 or SEQ **ID** NO: 161,
the L-FR3 has an amino acid sequence shown in SEQ **ID** NO: 162, SEQ **ID** NO: 163 or SEQ **ID** NO: 164,
the L-FR4 has an amino acid sequence shown in SEQ **ID** NO: 165 or SEQ **ID** NO: 166.

The framework region of the 3C7 humanized antibody has the following amino acid sequence:
H-FR1:
   QVQLVQSGAEVKKPGASVKVSCKASGYTFT (SEQ **ID** NO: 151),
   QVQLVQSGAEVKKPGASVKVSCKASGYIFT (SEQ **ID** NO: 152),
   QVQLVQSGAEVKKPGASVKVSCKASGYIFR (SEQ **ID** NO: 153),
H-FR2:
   WVRQAPGQGLEWMG (SEQ **ID** NO: 154),
   WVKQAPGQGLEWMG (SEQ **ID** NO: 155),
H-FR3:
   RATLTADTSTSTVYMELSSLRSEDTAVYYCAR (SEQ **ID** NO: 156),
H-FR4:
   WGQGTTVTVSS (SEQ **ID** NO: 157),
L-FR1:
   DIQMTQSPSSLSASVGDRVTITC (SEQ **ID** NO: 158),
L-FR2:
   WYQQKPGKAPKLLIY (SEQ **ID** NO: 159),
   WYQRKPGKAPKLLIY (SEQ **ID** NO: 160),
   WYQRKPGKSPKLLIY (SEQ **ID** NO: 161),
L-FR3:
   GVPSRFSGSGSGTDFTLTISSLQPEDFATYYC (SEQ **ID** NO: 162),
   GVPSRFSGSGSGTDYTLTISSLQPEDVATYYC (SEQ **ID** NO: 163),
   GVPSRFSGSGSGTDYTLTISSLQPEDFATYYC (SEQ **ID** NO: 164),
L-FR4:
   FGGGTKVEIK (SEQ ID NO: 165),
   FGGGTKLEIK (SEQ ID NO: 166).

According to the embodiments of the present invention, the humanized antibody or antigen-binding fragment thereof, wherein, the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to any one of SEQ ID NO: 167-170.

According to the embodiments of the present invention, the humanized antibody or antigen-binding fragment thereof, wherein, the antibody comprises a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to any one of SEQ ID NO: 171-174.

According to the embodiments of the present invention, the humanized antibody or antigen-binding fragment thereof, wherein, the antibody comprises
(a) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 167 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 171; or
(b) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 167 and a light chain variable region having an amino acid sequence with at least 85% identical to SEQ ID NO: 172; or
(c) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 167 and a light chain variable region having an amino acid sequence with at least 85% identical to SEQ ID NO: 173; or
(d) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 167 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 174; or
(e) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 168 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 171; or
(f) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 168 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 172; or
(g) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 168 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 173; or
(h) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 168 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 174; or
(i) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 169 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 171; or
(j) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 169 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 172; or
(k) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 169 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 173; or
(l) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 169 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 174; or
(m) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 170 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 171; or
(n) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 170 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 172; or
(o) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 170 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 173; or
(p) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 170 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 174.

The heavy chain variable region of the 3C7 humanized antibody having the amino acid sequence shown in SEQ ID NO: 167 is numbered: 3C7-VH0.

QVQLVQSGAEVKKPGASVKVSCKASGYTFTNYYIHWVRQAPGQGLEWMGAIYPD
NGDTSYNQRFKVRATLTADTSTSTVYMELSSLRSEDTAVYYCARAFTYWGQGTTVTVSS
(SEQ ID NO: 167).

The heavy chain variable region of the 3C7 humanized antibody having the amino acid sequence shown in SEQ ID NO: 168 is numbered: 3C7-VH1.

QVQLVQSGAEVKKPGASVKVSCKASGYIFTNYYIHWVRQAPGQGLEWMGAIYPDN
GDTSYNQRFKVRATLTADTSTSTVYMELSSLRSEDTAVYYCARAFTYWGQGTTVTVSS
(SEQ ID NO: 168).

The heavy chain variable region of the 3C7 humanized antibody having the amino acid sequence shown in SEQ ID NO: 169 is numbered: 3C7-VH2.

QVQLVQSGAEVKKPGASVKVSCKASGYIFRNYYIHWVRQAPGQGLEWMGAIYPDN
GDTSYNQRFKVRATLTADTSTSTVYMELSSLRSEDTAVYYCARAFTYWGQGTTVTVSS
(SEQ ID NO: 169).

The heavy chain variable region of the 3C7 humanized antibody having the amino acid sequence shown in SEQ ID NO: 170 is numbered: 3C7-VH3.

QVQLVQSGAEVKKPGASVKVSCKASGYIFRNYYIHWVKQAPGQGLEWMGAIYPDN
GDTSYNQRFKVRATLTADTSTSTVYMELSSLRSEDTAVYYCARAFTYWGQGTTVTVSS
(SEQ ID NO: 170).

The light chain variable region of the 3C7 humanized antibody having the amino acid sequence shown in SEQ ID NO: 171 is numbered: 3C7-VL0.

DIQMTQSPSSLSASVGDRVTITCRASENIYGALNWYQQKPGKAPKLLIYGATNLADG
VPSRFSGSGSGTDFTLTISSLQPEDFATYYCQNVLSTPYTFGGGTKVEIK (SEQ ID NO:
171).

The light chain variable region of the 3C7 humanized antibody having the amino acid sequence shown in SEQ ID NO: 172 is numbered: 3C7-VL1.

DIQMTQSPSSLSASVGDRVTITCGASENIYGALNWYQQKPGKAPKLLIYGATNLADG
VPSRFSGSGSGTDYTLTISSLQPEDVATYYCQNVLSTPYTFGGGTKVEIK (SEQ ID NO:
172).

The light chain variable region of the 3C7 humanized antibody having the amino acid sequence shown in SEQ ID NO: 173 is numbered: 3C7-VL2.

DIQMTQSPSSLSASVGDRVTITCGASENIYGALNWYQRKPGKAPKLLIYGATNLADG
VPSRFSGSGSGTDYTLTISSLQPEDFATYYCQNVLSTPYTFGGGTKLEIK (SEQ ID NO:
173).

The light chain variable region of the 3C7 humanized antibody having the amino acid sequence shown in SEQ ID NO: 174 is numbered: 3C7-VL3.

DIQMTQSPSSLSASVGDRVTITCGASENIYGALNWYQRKPGKSPKLLIYGATNLADG
VPSRFSGSGSGTDYTLTISSLQPEDVATYYCQNVLSTPYTFGGGTKLEIK (SEQ ID NO:
174).

According to the embodiments of the present invention, the present invention provides a humanized antibody or an antigen-binding fragment thereof, wherein the antibody comprises a heavy chain variable region and a light chain variable region; the heavy chain variable region comprises: VH-CDR1, VH-CDR2 and VH-CDR3, the light chain variable region comprises: VL-CDR1, VL-CDR2 and VL-CDR3, and the heavy chain variable region comprises a heavy chain framework region, and the light chain variable region comprises a light chain framework region; the heavy chain framework region comprises: H-FR1, H-FR2, H-FR3 and H-FR4, and the light chain framework region comprises: L-FR1, L-FR2, L-FR3 and L-FR4,
wherein, the VH-CDR1, VH-CDR2 and VH-CDR3 have the amino acid sequences shown in SEQ ID NO: 12, SEQ ID NO: 20 and SEQ ID NO: 25, respectively;
the VL-CDR1, VL-CDR2 and VL-CDR3 have the amino acid sequences shown in SEQ ID NO: 30, SEQ ID NO: 44 and SEQ ID NO: 55, respectively;
the H-FR1 has an amino acid sequence shown in QVQLVQSGAEVKKPGASVKVSCKASGYTFX45, wherein, X45 is selected from T or S,
the H-FR2 has an amino acid sequence shown in WVX46QAPGQGLEWX47G, wherein, X46 is selected from R or K, X47 is selected from M or I,
the H-FR3 has an amino acid sequence shown in SEQ ID NO: 180,
the H-FR4 has an amino acid sequence shown in SEQ ID NO: 181,
the L-FR1 has an amino acid sequence shown in SEQ ID NO: 182,
the L-FR2 has an amino acid sequence shown in WYQQKPGKX48PKLLX49Y, wherein X48 is selected from A or S, X49 is selected from I or V,
the L-FR3 has an amino acid sequence shown in GVPSRFSGSGSGTDX50TLTISSLQPEDFATYYC, wherein, X50 is selected from For Y,
the L-FR4 has an amino acid sequence shown in FGGGTKX51EIK, wherein X51 is selected from V or L.

According to the embodiments of the present invention, in the humanized antibody or antigen-binding fragment thereof, the VH-CDR1, VH-CDR2 and VH-CDR3 have the amino acid sequences shown in SEQ ID NO: 12, SEQ ID NO: 20 and SEQ ID NO: 25, respectively;
the VL-CDR1, VL-CDR2 and VL-CDR3 have the amino acid sequences shown in SEQ ID NO: 30, SEQ ID NO: 44 and SEQ ID NO: 55, respectively;
the H-FR1 has an amino acid sequence shown in SEQ ID NO: 175 or SEQ ID NO: 176,
the H-FR2 has an amino acid sequence shown in SEQ ID NO: 177, SEQ ID NO: 178 or SEQ ID NO: 179,
the H-FR3 has an amino acid sequence shown in SEQ ID NO: 180,
the H-FR4 has an amino acid sequence shown in SEQ ID NO: 181,
the L-FR1 has an amino acid sequence shown in SEQ ID NO: 182,
the L-FR2 has an amino acid sequence shown in SEQ ID NO: 183, SEQ ID NO: 184 or SEQ ID NO: 185,
the L-FR3 has an amino acid sequence shown in SEQ ID NO: 186 or SEQ ID NO: 187,
the L-FR4 has an amino acid sequence shown in SEQ ID NO: 188 or SEQ ID NO: 189.

The CDR region of the 3H9 humanized antibody has the following amino acid sequence:
VH-CDR1: FYNMH (SEQ ID NO: 12),
VH-CDR2: LIYPDNGDTSYNQKFKG (SEQ ID NO: 20),
VH-CDR3: GGLDY (SEQ ID NO: 25),
VL-CDR1: RASENIYSNLA (SEQ ID NO: 30),
VL-CDR2: AATNLAD (SEQ ID NO: 44),
VL-CDR3: QHFWGTPYT (SEQ ID NO: 55).

The framework region of the 3H9 humanized antibody has the following amino acid sequence:
H-FR1:
   QVQLVQSGAEVKKPGASVKVSCKASGYTFT (SEQ ID NO: 175),
   QVQLVQSGAEVKKPGASVKVSCKASGYTFS (SEQ ID NO: 176),
H-FR2:
   WVRQAPGQGLEWMG (SEQ ID NO: 177),
   WVRQAPGQGLEWIG (SEQ ID NO: 178),
   WVKQAPGQGLEWIG (SEQ ID NO: 179),
H-FR3:
   RATLTADTSTSTVYMELSSLRSEDTAVYYCAR (SEQ ID NO: 180),
H-FR4:
   WGQGTTVTVSS (SEQ ID NO: 181),
L-FR1:
   DIQMTQSPSSLSASVGDRVTITC (SEQ ID NO: 182),
L-FR2:
   WYQQKPGKAPKLLIY (SEQ ID NO: 183),
   WYQQKPGKSPKLLIY (SEQ ID NO: 184),
   WYQQKPGKSPKLLVY (SEQ ID NO: 185),
L-FR3:
   GVPSRFSGSGSGTDFTLTISSLQPEDFATYYC (SEQ ID NO: 186),
   GVPSRFSGSGSGTDYTLTISSLQPEDFATYYC (SEQ ID NO: 187),
L-FR4:
   FGGGTKVEIK (SEQ ID NO: 188),
   FGGGTKLEIK (SEQ ID NO: 189).

According to the embodiments of the present invention, the antibody in the humanized antibody or antigen-binding fragment thereof comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to any one of SEQ ID NO: 190-193.

According to the embodiments of the present invention, the antibody in the humanized antibody or antigen-binding fragment thereof comprises a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to any one of SEQ ID NO: 194-197.

According to the embodiments of the present invention, the humanized antibody or antigen-binding fragment thereof, wherein, the antibody comprises
(a) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 190 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 194; or
(b) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 190 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 195; or
(c) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 190 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 196; or
(d) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 190 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 197; or
(e) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 191 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 194; or
(f) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 191 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 195; or
(g) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 191 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 196; or
(h) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 191 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 197; or
(i) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 192 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 194; or
(j) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 192 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 195; or
(k) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 192 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 196; or
(l) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 192 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 197; or
(m) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 193 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 194; or
(n) a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 193 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 195; or
(o) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 193 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 196; or
(p) the antibody comprises a heavy chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 193 and a light chain variable region having an amino acid sequence with at least 85%, at least 86%, at least 87%, at least 88%, at least 89%, at least 90%, at least 91%, at least 92%, at least 93%, at least 94%, at least 95%, at least 96%, at least 97%, at least 98% or at least 99% identical to SEQ ID NO: 197.

The heavy chain variable region of the 3H9 humanized antibody having the amino acid sequence shown in SEQ ID NO: 190 is numbered: 3H9-VH0.

QVQLVQSGAEVKKPGASVKVSCKASGYTFTFYNMHWVRQAPGQGLEWMGLIYPD
NGDTSYNQKFKGRATLTADTSTSTVYMELSSLRSEDTAVYYCARGGLDYWGQGTTVTVS
S (SEQ ID NO: 190).

The heavy chain variable region of the 3H9 humanized antibody having the amino acid sequence shown in SEQ ID NO: 191 is numbered: 3H9-VH1.

QVQLVQSGAEVKKPGASVKVSCKASGYTFTFYNMHWVRQAPGQGLEWIGLIYPDN
GDTSYNQKFKGRATLTADTSTSTVYMELSSLRSEDTAVYYCARGGLDYWGQGTTVTVSS
(SEQ ID NO: 191).

The heavy chain variable region of the 3H9 humanized antibody having the amino acid sequence shown in SEQ ID NO: 192 is numbered: 3H9-VH2.

QVQLVQSGAEVKKPGASVKVSCKASGYTFSFYNMHWVRQAPGQGLEWIGLIYPDN

GDTSYNQKFKGRATLTADTSTSTVYMELSSLRSEDTAVYYCARGGLDYWGQGTTVTVSS
(SEQ ID NO: 192).

The heavy chain variable region of the 3H9 humanized antibody having the amino acid sequence shown in SEQ ID NO: 193 is numbered: 3H9-VH3.

QVQLVQSGAEVKKPGASVKVSCKASGYTFSFYNMHWVKQAPGQGLEWIGLIYPDN
GDTSYNQKFKGRATLTADTSTSTVYMELSSLRSEDTAVYYCARGGLDYWGQGTTVTVSS
(SEQ ID NO: 193).

The light chain variable region of the 3H9 humanized antibody having the amino acid sequence shown in SEQ ID NO: 194 is numbered: 3H9-VL0.

DIQMTQSPSSLSASVGDRVTITCRASENIYSNLAWYQQKPGKAPKLLIYAATNLADG
VPSRFSGSGSGTDFTLTISSLQPEDFATYYCQHFWGTPYTFGGGTKVEIK (SEQ ID NO:
194).

The light chain variable region of the 3H9 humanized antibody having the amino acid sequence shown in SEQ ID NO: 195 is numbered: 3H9-VL1.

DIQMTQSPSSLSASVGDRVTITCRASENIYSNLAWYQQKPGKSPKLLIYAATNLADG
VPSRFSGSGSGTDYTLTISSLQPEDFATYYCQHFWGTPYTFGGGTKVEIK (SEQ ID NO:
195).

The light chain variable region of the 3H9 humanized antibody having the amino acid sequence shown in SEQ ID NO: 196 is numbered: 3H9-VL2.

DIQMTQSPSSLSASVGDRVTITCRASENIYSNLAWYQQKPGKSPKLLVYAATNLADG
VPSRFSGSGSGTDYTLTISSLQPEDFATYYCQHFWGTPYTFGGGTKVEIK (SEQ ID NO:
196).

The light chain variable region of the 3H9 humanized antibody having the amino acid sequence shown in SEQ ID NO: 197 is numbered: 3H9-VL3.

DIQMTQSPSSLSASVGDRVTITCRASENIYSNLAWYQQKPGKSPKLLVYAATNLADG
VPSRFSGSGSGTDYTLTISSLQPEDFATYYCQHFWGTPYTFGGGTKLEIK (SEQ ID NO:
197).

According to the embodiments of the present invention, the antibody the humanized antibody or antigen-binding fragment thereof described in the present invention comprises at least one of a heavy chain constant region and a light chain constant region, and at least a portion of at least one of the heavy chain constant region and the light chain constant region is derived from at least one of a human antibody or a mutant thereof.

According to the embodiments of the present invention, the heavy chain constant region and the light chain constant region of the humanized antibody are both derived from human IgG antibodies or mutants thereof.

According to the embodiments of the present invention, the heavy chain constant region of the humanized antibody is derived from a human IgG1 or IgG4 antibody or a mutant thereof.

According to the embodiments of the present invention, the light chain constant region of the humanized antibody is derived from a human IgGκ or IgGλ constant region.

According to the embodiments of the present invention, the full-length sequence of the heavy chain constant region of the humanized antibody is shown in SEQ ID NO: 117.

According to the embodiments of the present invention, the full-length sequence of the light chain constant region of the humanized antibody is shown in SEQ ID NO: 118.

Compared with chimeric antibodies or other antibodies in the prior art, the humanized antibodies of the present invention have better anti-tumor activity, lower immunogenicity and/or better drugability.

Wherein, the antibodies of the present invention specifically bind to FGFR2b and have no detectable binding to FGFR2c.

Wherein, the antibodies of the present invention specifically bind to FGFR2b and have no detectable binding to FGFR2c, FGFR1 or FGFR4.

In the third aspect of the present invention, the present invention provides an isolated nucleic acid molecule.

According to the embodiments of the present invention, the nucleic acid molecule of the present invention comprises a nucleotide sequence encoding the above-mentioned antibody or antigen-binding fragment thereof of the present invention.

According to the embodiments of the invention, the nucleic acid molecule of the present invention is DNA.

According to the embodiments of the present invention, the present invention also provides a nucleic acid construct, the nucleic acid construct comprises the nucleic acid molecule described in the present invention; further, the nucleic acid construct comprises one or more control sequences operably linked to the nucleic acid molecule, wherein the one or more control sequences direct the production of the antibody or antigen-binding fragment thereof described in the present invention in an appropriate host cell, and the one or more control sequences are selected from: a promoter, an enhancer, a stop signal, a signal peptide, a leader sequence, a transcription terminator, and any group thereof.

In the fourth aspect of the present invention, the present invention provides an expression vector.

According to the embodiments of the present invention, the expression vector of the present invention is selected from: plasmid, cosmid, virus, minichromosome and artificial chromosome.

According to the embodiments of the present invention, the expression vector of the present invention carries the nucleic acid molecule of the present invention.

According to the embodiments of the present invention, the expression vector of the present invention is a eukaryotic expression vector.

The eukaryotic expression vector is selected from: pCRII, pCR3 and pcDNA3.4, pBSII, pET 15, pGEX, pEGFP-N1, pETL, pDSR-α and pFastBacDual, etc.

In the fifth aspect of the present invention, the present invention provides host cell.

The host cell is escherichia coli, yeast or a eukaryotic cell.

According to the embodiments of the present invention, the host cell comprises the expression vector described herein.

According to the embodiments of the present invention, the host cell comprises the nucleic acid molecule of the present invention.

According to the embodiments of the present invention, the host cell is a eukaryotic host cell.

According to the embodiments of the present invention, the host cell is a mammalian host cell.

In the sixth aspect of the present invention, the present invention provides a pharmaceutical composition.

According to the embodiments of the present invention, the pharmaceutical composition of the present invention comprises at least one of the antibody or antigen-binding fragment thereof, the nucleic acid molecule, the expression vector or the host cell.

According to the embodiments of the present invention, the pharmaceutical composition disclosed herein further comprises pharmaceutically acceptable carriers.

In the seventh aspect of the present invention, the present invention provides use of the antibody or antigen-binding fragment thereof, the nucleic acid molecule, the expression vector, the host cell or the pharmaceutical composition of the present invention in the preparation of a drug for treating FGFR2b-related diseases.

According to the embodiments of the present invention, the FGFR2b-related disease relates to a cancer characterized by expression or overexpression of FGFR2b;

Specifically, the above-mentioned cancers are ovarian cancer, endometrial cancer, breast cancer, lung cancer, bladder cancer, colon cancer, prostate cancer, cervical cancer, colorectal cancer, pancreatic cancer, gastric cancer, esophageal cancer, liver cancer, renal cell carcinoma, head and neck cancer, mesothelioma, melanoma, sarcoma and brain tumor.

In the eighth aspect of the present invention, the present invention provides a method for treating an FGFR2b-related disease in a subject, the method comprising administering to the subject in need thereof a therapeutically effective amount of the antibody or antigen-binding fragment thereof, the nucleic acid, the expression vector, the host cell or the pharmaceutical composition of the present invention.

According to the embodiments of the present invention, the administration is oral, nasal, intravenous, subcutaneous, sublingual or intramuscular administration.

In the ninth aspect of the present invention, the present invention provides a bispecific or multispecific antibody comprising the antibody or antigen-binding fragment thereof described herein.

In the tenth aspect of the present invention, the present invention provides an antibody-drug conjugate comprising the antibody or antigen-binding fragment thereof described herein. The drug conjugate described herein is preferably a small molecule drug, and the antibody in the antibody-drug conjugate targets the small molecule drug to the therapeutic target through specific binding.

In the eleventh aspect of the present invention, the present invention provides use of the antibody or antigen-binding fragment thereof described herein in the manufacture of a diagnostic reagent for detecting FGFR2b-related diseases.

In the twelfth aspect of the present invention, the present invention provides a kit for detecting FGFR2b, comprising the antibody or antigen-binding fragment thereof described herein. The kit further comprises a container, instructions for use, a buffer, and the like. In the kit described herein, the antibody described herein may be immobilized on a detection plate.

Additional aspects and advantages of the present invention will be set forth in part form following description, and in part will be obvious from the following description, or may be learned by practice of the invention.

### Description of the drawings

Figure 1 is a graph showing the binding results of expressed antibodies to FGFR2b antigen confirmed by ELISA;
Figure 2 is a graph showing the binding results of expressed antibodies to FGFR2b antigen confirmed by ELISA;
Figure 3 is a graph showing the binding results of expressed antibodies to FGFR2b antigen confirmed by ELISA;
Figure 4 is a graph showing the results of flow cytometry detection of the binding of KATO III cells that highly express FGFR2b to antibodies;
Figure 5 is a graph showing the results of flow cytometry detection of the binding of KATO III cells that highly express FGFR2b to antibodies;
Figure 6 is a graph showing the results of flow cytometry detection of the binding of KATO III cells that highly express FGFR2b to antibodies;
Figure 7 is a graph showing the results of evaluating the level of down-regulation of FGFR2b protein tyrosine phosphorylation by antibodies *in vitro* under KGF stimulation using the HEK293/SRE-Luc-FGFR2b cell model;
Figure 8 is a graph showing the results of evaluating the level of down-regulation of FGFR2b protein tyrosine phosphorylation by antibodies *in vitro* under KGF stimulation using the HEK293/SRE-Luc-FGFR2b cell model;
Figure 9 is a graph showing the results of evaluating the level of down-regulation of FGFR2b protein tyrosine phosphorylation by antibodies *in vitro* under FGF10 stimulation using the HEK293/SRE-Luc-FGFR2b cell model;
Figure 10 is a graph showing the results of evaluating the level of down-regulation of FGFR2b protein tyrosine phosphorylation by antibodies *in vitro* under FGF10 stimulation using the HEK293/SRE-Luc-FGFR2b cell model;
Figure 11 is a graph showing the results of evaluating the level of down-regulation of FGFR2b protein tyrosine phosphorylation by antibodies *in vitro* under FGF1 stimulation using the HEK293/SRE-Luc-FGFR2b cell model;
Figure 12 is a graph showing the results of evaluating the level of down-regulation of FGFR2b protein tyrosine phosphorylation by antibodies *in vitro* under FGF1 stimulation using the HEK293/SRE-Luc-FGFR2b cell model;
Figure 13 is an evaluation of the tumor growth inhibition rate (TGI) of the antibody using the SNU16 subcutaneous transplanted tumor mouse model;
Figure 14 is an evaluation of the effect of antibodies on the body weight of tumor-bearing mice using the SNU16 subcutaneous transplanted tumor mouse model;
Figure 15 is a graph showing the binding results of KATO III cells that highly express FGFR2b and humanized antibodies detected by flow cytometry;
Figure 16 is a graph showing the binding results of KATO III cells that highly express FGFR2b and humanized antibodies detected by flow cytometry;
Figure 17 is a graph showing the binding results of KATO III cells that highly express FGFR2b and humanized antibodies detected by flow cytometry;
Figure 18 is a graph showing the results of evaluating the level of down-regulation of FGFR2b protein tyrosine phosphorylation by humanized antibodies *in vitro* under KGF stimulation using the HEK293/SRE-Luc-FGFR2b cell model;
Figure 19 is a graph showing the results of evaluating the level of down-regulation of FGFR2b protein tyrosine phosphorylation by humanized antibodies *in vitro* under KGF stimulation using the HEK293/SRE-Luc-FGFR2b cell model;
Figure 20 is a graph showing the results of evaluating the level of down-regulation of FGFR2b protein tyrosine phosphorylation by humanized antibodies *in vitro* under KGF stimulation using the HEK293/SRE-Luc-FGFR2b cell model.

### EXAMPLES

The following description of the present invention is intended only to illustrate various embodiments of the present invention. Therefore, the specific modifications discussed should not be construed as limiting the scope of the present invention. Those skilled in the art will readily appreciate that various equivalents, variations, and modifications may be implemented without departing from the scope of the present invention, and such equivalent embodiments are intended to be encompassed herein. All references cited herein, including publications, patents, and patent applications, are hereby incorporated by reference in their entirety.

In the present invention, the term "antibody" includes any immunoglobulin, monoclonal antibody, polyclonal antibody, multivalent antibody, bivalent antibody, monovalent antibody, multispecific antibody, bispecific antibody and antigen-binding fragments thereof that bind to a specific antigen. A natural intact antibody comprises two heavy (H) chains and two light (L) chains. Both the L chain and the H chain contain a variable region (V region) with a large variation in amino acid sequence and a constant region (C region) with a relatively stable amino acid sequence. The variable regions of the L chain and H chain are denoted as VL and VH. There are some areas in the V region where the amino acid sequences are very prone to variation, which are called hypervariable regions (HVR) or complementarity-determining regions (CDR). The L chain and H chain each have three CDRs (the H chain contains VH-CDR1, VH-CDR2 and VH-CDR3, and the L chain contains VL-CDR1, VL-CDR2 and VL-CDR3). The CDR boundaries of the antibodies disclosed herein may be defined or labeled according to the conventions of Kabat, IMGT, or Chothia (Al-Lazikani, B., Chothia, C., Lesk, A.M., J. Mol. Biol., 273(4), 927 (1997); Chothia, C. et al., J. Mol. Biol., Dec 5;186(3):651-63 (1985); Chothia, C. and Lesk, A.M., J. Mol. Biol., 196, 901 (1987); Chothia, C. et al., Nature, Dec 21-28;342(6252):877-83 (1989); Kabat E.A. et al. , National Institutes of Health, Bethesda, Md. (1991);
Marie-Paule Lefranc et al., Developmental and Comparative Immunology, 27:55-77 (2003). These three CDRs are interspersed with flanking segments called framework regions (FRs). FRs are more conserved than CDRs and form a scaffold supporting the hypervariable loops. The FRs of a variable domain typically consist of four FR domains: FR1, FR2, FR3 and FR4. The carboxyl termini of the L and H chains are relatively stable, with minimal variation. This region is called the constant region. The constant regions of the heavy and light chains do not participate in antigen binding but exhibit various effector functions.

In the present invention, the term "antigen-binding fragment" refers to an antibody fragment comprising one or more CDRs formed from a portion of an intact antibody, or any other antibody fragment that can bind to an antigen but does not contain the intact native antibody structure. Examples of antigen-binding fragments include, but are not limited to, diabodies, Fab, Fab', F(ab')2, Fv fragments, disulfide-stabilized Fv fragments (dsFv), (dsFv)2, bispecific dsFv (dsFv-dsFv'), disulfide-stabilized diabodies (dsdiabodies), single-chain antibodies (scFv), single-chain Fv-Fc antibodies (scFv-Fc), scFv dimers (divalent diabodies), bispecific antibodies, multispecific antibodies, camelized single domain antibodies, nanobodies, domain antibodies, and bivalent domain antibodies. Antigen-binding fragments are capable of binding to the same antigen as the parent antibody.

In the present invention, "Fab'" refers to a Fab fragment that includes a portion of the hinge region.

"Fv" in relation to antibodies refers to the smallest antibody fragment with a complete antigen-binding site. An Fv fragment consists of the variable region of a single light chain bound to the variable region of a single heavy chain.

"dsFv" refers to a disulfide-stabilized Fv fragment in which the linkage between the variable region of a single light chain and the variable region of a single heavy chain is a disulfide bond.

"Single-chain Fv" or "scFv" refers to an engineered antibody consisting of a light chain variable region and a heavy chain variable region linked directly or via a peptide linker sequence.

"Camelized single domain antibody", "heavy chain antibody," or "HCAb" refers to an antibody containing two VH domains and no light chain.

"Nanobody" refers to an antibody fragment consisting of a VH domain from a conventional IgG heavy chain antibody and two heavy chain constant domains, such as CH2 and CH3.

"Domain antibodies" are antibody fragments containing only the variable region of a heavy chain or a light chain. In some cases, two or more VH domains are covalently joined with a peptide linker to create a bivalent or multivalent domain antibody. The two VH domains of a bivalent domain antibody can target the same or different antigens.

The term "bivalent" refers to an antibody or antigen-binding fragment that has two antigen-binding sites; the term "monovalent" refers to an antibody or antigen-binding fragment that has only a single antigen-binding site; and the term "multivalent" refers to an antibody or antigen-binding fragment that has multiple antigen-binding sites.

The term "bispecific" antibody refers to an artificial antibody or antigen-binding fragment that has properties derived from two different monoclonal antibodies and is capable of binding to two different epitopes. The two epitopes may be present on the same antigen, or they may be present on two different antigens. A "multispecific" antibody is an antibody that can simultaneously bind to two or more different epitopes or antigens.

In the present invention, the terms "sequence identity", "sequence identity" and "sequence homology" are interchangeable and refer to nucleic acid sequences or amino acid sequences that have at least 85%, at least 90%, at least 95% (e.g., at least 85%, 86%, 87%, 88%, 89%, 90%, 91%, 92%, 93%, 94%, 95%, 96%, 97%, 98%, 99% or 100%) sequence identity with another sequence when optimally aligned. In the present invention, at least 85% identity can be further preferably at least 90% identity, more preferably at least 95% identity.

In the present invention, the term "anti-FGFR2b antibody" refers to an antibody that can specifically bind to FGFR2b. In some embodiments, the anti-FGFR2b antibodies provided herein are capable of specifically binding to FGFR2b, but not to FGFR1b, FGFR2c, FGFR1c and FGFR4, or bind less strongly to FGFR1b, FGFR2c, FGFR1c and FGFR4 (e.g., the binding affinity to FGFR1b, FGFR2c, FGFR1c, and FGFR4 is at least 10-fold lower, or at least 50-fold lower, or at least 100-fold lower or at least 200-fold lower than the binding affinity to FGFR2b). In some embodiments, the anti-FGFR2b antibodies provided herein have no detectable binding to FGFR1b, FGFR2c, FGFR1c and FGFR4.

In the present invention, the term "specific binding" refers to the binding reaction between an antibody and the antigen against which it is directed. An antibody that specifically binds to an antigen (or an antibody specific for an antigen) means that the antibody binds to the antigen with an affinity (KD) of less than about 10-5M, for example, less than about 10⁻⁶M, 10⁻⁷M, 10⁻⁸M, 10⁻⁹M or 10⁻¹⁰M or less. KD refers to the dissociation equilibrium constant of a specific antibody-antigen interaction. The smaller the equilibrium dissociation constant, the tighter the antibody-antigen binding and the higher the affinity between the antibody and the antigen.

In the present invention, the term "chimeric" refers to an antibody or antigen-binding fragment in which a portion of the antibody (such as the heavy chain and/or light chain) is derived from one species and the remaining portion is derived from a different species. A chimeric antibody may include a constant region derived from a human and a variable region derived from a non-human animal such as a mouse, wherein the non-human animal is a mammal, for example, a mouse, rat, rabbit, goat, sheep, guinea pig or hamster.

In the present invention, the term "humanized" antibody refers to a chimeric antibody comprising amino acid residues from non-human HVRs and amino acid residues from human FRs. In certain aspects, a humanized antibody will comprise substantially all of at least one, and typically two, variable domains, in which all or substantially all of the HVRs (e.g., CDRs) correspond to those of a non-human antibody and all or substantially all of the FRs correspond to those of a human antibody. In certain aspects in which all or substantially all FRs of a humanized antibody correspond to FRs of a human antibody, any FR of the humanized antibody may contain one or more amino acid residues from a non-human FR (e.g., one or more Vernier position residues of a FR). A humanized antibody optionally may comprise at least a portion of an antibody constant region derived from a human antibody. A "humanized form" of an antibody, eg, a non-human antibody, is an antibody that has been humanized.

In the present invention, the term "amino acid" refers to a compound containing both amino and carboxyl functional groups, such as α-amino acids. Two or more amino acids can be combined to form a polypeptide through amide bonds (also called peptide bonds). Individual amino acids are encoded by nucleic acids consisting of three nucleotides (called codons or base triplets). Each amino acid is encoded by at least one codon. The fact that the same amino acid is encoded by different codons is called "degeneracy of the genetic code." Amino acids include both natural and unnatural amino acids. Natural amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y) and valine (Val, V).

In the present invention, the term "isolated" material has been altered from its natural state by the hand of man. If an "isolated" composition or substance occurs in nature, it has been altered from its original environment or removed from its original environment, or both. For example, a polynucleotide or polypeptide naturally present in a living animal is not "isolated", but if the polynucleotide or polypeptide is sufficiently separated from the coexisting materials of its natural state so that it exists in a substantially pure state, then the polynucleotide or polypeptide is "isolated".

In the present invention, the term "expression vector" refers to a vehicle into which a polynucleotide encoding a protein can be operably inserted to cause expression of the protein. Expression vectors may contain a variety of elements for controlling expression, including promoter sequences, transcription initiation sequences, enhancer sequences, selection elements, and reporter genes. Furthermore, the vector may contain an origin of replication.

In the present invention, the term "host cell" refers to a cell into which an exogenous polynucleotide and/or vector is introduced.

In the present invention, the term "antibody-drug conjugate (ADC)" refers to the antibody coupled to the effector molecule, and preferably chemically coupled. The effector molecule is preferably a drug with therapeutic activity, such as one or more of a toxic protein, a chemotherapy drug, a small molecule drug or a radionuclide.

In the present invention, the terms "treating" and "treatment" encompass preventing or alleviating a condition, slowing the onset or rate of development of a condition, reducing the risk of developing a condition, preventing or delaying the development of symptoms associated with a condition, reducing or eliminating symptoms associated with a condition, producing complete or partial regression of a condition, curing a condition, or some combination thereof.

In the present invention, the term "subject" refers to any animal or human being. The subject is preferably a mammal, more preferably a human. The subject may be a patient.

In the present invention, the term "cancer" refers to any medical condition characterized by malignant cell growth or neoplasm, abnormal proliferation, infiltration or metastasis, and includes both solid tumors and non-solid cancers. As used herein, "solid tumor" refers to a solid mass of neoplastic and/or malignant cells. "Non-solid cancer" refers to hematological malignancies such as leukemia, lymphoma, myeloma and other hematological malignancies. Examples of cancer or tumors include hematologic malignancies (e.g., lymphoma, Hodgkin's lymphoma, non-Hodgkin's lymphoma, and B-cell lymphoma), oral cancer (e.g., cancer of the lip, tongue, or pharynx), digestive organs (e.g., esophagus, stomach, small intestine, colon, large intestine, or rectum), peritoneum, liver, and bile ducts, pancreas, respiratory system such as the larynx or lungs (small cell and non-small cell), bone, connective tissue, skin (e.g., melanoma), breast, reproductive organs (fallopian tubes, uterus, cervix, testicles, ovaries, or prostate), urinary tract (e.g., bladder or kidney), brain, and tumors of endocrine glands such as the thyroid gland. In certain embodiments, the cancer is selected from ovarian cancer, endometrial cancer, breast cancer, lung cancer (small cell or non-small cell lung cancer), bladder cancer, colon cancer, prostate cancer, cervical cancer, colorectal cancer, pancreatic cancer, gastric cancer, esophageal cancer, hepatocellular carcinoma (liver cancer), renal cell carcinoma (kidney cancer), head and neck cancer, mesothelioma, melanoma, sarcoma, and brain tumor (e.g., glioma, such as glioblastoma).

In the present invention, the term "pharmaceutically acceptable carrier" refers to ingredients in pharmaceutical formulations other than active ingredients, which are generally chemically and/or physically compatible with other ingredients constituting the formulation and are non-toxic to subjects. Pharmaceutically acceptable carriers include, but are not limited to, buffers, excipients, stabilizers, preservatives, and the like. Specific examples may be one or more of water, saline, phosphate buffered saline, glucose, glycerol, ethanol, etc. and combinations thereof. In many cases, isotonic agents, such as sugars, polyalcohols (eg, mannitol, sorbitol), or sodium chloride, are included in the pharmaceutical composition. Of course, the pharmaceutically acceptable carriers can also include trace amounts of auxiliary substances, such as wetting agents or emulsifiers, preservatives or buffering agents, to extend the shelf life or efficacy of antibody.

In the present invention, the term "effective amount" refers to a dose that can induce a biological or medical response in cells, tissues, organs or organisms (eg, individuals/subjects) and is sufficient to achieve the desired preventive and/or therapeutic effect.

The scheme of the present invention will be explained below in conjunction with the embodiments. Those skilled in the art will understand that the following examples are only used to illustrate the present invention, and should not be construed as limiting the scope of the present invention. If no specific technology or condition is specified in the embodiments, the technology or condition described in the literature in this field or the product specification shall be followed. The reagents or instruments used without the manufacturer's indication are conventional products that can be obtained from the market.

### Example 1 Phage screening technology and flow cytometry screening of FGFR2b monoclonal antibodies

The antibody scFv format was displayed on phages and selected using phage library screening. ELISA screening yielded monoclonal antibodies that strongly bound to FGFR2b and weakly or not at all to FGFR2c. The gene encoding the extracellular domain of the FGFR2b protein was designed using the NCBI database and constructed into a mammalian eukaryotic expression system. The extracellular domain of the FGFR2b protein (NP_075259.4) was expressed and used for mouse immunization and mouse monoclonal antibody screening. After Balb/C mice were immunized by subcutaneous injection four times, the antibody titer of the sera of the immunized mice was measured by ELISA and reached 10⁵. Mice spleens were processed to extract lymphocyte mRNA. The VH and VL sequences of the antibodies were amplified and linked using a linker (preferably the amino acid sequence set forth in SEQ ID NO:122). An immune library was constructed. Phage from the immune library were then panned with FGFR2b protein. After three rounds of panning, ELISA was used to screen for monoclonal antibody phage that bound to FGFR2b but weakly or not at all to FGFR2c. The binding results of 26 candidate phage to FGFR2b and FGFR2c are shown in Tables 1 and 2. The "Negative control" in Tables 1 and 2 refers to a monoclonal antibody that did not bind to FGFR2b. The OD450 values in Tables 1 and 2 respectively reflect the ability of the candidates to bind to the extracellular domains of FGFR2b and FGFR2c proteins. Compared with the negative control, the phage detection readings of the candidates and FGFR2b have reached saturation, which is equivalent to the FGFR2c detection readings and the negative control, meeting the screening requirements.

**Table 1 ELSIA results of candidate binding to FGFR2b**

| Clone (No.) | OD (450 nm) value | Clone (No.) | OD (450 nm) value |
|---|---|---|---|
| 2B1 | 2.327 | 3H6 | 2.288 |
| 3A12 | 2.412 | 3H9 | 2.224 |
| 3A8 | 2.218 | 3B4 | 2.467 |
| 3C7 | 2.023 | 3B7 | 2.026 |
| 3C9 | 2.085 | 3D7 | 2.200 |
| 3D6 | 1.922 | 3F11 | 2.001 |
| 3E1 | 2.174 | 1G6 | 2.437 |
| 3E2 | 1.869 | 1B8 | 2.648 |
| 3E3 | 2.28 | 1D7 | 2.141 |
| 3E7 | 1.881 | 1C10 | 2.552 |
| 3F5 | 1.936 | 1A3 | 2.668 |
| 3F10 | 1.851 | 2B2 | 2.377 |
| 3G10 | 1.945 | 3A3 | 2.333 |
| Negative control | 0.024 | N/A | N/A |

**Table 2 ELSIA results of candidate binding to FGFR2c**

| Clone (No.) | OD (450 nm) value | Clone (No.) | OD (450 nm) value |
|---|---|---|---|
| 2B1 | 0.049 | 3H6 | 0.062 |
| 3A12 | 0.100 | 3H9 | 0.073 |
| 3A8 | 0.065 | 3B4 | 0.042 |
| 3C7 | 0.040 | 3B7 | 0.043 |
| 3C9 | 0.033 | 3D7 | 0.037 |
| 3D6 | 0.037 | 3F11 | 0.045 |
| 3E1 | 0.056 | 1G6 | 0.073 |
| 3E2 | 0.036 | 1B8 | 0.082 |
| 3E3 | 0.033 | 1D7 | 0.059 |
| 3E7 | 0.034 | 1C10 | 0.068 |
| 3F5 | 0.038 | 1A3 | 0.212 |
| 3F10 | 0.039 | 2B2 | 0.051 |
| 3G10 | 0.042 | 3A3 | 0.050 |
| Negative control | 0.045 | N/A | N/A |

| | | | |
|---|---|---|---|
| "N/A" in Tables 1 and 2 indicates blank. | | | |

### Example 2 Expression and purification of antibodies and detection of antibody binding properties

A series of antibody expression vectors were constructed using molecular cloning methods and recombinantly expressed in the 293F expression system. Nucleotide sequences encoding the light and heavy chains of a series of monoclonal antibodies were obtained from plasmids extracted from panning phage cultures. ScFv sequences were obtained through gene amplification, and the human IgG4 constant region (amino acid sequence shown in SEQ ID NO:125) was selected and incorporated into a eukaryotic expression vector using molecular cloning methods to construct a series of antibody pcDNA3.4-scFv-Fc expression vectors. Then, a series of verified expression vectors were extracted using an Invitrogen plasmid extraction kit, linearized with restriction enzymes, purified, and stored at -20°C.

293F host cells were revived in Opim medium and harvested at a cell density of approximately 3 * 10⁶ cells/mL for transfection with PEI reagent. On the second day of culture, the culture medium was fed and glucose was added to a concentration of 8 g/L. On the sixth day of culture, the cell culture medium was harvested.

A series of antibodies were tested at the translational level. Cell culture fluid was purified using a Protein A column, and absorbance peaks were collected for mass spectrometry analysis. Mass spectrometry revealed that the molecular weight of a series of chimeric antibody scFv-Fcs was approximately 100 kD, consistent with the theoretical molecular weight, indicating a dimer. The collected samples were also analyzed by 10% SDS-PAGE electrophoresis after both reduced and non-reduced conditions. The reduced SDS-PAGE electrophoresis pattern showed a single band at approximately 50 kD, while the non-reduced SDS-PAGE electrophoresis pattern revealed a single band at approximately 100 kD, consistent with the theoretical band size. The purified samples were dialyzed overnight at 4°C against 0.02 M PBS buffer (pH 7.4).

### 2.1 Binding of the expressed antibody to the FGFR2b antigen was confirmed by ELISA.

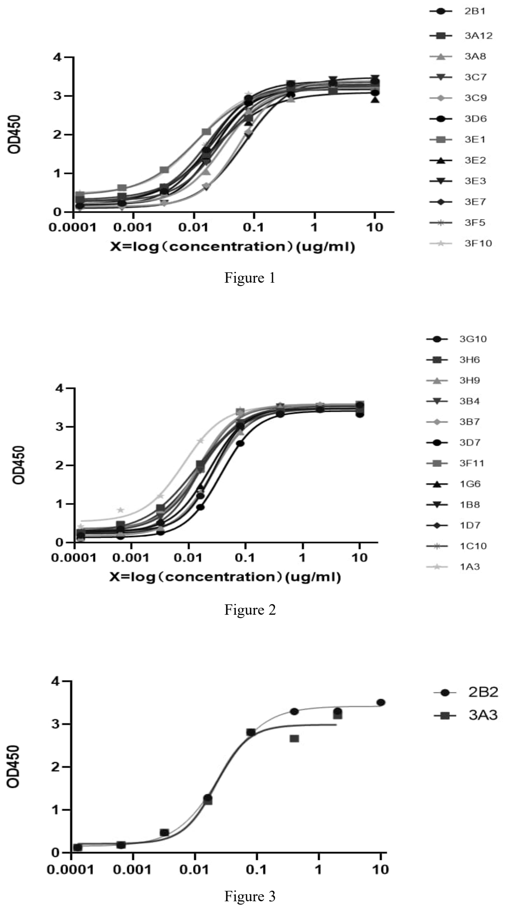

An ELISA plate was coated with 1 µg/ml of FGFR2b (purchased from Kaixia Biotechnology, catalog number: FGR-HM1BB) at 4°C overnight. Nonspecific binding sites were then blocked with 3% BSA at room temperature for 1 hour. The plate was then incubated with various concentrations of the mAb (scFv-Fc) expressed in the above example for 1 hour. Bound mAb was detected with HRP-goat anti-human antibody, followed by washing. TMB substrate (Yunqiao Biotechnology) was added and incubated at 45°C. 0nm reading, the test results are shown in Table 3 and Figures 1-3. Figures 1-3 show the binding values of antibodies at different concentrations to the extracellular region of FGFR2b protein. As the antibody concentration increases, the binding ability of the antibody to FGFR2b increases. When the antibody concentration reaches a certain value, the OD450 value of the antibody binding to the extracellular region of FGFR2b protein reaches saturation. Table 3 shows the EC50 value obtained by fitting the curves of Figures 1-3 using GraphPad software. The smaller the EC50 value, the higher the affinity of the antibody to FGFR2b.

**Table 3 Affinity results of antibodies to FGFR2b antigen**

| Clone (No.) | EC50 (ug/mL) | Clone (No.) | EC50 (ug/mL) |
|---|---|---|---|
| 2B1 | 0.02764 | 3G10 | 0.02764 |
| 3A12 | 0.01279 | 3H6 | 0.01279 |
| 3A8 | 0.02784 | 3H9 | 0.02784 |
| 3C7 | 0.01661 | 3B4 | 0.01661 |
| 3C9 | 0.01474 | 3B7 | 0.01474 |
| 3D6 | 0.03686 | 3D7 | 0.03686 |
| 3E1 | 0.01557 | 3F11 | 0.01557 |
| 3E2 | 0.02346 | 1G6 | 0.02346 |
| 3E3 | 0.02727 | 1B8 | 0.02727 |
| 3E7 | 0.01544 | 1D7 | 0.01544 |
| 3F5 | 0.01588 | 1C10 | 0.01588 |
| 3F10 | 0.00818 | 1A3 | 0.00818 |
| 2B2 | 0.02156 | 3A3 | 0.02156 |

### 2.2 Flow cytometry detection of antibody binding between KATO III cells that overexpress FGFR2b

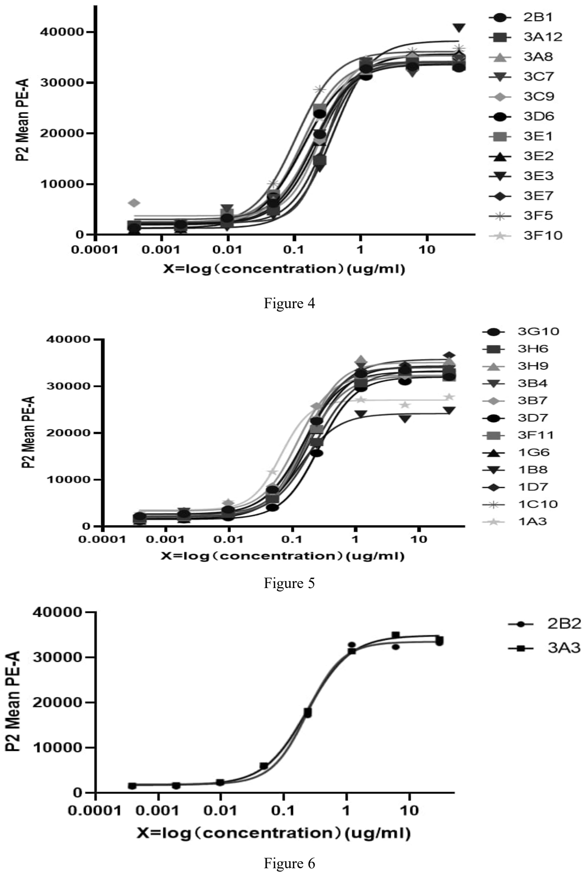

1*10⁵ cells were resuspended in a 96-well plate and incubated with various concentrations of mAb (scFv-Fc) for 1 hour. The cells were then washed twice in PBS and detected by incubation with PE-goat anti-human IgG (Jackson ImmunoResearch) at 4 °C for 30 minutes. The cells were analyzed by FACS (Becman). The results are shown in Table 4 and Figures 4-6. Figures 4-6 show the binding of different concentrations of antibody to KATO III cells that overexpress FGFR2b. As the antibody concentration increases, the binding affinity of the antibody to KATO III cells overexpressing FGFR2b increases. When the antibody concentration reaches a certain value, the mean fluorescence intensity of the antibody binding to FGFR2b reaches saturation. Table 4 shows the EC50 values obtained by fitting the curves in Figure 4-6 using GraphPad software. Lower EC50 values indicate higher affinity of the antibody for FGFR2b as measured by flow cytometry.

**Table 4 Affinity results of antibodies to FGFR2b**

| Clone (No.) | EC50 (ug/mL) | Clone (No.) | EC50 (ug/mL) |
|---|---|---|---|
| 2B1 | 0.1946 | 3G10 | 0.1491 |
| 3A12 | 0.292 | 3H6 | 0.2263 |
| 3A8 | 0.1993 | 3H9 | 0.1606 |
| 3C7 | 0.1997 | 3B4 | 0.1681 |
| 3C9 | 0.2534 | 3B7 | 0.1168 |
| 3D6 | 0.1363 | 3D7 | 0.2589 |
| 3E1 | 0.1323 | 3F11 | 0.1643 |
| 3E2 | 0.236 | 1G6 | 0.1859 |
| 3E3 | 0.3761 | 1B8 | 0.1325 |
| 3E7 | 0.2981 | 1D7 | 0.1674 |
| 3F5 | 0.1046 | 1C10 | 0.152 |
| 3F10 | 0.1591 | 1A3 | 0.0656 |
| 2B2 | 0.2328 | 3A3 | 0.2363 |

### 2.3 ELISA for cross-species binding of antibodies to recombinant cynomolgus monkey, mouse, and human FGFR2b

Recombinant cynomolgus macaque and mouse FGFR2b were purchased from acro, and human FGFR2b was purchased from Kaixia Biotechnology. The antigen was 1ug/mL coated on the ELISA plate at 4 °C overnight, and then the non-specific binding sites were blocked with 3% BSA at room temperature for 1h. The plate was incubated with an antibody at a concentration of 1ug/mL for 1h, and the bound mAb (scFv-Fc) was detected with HRP-goat anti-human antibody. The plate was then washed, TMB substrate (Yunqiao Biotechnology) was added, and the plate was read at 450nm. The results showed that there was no significant difference in the binding of all antibodies to antigens from different species. The results are shown in Tables 5 and 6. The OD450 values in Tables 5 and 6 respectively reflect the ability of the candidate to bind to the extracellular region of FGFR2b protein from various species, proving that the candidate of the present application has interspecies cross-reactivity; the "Negative control" in Tables 5 and 6 is a monoclonal antibody that does not bind to FGFR2b.

**Table 5 ELSIA results of binding of candidate compounds to monkey FGFR2b**

| Clone (No.) | OD (450 nm) value | Clone (No.) | OD (450 nm) value |
|---|---|---|---|
| 2B1 | 3.004 | 3E3 | 3.094 |
| 3A12 | 3.274 | 3E7 | 3.145 |
| 3C3 | 3.202 | 3F5 | 3.166 |
| 3C7 | 3.194 | 3F10 | 3.217 |
| 3C9 | 3.248 | 3G10 | 3.122 |
| 3D6 | 3.397 | 3H6 | 3.35 |
| 3E1 | 3.336 | 3H9 | 3.285 |
| 3E2 | 3.353 | 3B4 | 3.321 |
| 3B7 | 3.116 | 3D7 | 3.297 |
| 3F11 | 3.142 | 1G6 | 3.239 |
| 1B8 | 3.249 | 1D7 | 3.168 |
| 1C10 | 3.296 | 1A3 | 3.266 |
| 2B2 | 3.195 | 3A3 | 3.204 |
| Negative control | 0.024 | N/A | N/A |

**Table 6. ELSIA results for binding of candidate compounds to mouse FGFR2b**

| Clone (No.) | OD (450 nm) value | Clone (No.) | OD (450 nm) value |
|---|---|---|---|
| 2B1 | 2.723 | 3E3 | 3.055 |
| 3A12 | 2.909 | 3E7 | 2.288 |
| 3C3 | 3.033 | 3F5 | 2.375 |
| 3C7 | 3.102 | 3F10 | 2.87 |
| 3C9 | 2.573 | 3G10 | 3.002 |
| 3D6 | 2.591 | 3H6 | 2.391 |
| 3E1 | 2.924 | 3H9 | 2.992 |
| 3E2 | 2.964 | 3B4 | 2.877 |
| 3B7 | 2.974 | 3D7 | 2.48 |
| 3F11 | 2.924 | 1G6 | 2.758 |
| 1B8 | 2.014 | 1D7 | 3.038 |
| 1C10 | 2.943 | 1A3 | 1.812 |
| 2B2 | 3.087 | 3A3 | 2.63 |
| Negative control | 0.027 | N/A | N/A |

| | | | |
|---|---|---|---|
| "N/A" in Tables 5 and 6 indicates blank. | | | |

### 2.4 Confirmation of Antibody Binding to Human and Mouse FGFR2b Antigens by Biofilm Interferometry

An AHC2 (octet) sensor chip was activated in SD buffer (PBS, 0.1% BSA, 0.02% Tween). Antibodies (5 µg/mL, scFv-Fc) were immobilized on the sensor chip and tested for binding to various concentrations of human and mouse FGFR2b. Association constants (KD) and dissociation constants (Kds) were calculated using ForteBio Data Analysis software (version 9.0). The results are shown in Tables 7 and 8. The association constants for the candidate binding to human FGFR2b ranged from 2 nM to 10 nM, indicating good affinity. The association constants for the candidate binding to mouse FGFR2b ranged from 8 nM to 90 nM, also indicating good binding.

**Table 7 Dissociation and Association Constants of the Candidates with hFGFR2b**

| Sample ID | KD (M) | kon(1/Ms) | kdis(1/s) | Full R^2 |
|---|---|---|---|---|
| 2B1 | 6.67E-09 | 2.68E+05 | 1.79E-03 | 0.9829 |
| 3A12 | 2.82E-09 | 2.35E+05 | 6.62E-04 | 0.9756 |
| 3A8 | 3.48E-09 | 2.59E+05 | 9.00E-04 | 0.9799 |
| 3C7 | 3.99E-09 | 2.87E+05 | 1.14E-03 | 0.979 |
| 3C9 | 2.70E-09 | 3.53E+05 | 9.54E-04 | 0.9682 |
| 3D6 | 1.50E-08 | 7.59E+04 | 1.14E-03 | 0.9676 |
| 3E1 | 4.50E-09 | 3.37E+05 | 1.52E-03 | 0.9794 |
| 3E2 | 4.45E-09 | 2.47E+05 | 1.10E-03 | 0.9825 |
| 3E3 | 8.18E-09 | 2.25E+05 | 1.84E-03 | 0.9773 |
| 3E7 | 4.35E-09 | 2.56E+05 | 1.11E-03 | 0.9827 |
| 3F5 | 5.10E-09 | 2.81E+05 | 1.43E-03 | 0.9754 |
| 3F10 | 7.62E-09 | 3.03E+05 | 2.31E-03 | 0.9714 |
| 3G10 | 6.25E-09 | 3.18E+05 | 1.99E-03 | 0.9771 |
| 3H6 | 8.41E-09 | 1.98E+05 | 1.66E-03 | 0.9831 |
| 3H9 | 5.69E-09 | 2.67E+05 | 1.52E-03 | 0.9683 |
| 2B2 | 6.18E-09 | 4.62E+05 | 2.86E-03 | 0.97 |
| 3A3 | 3.71E-09 | 3.59E+05 | 1.33E-03 | 0.9727 |
| 3B4 | 6.28E-09 | 3.11E+05 | 1.95E-03 | 0.9741 |
| 3B7 | 7.60E-09 | 2.67E+05 | 2.03E-03 | 0.9766 |
| 3D7 | 8.58E-09 | 2.36E+05 | 2.03E-03 | 0.9764 |
| 3F11 | 6.13E-09 | 3.31E+05 | 2.03E-03 | 0.9734 |
| 1A3 | 1.22E-08 | 3.92E+05 | 4.77E-03 | 0.9789 |
| 1G6 | 3.97E-09 | 3.74E+05 | 1.49E-03 | 0.9645 |
| 1B8 | 1.33E-08 | 4.72E+05 | 6.29E-03 | 0.975 |
| 1D7 | 4.51E-09 | 4.60E+05 | 2.08E-03 | 0.9698 |
| 1C10 | 1.11E-08 | 2.50E+05 | 2.78E-03 | 0.9565 |

**Table 8 Dissociation and Association Constants of the Candidates with mFGFR2b**

| Sample ID | KD (M) | kon(1/Ms) | kdis(1/s) | Full R^2 |
|---|---|---|---|---|
| 2B1 | 2.30E-08 | 5.48E+04 | 1.26E-03 | 0.9959 |
| 3A12 | 1.07E-08 | 4.54E+04 | 4.85E-04 | 0.9945 |
| 3A8 | 7.17E-09 | 4.27E+04 | 3.06E-04 | 0.9954 |
| 3C7 | 8.71E-09 | 4.19E+04 | 3.65E-04 | 0.995 |
| 3C9 | 9.95E-09 | 4.85E+04 | 4.83E-04 | 0.9932 |
| 3D6 | 2.17E-08 | 2.74E+04 | 5.93E-04 | 0.9924 |
| 3E1 | 2.18E-08 | 5.78E+04 | 1.26E-03 | 0.975 |
| 3E2 | 3.65E-08 | 4.18E+04 | 1.52E-03 | 0.9675 |
| 3E3 | 5.27E-08 | 4.05E+04 | 2.14E-03 | 0.9614 |
| 3E7 | 4.09E-08 | 4.28E+04 | 1.75E-03 | 0.959 |
| 3F5 | 3.61E-08 | 4.48E+04 | 1.62E-03 | 0.9678 |
| 3F10 | 5.25E-08 | 4.13E+04 | 2.17E-03 | 0.9654 |
| 3G10 | 4.19E-08 | 4.66E+04 | 1.95E-03 | 0.9618 |
| 3H6 | 4.37E-08 | 3.57E+04 | 1.56E-03 | 0.9807 |
| 3H9 | 2.38E-08 | 5.42E+04 | 1.29E-03 | 0.9662 |
| 2B2 | 5.35E-08 | 4.39E+04 | 2.35E-03 | 0.9707 |
| 3A3 | 3.96E-08 | 3.08E+04 | 1.22E-03 | 0.9728 |
| 3B4 | 2.73E-08 | 5.49E+04 | 1.50E-03 | 0.9786 |
| 3B7 | 3.39E-08 | 5.07E+04 | 1.72E-03 | 0.9812 |
| 3D7 | 8.17E-08 | 2.91E+04 | 2.38E-03 | 0.966 |
| 3F11 | 2.60E-08 | 5.78E+04 | 1.50E-03 | 0.9819 |
| 1A3 | 9.98E-08 | 5.42E+04 | 5.40E-03 | 0.9608 |
| 1G6 | 4.22E-08 | 3.04E+04 | 1.28E-03 | 0.9753 |
| 1B8 | 7.49E-08 | 6.40E+04 | 4.80E-03 | 0.9513 |
| 1D7 | 2.76E-08 | 4.25E+04 | 1.17E-03 | 0.9729 |
| 1C10 | 7.56E-08 | 2.86E+04 | 2.16E-03 | 0.9629 |

### 2.5 ELISA Characterization of Antibody Binding Specificity to Various FGFR Family Members

LISA was used to characterize the binding specificity of antibodies (scFv-Fc) to various FGFR family members (i.e., FGFR1, FGFR2c, and FGFR4). All proteins were purchased from acro. The antibody incubation concentration was 10 µg/mL. The results are shown in Tables 9-11. The results indicate that all antibodies did not bind or bound weakly to FGFR1, FGFR2c, and FGFR4, indicating good specificity.

**Table 9 ELSIA results of candidate binding to FGFR2c**

| Clone (No.) | OD (450 nm) value | Clone (No.) | OD (450 nm) value |
|---|---|---|---|
| 2B1 | 0.098 | 3G10 | 0.096 |
| 3A12 | 0.098 | 3H6 | 0.093 |
| 3C3 | 0.106 | 3H9 | 0.45 |
| 3C7 | 0.091 | 3B4 | 0.327 |
| 3C9 | 0.118 | 3B7 | 0.089 |
| 3D6 | 0.542 | 3D7 | 0.127 |
| 3E1 | 0.118 | 3F11 | 0.086 |
| 3E2 | 0.138 | 1G6 | 0.176 |
| 3E3 | 0.199 | 1B8 | 0.076 |
| 3E7 | 0.114 | 1D7 | 0.091 |
| 3F5 | 0.111 | 1C10 | 0.553 |
| 3F10 | 0.106 | 1A3 | 0.134 |
| 2B2 | 0.113 | 3A3 | 0.137 |

**Table 10 ELSIA results of candidate binding to FGFR1**

| Clone (No.) | OD (450 nm) value | Clone (No.) | OD (450 nm) value |
|---|---|---|---|
| 2B1 | 0.076 | 3G10 | 0.083 |
| 3A12 | 0.076 | 3H6 | 0.077 |
| 3C3 | 0.085 | 3H9 | 0.646 |
| 3C7 | 0.077 | 3B4 | 0.458 |
| 3C9 | 0.113 | 3B7 | 0.083 |
| 3D6 | 0.693 | 3D7 | 0.192 |
| 3E1 | 0.08 | 3F11 | 0.1 |
| 3E2 | 0.122 | 1G6 | 0.217 |
| 3E3 | 0.19 | 1B8 | 0.092 |
| 3E7 | 0.093 | 1D7 | 0.074 |
| 3F5 | 0.108 | 1C10 | 0.626 |
| 3F10 | 0.086 | 1A3 | 0.143 |
| 2B2 | 0.109 | 3A3 | 0.097 |

**Table 11 ELSIA results of candidate binding to FGFR4**

| Clone (No.) | OD (450 nm) value | Clone (No.) | OD (450 nm) value |
|---|---|---|---|
| 2B1 | 0.103 | 3G10 | 0.175 |
| 3A12 | 0.106 | 3H6 | 0.157 |
| 3C3 | 0.124 | 3H9 | 0.649 |
| 3C7 | 0.133 | 3B4 | 0.524 |
| 3C9 | 0.139 | 3B7 | 0.116 |
| 3D6 | 0.797 | 3D7 | 0.256 |
| 3E1 | 0.096 | 3F11 | 0.093 |
| 3E2 | 0.149 | 1G6 | 0.232 |
| 3E3 | 0.265 | 1B8 | 0.171 |
| 3E7 | 0.125 | 1D7 | 0.156 |
| 3F5 | 0.145 | 1C10 | 0.588 |
| 3F10 | 0.123 | 1A3 | 0.099 |
| 2B2 | 0.093 | 3A3 | 0.136 |

### Example 3 Evaluation of in Vitro Activity Using a HEK293/SRE-Luc-FGFR2b Cell Model

A HEK293/SRE-Luc-FGFR2b cell model was constructed using lentiviral technology. Upon stimulation with KGF, FGF1 and FGF10, tyrosine phosphorylation of the FGFR2b protein on the HEK293/SRE-Luc-FGFR2b cell membrane was upregulated, activating the FGFR2b downstream signaling pathway.

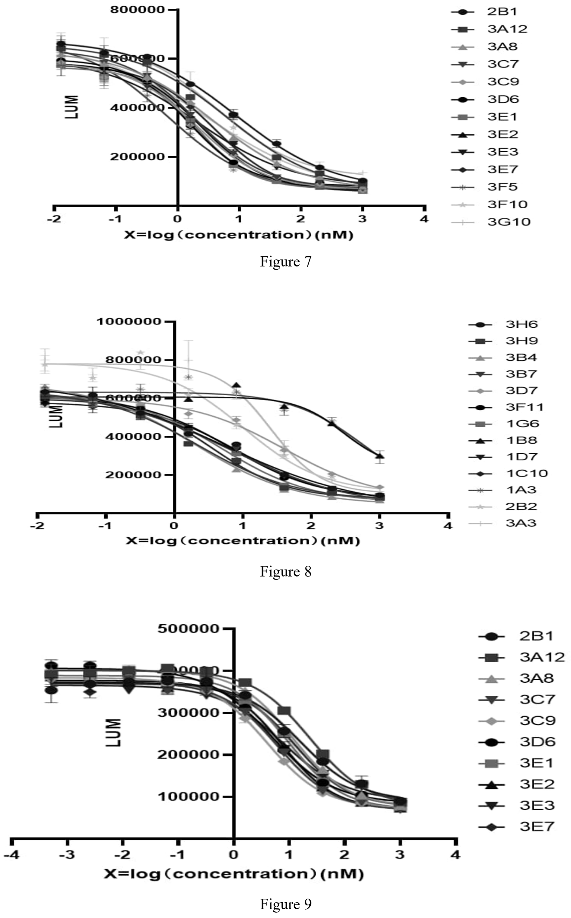

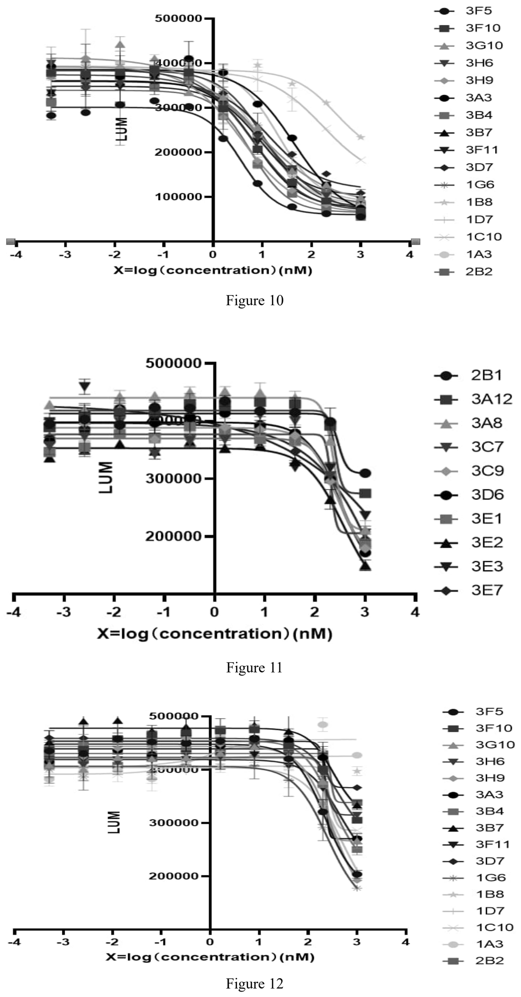

High-affinity monoclonal antibodies that block the binding of KGF, FGF1 and FGF10 to FGFR2b were screened for cellular activity. The test proteins were able to bind to the FGFR2b protein on the HEK293/SRE-Luc-FGFR2b cell membrane, blocking stimulation with KGF, FGF1 and FGF10 and downregulating tyrosine phosphorylation of the FGFR2b protein. In the experiment, the concentration of the ligand was quantified, and the in vitro activity of the monoclonal antibody was measured by chemiluminescence to determine the level of tyrosine phosphorylation downregulated by FGFR2b under different concentrations of the monoclonal antibody. The monoclonal antibody described was the scFv-Fc monoclonal antibody prepared in Example 2. The level of tyrosine phosphorylation downregulated by FGFR2b under KGF stimulation is shown in Table 12 and Figures 7-8; the level of tyrosine phosphorylation downregulated by FGF10 is shown in Table 13 and Figures 9-10; and the level of tyrosine phosphorylation downregulated by FGF1 is shown in Figures 11-12. The results show that tyrosine phosphorylation downregulated by FGFR2b under KGF and FGF10 stimulation is significant. Under FGF1 stimulation, there is a high degree of monoclonal antibody dependence, but there is a certain level of tyrosine phosphorylation downregulation.

**Table 12 Level of Tyrosine Phosphorylation Downregulated by FGFR2b under KGF Stimulation**

| Clone (No.) | IC50 (nM) | Clone (No.) | IC50 (nM) |
|---|---|---|---|
| 2B1 | 8.741 | 3H6 | 5.066 |
| 3A12 | 6.566 | 3H9 | 2.219 |
| 3A8 | 3.786 | 3B4 | 1.959 |
| 3C7 | 1.93 | 3B7 | 6.331 |
| 3C9 | 2.007 | 3D7 | 40.14 |
| 3D6 | 1.506 | 3F11 | 6.63 |
| 3E1 | 2.037 | 1G6 | 4.801 |
| 3E2 | 2.437 | 1B8 | 290.2 |
| 3E3 | 2.081 | 1D7 | 8.476 |
| 3E7 | 3.899 | 1C10 | 3.665 |
| 3F5 | 0.662 | 1A3 | 579.8 |
| 3F10 | 2.964 | 2B2 | 10.19 |
| 3G10 | 4.412 | 3A3 | 25.41 |

**Table 13 Level of Tyrosine Phosphorylation Downregulated by FGFR2b under KGF10 Stimulation**

| Clone (No.) | IC50 (nM) | Clone (No.) | IC50 (nM) |
|---|---|---|---|
| 2B1 | 15.85 | 3F5 | 3.733 |
| 3A12 | 23.48 | 3F10 | 6.405 |
| 3A8 | 11.7 | 3G10 | 7.467 |
| 3C7 | 6.189 | 3H6 | 12.91 |
| 3C9 | 4.019 | 3H9 | 3.816 |
| 3D6 | 4.64 | 3A3 | 44.02 |
| 3E1 | 9.718 | 3B4 | 5.814 |
| 3E2 | 7.473 | 3B7 | 7.267 |
| 3E3 | 7.617 | 3F11 | 9.253 |
| 3E7 | 12.79 | 3D7 | 13.21 |
| 1C10 | 167.6 | 1G6 | 11.22 |
| 1A3 | 8.765 | 1B8 | 320 |
| 2B2 | 11.7 | 1D7 | 20.59 |

### Example 4 Anti-tumor efficacy testing of FGFR2b antibodies in SNU16 subcutaneous transplant tumor model

Human gastric cancer cells (SNU16) in logarithmic growth phase (overexpressing FGFR2b) were centrifuged and counted. The cells were then adjusted to a cell density of approximately 5.0*10⁷/mL using a mixture of serum-free RPMI-1640 medium and Matrigel (at a 1:1 ratio). A volume of 0.1 mL/Mouse was injected subcutaneously into the backs of Babl/c-nude mice. When the average tumor volume reached approximately 100 mm³, the mice were randomly divided into groups for dosing. The corresponding antibodies were administered intraperitoneally twice weekly for 2-3 weeks. FR21, a Phase 3 clinical trial antibody targeting the same target, was used as a positive control. Starting from day 0 of dosing, tumor diameter was measured and mouse body weight was weighed twice weekly to calculate tumor volume and body weight trends. Tumor growth inhibition (TGI) was used as the experimental evaluation metric. (TGI)% = [1-T/C] × 100%, wherein T and C represent the tumor volumes at the end of the experiment. Statistical analysis was performed using SPSS 16.0 software, and inter-group comparisons were performed using one-way ANOVA. P < 0.05 (*) indicates statistical significance.

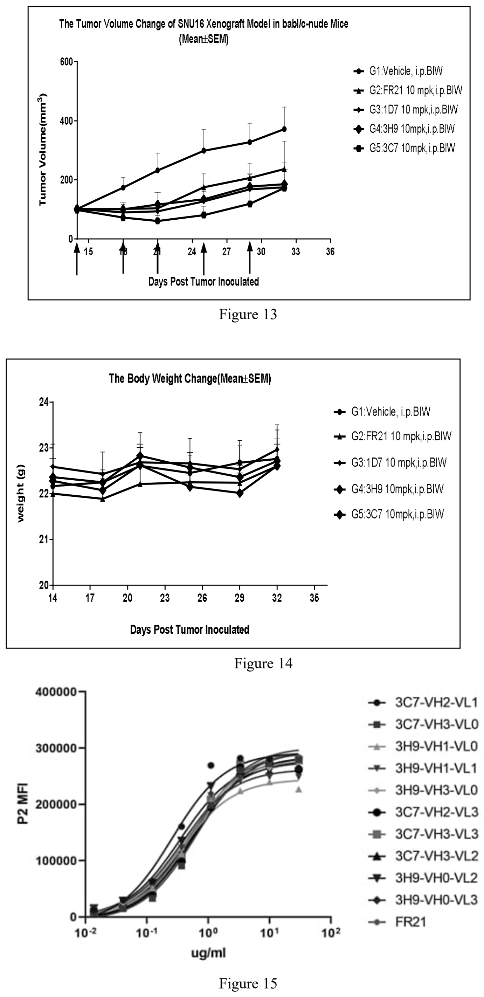

The experimental results are shown in Figures 13 and 14. After five doses, the positive monoclonal antibody FR21 (10 mpk) had a tumor inhibition TGI of 35.99%. The present invention's 1D7 10 mpk had a tumor inhibition TGI of 52.84%; 3C7 10 mpk had a tumor inhibition TGI of 50.36%; and 3H9 10 mpk had a tumor inhibition TGI of 53.33%. The tumor inhibition effects of 1D7, 3C7 and 3H9 at 10 mpk were superior to those of FR21 at the same dose. FGFR2 monoclonal antibody had no significant effect on the weight gain of tumor-bearing mice. Compared with before administration, the weight of mice in each group increased slightly, with a growth rate of 1.17~5.25%. All antibodies had good safety.

### Example 5 Preparation of Humanized Antibodies

The humanized mutant variable region sequences of murine 3C7 and 3H9, the variable region of the positive antibody FR21, and the humanized antibody constant region sequences were assembled using whole-genome gene synthesis. The resulting constructs were constructed into the pcDNA3.1 vector and transiently transfected into HEK293 cells. Purification using Protein A medium yielded humanized antibodies with a purity exceeding 95%, as shown in Table 14 below. The humanized heavy chain variable region and light chain variable region are as described herein, and the humanized heavy chain constant region is as described herein. An example of a humanized antibody of the present invention is 3C7-VH0-VL0, indicating that the heavy chain variable region of this humanized antibody is composed of 3C7-VH0, the light chain variable region is composed of 3C7-VL0, and the heavy chain constant region and light chain constant region are each composed of the IgG1 constant region described herein.

**Table 14 Humanized Antibody Numbers**

| Ab | Ab | Ab |
|---|---|---|
| FR21 | 3C7-VH2-VL2 | 3H9-VH1-VL1 |
| 3C7-VH0-VL0 | 3C7-VH2-VL3 | 3H9-VH1-VL2 |
| 3C7-VH0-VL1 | 3C7-VH3-VL0 | 3H9-VH1-VL3 |
| 3C7-VH0-VL2 | 3C7-VH3-VL1 | 3H9-VH2-VL0 |
| 3C7-VH0-VL3 | 3C7-VH3-VL2 | 3H9-VH2-VL1 |
| 3C7-VH1-VL0 | 3C7-VH3-VL3 | 3H9-VH2-VL2 |
| 3C7-VH1-VL1 | 3H9-VH0-VL0 | 3H9-VH2-VL3 |
| 3C7-VH1-VL2 | 3H9-VH0-VL1 | 3H9-VH3-VL0 |
| 3C7-VH1-VL3 | 3H9-VH0-VL2 | 3H9-VH3-VL1 |
| 3C7-VH2-VL0 | 3H9-VH0-VL3 | 3H9-VH3-VL2 |
| 3C7-VH2-VL1 | 3H9-VH1-VL0 | 3H9-VH3-VL3 |

### Example 6 Flow Cytometry Detection of Binding of Test Antibodies to Human FGFR2b

Flow cytometry was used to detect antibody binding to KATO III cells overexpressing FGFR2b. 1*10⁵ cells were resuspended in a 96-well plate and incubated with various concentrations of mAb for 1 hour. The cells were then washed twice in PBS and the bound antibody was detected by incubation with PE-goat anti-human IgG (Jackson ImmunoResearch) at 4 °C for 30 min. The cells were analyzed on a FACS (Beckman). The results are shown in Figures 15-17 and Table 15.

**Table 15**

| Antibody | EC50 (ug/mL) | Antibody | EC50 (ug/mL) | Antibody | EC50 (ug/mL) |
|---|---|---|---|---|---|
| FR21 | 0.5858 | 3C7-VH2-VL2 | 0.5591 | 3H9-VH1-VL1 | 0.3786 |
| 3C7-VH0-VL0 | 0.3835 | 3C7-VH2-VL3 | 0.572 | 3H9-VH1-VL2 | 0.09087 |
| 3C7-VH0-VL1 | 0.6403 | 3C7-VH3-VL0 | 0.6237 | 3H9-VH1-VL3 | 0.2146 |
| 3C7-VH0-VL2 | 0.5282 | 3C7-VH3-VL1 | 0.4457 | 3H9-VH2-VL0 | 0.3322 |
| 3C7-VH0-VL3 | 0.4425 | 3C7-VH3-VL2 | 0.5226 | 3H9-VH2-VL1 | 0.3094 |
| 3C7-VH1-VL0 | 0.3958 | 3C7-VH3-VL3 | 0.5363 | 3H9-VH2-VL2 | 0.3365 |
| 3C7-VH1-VL1 | 0.5061 | 3H9-VH0-VL0 | 0.336 | 3H9-VH2-VL3 | 0.2124 |
| 3C7-VH1-VL2 | 0.6006 | 3H9-VH0-VL1 | 0.1797 | 3H9-VH3-VL0 | 0.4369 |
| 3C7-VH1-VL3 | 0.5649 | 3H9-VH0-VL2 | 0.357 | 3H9-VH3-VL1 | 0.3573 |
| 3C7-VH2-VL0 | 0.4356 | 3H9-VH0-VL3 | 0.4685 | 3H9-VH3-VL2 | 0.2314 |
| 3C7-VH2-VL1 | 0.3022 | 3H9-VH1-VL0 | 0.3778 | 3H9-VH3-VL3 | 0.5538 |

### Example 7 BLI Method for Detecting the Affinity of the Test Antibodies for Human FGFR2b Protein

Binding of the antibodies to human FGFR2b protein was confirmed using biofilm interferometry. The positive control antibody FR21, was derived from patent US2019153105A1. An AHC2 (octet) sensor chip was activated in SD buffer (PBS, 0.1% BSA, 0.02% Tween). The antibody (5 µg/ml) was immobilized on the sensor chip and tested for binding to various concentrations of human FGFR2b protein. The association and dissociation constants were calculated using ForteBio Data Analysis software (version 9.0). The results are shown in Table 16 below. The affinity of the humanized antibody candidate was comparable to that of the positive control antibody FR21.

**Table 16**

| Antibody | KD (M) | kon(1/Ms) | kdis(1/s) | Full R^2 |
|---|---|---|---|---|
| FR21 | 1.52E-09 | 2.79E+05 | 4.24E-04 | 0.9895 |
| 3C7-VH0-VL0 | 1.04E-08 | 2.93E+05 | 3.05E-03 | 0.9893 |
| 3C7-VH0-VL1 | 1.40E-08 | 2.24E+05 | 3.15E-03 | 0.9899 |
| 3C7-VH0-VL2 | 1.16E-08 | 2.69E+05 | 3.13E-03 | 0.9863 |
| 3C7-VH0-VL3 | 1.36E-08 | 2.38E+05 | 3.23E-03 | 0.9883 |
| 3C7-VH1-VL0 | 1.89E-09 | 5.04E+05 | 9.55E-04 | 0.9865 |
| 3C7-VH1-VL2 | 2.71E-09 | 4.25E+05 | 1.15E-03 | 0.9847 |
| 3C7-VH1-VL3 | 3.04E-09 | 4.03E+05 | 1.22E-03 | 0.986 |
| 3C7-VH1-VL1 | 3.24E-09 | 4.05E+05 | 1.31E-03 | 0.987 |
| 3C7-VH2-VL0 | 1.63E-09 | 5.09E+05 | 8.29E-04 | 0.9872 |
| 3C7-VH2-VL1 | 2.92E-09 | 2.46E+05 | 7.18E-04 | 0.9827 |
| 3C7-VH2-VL2 | 2.03E-09 | 4.35E+05 | 8.84E-04 | 0.9835 |
| 3C7-VH2-VL3 | 2.77E-09 | 2.72E+05 | 7.54E-04 | 0.9812 |
| 3C7-VH3-VL0 | 2.51E-09 | 3.31E+05 | 8.31E-04 | 0.9839 |
| 3C7-VH3-VL1 | 2.46E-09 | 4.29E+05 | 1.06E-03 | 0.9848 |
| 3C7-VH3-VL2 | 2.48E-09 | 3.34E+05 | 8.29E-04 | 0.9892 |
| 3C7-VH3-VL3 | 2.43E-09 | 3.20E+05 | 7.77E-04 | 0.9888 |
| 3H9-VH0-VL0 | 6.51E-09 | 4.63E+05 | 3.01E-03 | 0.9891 |
| 3H9-VH0-VL1 | 3.55E-09 | 5.05E+05 | 1.79E-03 | 0.985 |
| 3H9-VH0-VL2 | 5.06E-09 | 3.67E+05 | 1.86E-03 | 0.9883 |
| 3H9-VH0-VL3 | 5.09E-09 | 3.66E+05 | 1.86E-03 | 0.9887 |
| 3H9-VH1-VL0 | 1.85E-08 | 2.34E+05 | 4.33E-03 | 0.9884 |
| 3H9-VH1-VL1 | 8.50E-09 | 2.84E+05 | 2.41E-03 | 0.9833 |
| 3H9-VH1-VL2 | 5.37E-09 | 4.32E+05 | 2.32E-03 | 0.9875 |
| 3H9-VH1-VL3 | 5.05E-09 | 5.03E+05 | 2.54E-03 | 0.9857 |
| 3H9-VH2-VL0 | 2.64E-09 | 4.73E+05 | 1.25E-03 | 0.9859 |
| 3H9-VH2-VL1 | 1.32E-09 | 5.70E+05 | 7.52E-04 | 0.9802 |
| 3H9-VH2-VL2 | 1.90E-09 | 4.12E+05 | 7.84E-04 | 0.9854 |
| 3H9-VH2-VL3 | 1.37E-09 | 5.75E+05 | 7.88E-04 | 0.9809 |
| 3H9-VH3-VL0 | 3.13E-09 | 2.99E+05 | 9.35E-04 | 0.9819 |
| 3H9-VH3-VL1 | 6.14E-10 | 5.27E+05 | 3.24E-04 | 0.9814 |
| 3H9-VH3-VL2 | 7.09E-10 | 5.26E+05 | 3.73E-04 | 0.982 |
| 3H9-VH3-VL3 | 7.43E-10 | 5.36E+05 | 3.99E-04 | 0.983 |

### Example 8 Evaluation of the in vitro activity of the test antibody using a HEK293/SRE-Luc-FGFR2b cell model

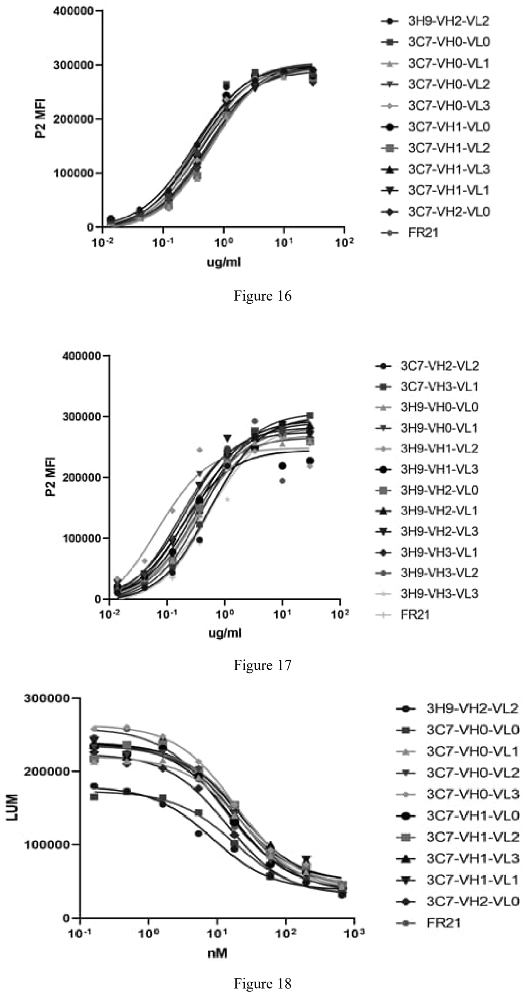

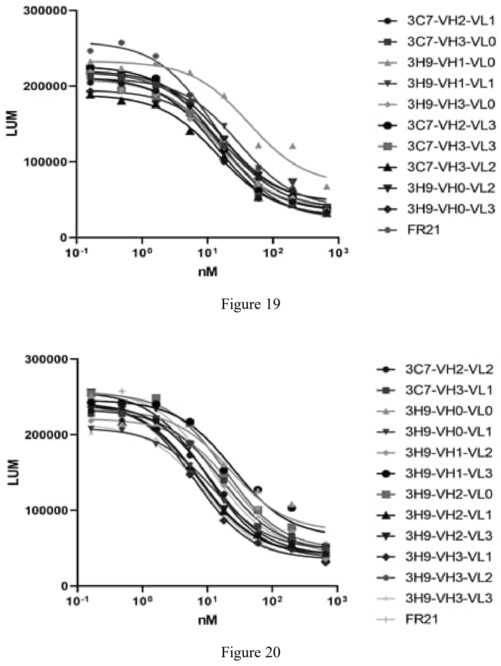

A HEK293/SRE-Luc-FGFR2b cell model was constructed using lentiviral technology. Upon stimulation with KGF, tyrosine phosphorylation of the FGFR2b protein on the HEK293/SRE-Luc-FGFR2b cell membrane was upregulated, activating the FGFR2b downstream signaling pathway. By binding to the FGFR2b protein on the HEK293/SRE-Luc-FGFR2b cell membrane, the test antibody may inhibit KGF-induced activation of the FGFR2b downstream signaling pathway. In the experiment, the ligand concentration was quantified, and chemiluminescence was used to detect the downregulation of tyrosine phosphorylation of the FGFR2b protein in response to different antibody concentrations. This served as a measure of the in vitro activity of the test antibody. The positive control antibody FR21, is derived from patent US2019153105A1. The results are shown in Figures 18-20 and Table 17. Except for 3H9-VH0-VL0, the in vitro activities of the humanized antibody candidates are comparable to or better than the positive antibody FR21.

**Table 17**

| Antibody | IC50 (nM) | Antibody | IC50 (nM) | Antibody | IC50 (nM) |
|---|---|---|---|---|---|
| FR21 | 12.67 | 3C7-VH2-VL2 | 10.6 | 3H9-VH1-VL1 | 33.44 |
| 3C7-VH0-VL0 | 16.23 | 3C7-VH2-VL3 | 13.89 | 3H9-VH1-VL2 | 25.46 |
| 3C7-VH0-VL1 | 21.79 | 3C7-VH3-VL0 | 16.81 | 3H9-VH1-VL3 | 27.3 |
| 3C7-VH0-VL2 | 20.32 | 3C7-VH3-VL1 | 16.31 | 3H9-VH2-VL0 | 19.12 |
| 3C7-VH0-VL3 | 19.38 | 3C7-VH3-VL2 | 14.99 | 3H9-VH2-VL1 | 8.99 |
| 3C7-VH1-VL0 | 15.87 | 3C7-VH3-VL3 | 19.59 | 3H9-VH2-VL2 | 9.424 |
| 3C7-VH1-VL1 | 21.23 | 3H9-VH0-VL0 | 438.3 | 3H9-VH2-VL3 | 10.18 |
| 3C7-VH1-VL2 | 19.14 | 3H9-VH0-VL1 | 12.36 | 3H9-VH3-VL0 | 17.16 |
| 3C7-VH1-VL3 | 18.01 | 3H9-VH0-VL2 | 17.83 | 3H9-VH3-VL1 | 6.256 |
| 3C7-VH2-VL0 | 14.84 | 3H9-VH0-VL3 | 21.73 | 3H9-VH3-VL2 | 8.09 |
| 3C7-VH2-VL1 | 13.44 | 3H9-VH1-VL0 | 45.91 | 3H9-VH3-VL3 | 10.66 |

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples. **In** addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. An antibody or an antigen-binding fragment thereof that binds to FGFR2, wherein the antibody comprises a heavy chain variable region and a light chain variable region; the heavy chain variable region comprises: VH-CDR1, VH-CDR2 and VH-CDR3,
wherein, the VH-CDR1 has an amino acid sequence shown in SEQ ID NO: 58 or SEQ ID NO: 59,
X1YNX2H (SEQ ID NO: 58),
X1YYX2H (SEQ ID NO: 59),
wherein, X1 is selected from T, N, D, S, F, I, Y or P, and X2 is selected from I, M, L or V,
the VH-CDR2 has an amino acid sequence shown in SEQ ID NO: 60 or SEQ ID NO: 61,
AIYPDNGDTX4YNX5X6FX7X8 (SEQ ID NO: 60),
LIYPX3NGDTSYNQKFX7X8 (SEQ ID NO: 61),
wherein, X3 is selected from D or E, X4 is selected from S or F, X5 is selected from Q or R, X6 is selected from K or R, X7 is selected from K, R or T, and X8 is selected from V, D, G or A,
the VH-CDR3 has an amino acid sequence shown in SEQ ID NO: 62 or SEQ ID NO: 26,
GX9X10X11Y (SEQ ID NO: 62),
AFTY (SEQ ID NO: 26),
wherein, X9 is selected from D or G, X10 is selected from F or L, X11 is selected from A or D; and
the light chain variable region comprises: VL-CDR1, VL-CDR2 and VL-CDR3, wherein, the VL-CDR1 has an amino acid sequence shown in SEQ ID NO: 35, SEQ ID NO: 37 or SEQ ID NO: 126,
X12ASX13X14X15X16X17X18X19X20 (SEQ ID NO: 126),
wherein, X12 is selected from G, R or K, X13 is selected from E, Q or G, X14 is selected from N or D, X15 is selected from I, L or V, X16 is selected from Y, S, G, N or H, X17 is selected from G, S, T or N, X18 is selected from A, N, Y or D, X19 is selected from L or V, X20 is selected from N, A or S,
QATQDIVKNLN (SEQ ID NO: 37),
KASQSVDYDGDSYMN (SEQ ID NO: 35),
the VL-CDR2 has an amino acid sequence shown in SEQ ID NO: 45, SEQ ID NO: 127 or SEQ ID NO: 128,
SASYRX21 (SEQ ID NO: 127),
wherein, X21 is selected from S or T,
GISNRFS (SEQ ID NO: 45),
X22AX23X24LAX25 (SEQ ID NO: 128),
wherein, X22 is selected from G, A, N or Y, X23 is selected from T or K, X24 is selected from T, N or E, and X25 is selected from D or E,
the VL-CDR3 has an amino acid sequence shown in SEQ ID NO: 129 or SEQ ID NO: 130,
QX26X27X28X29TPX30T (SEQ ID NO: 129),
wherein, X26 is selected from N, H or Q, X27 is selected from V, F or H, X28 is selected from L, W or Y, X29 is selected from S, N or G, and X30 is selected from Y or F,
LQX31X32X33X34PX35T (SEQ ID NO: 130),
wherein, X31 is selected from G or F, X32 is selected from T or Y, X33 is selected from H or E, X34 is selected from Q or F, and X35 is selected from Y or L.

2. The antibody or an antigen-binding fragment thereof of claim 1, wherein,
(a) the VH-CDR1 has an amino acid sequence shown in SEQ ID NO: 2 or SEQ ID NO: 3,
NYYIH (SEQ ID NO: 2),
DYYIH (SEQ ID NO: 3),
the VH-CDR2 has an amino acid sequence shown in SEQ ID NO: 15 or SEQ ID NO: 16 or SEQ ID NO: 149,
AIYPDNGDTSYNQRFRV (SEQ ID NO: 15),
AIYPDNGDTSYNQKFKV (SEQ ID NO: 16),
AIYPDNGDTSYNQRFKV (SEQ ID NO: 149), the VH-CDR3 has an amino acid sequence SEQ ID NO: 26,
AFTY (SEQ ID NO: 26), or
(b) wherein, the VH-CDR1 has an amino acid sequence shown in any one of SEQ ID NO: 4-13,
TYNMH (SEQ ID NO: 4),
SYNLH (SEQ ID NO: 5),
FYNVH (SEQ ID NO: 6),
IYNIH (SEQ ID NO: 7),
FYNIH (SEQ ID NO: 8),
YYNMH (SEQ ID NO: 9),
PYNIH (SEQ ID NO: 10),
TYNIH (SEQ ID NO: 11),
FYNMH (SEQ ID NO: 12),
YYNVH (SEQ ID NO: 13),
the VH-CDR2 has an amino acid sequence shown in any one of SEQ ID NO: 17-22,
AIYPDNGDTFYNRKFKD (SEQ ID NO: 17),
LIYPENGDTSYNQKFKG (SEQ ID NO: 18),
LIYPDNGDTSYNQKFKA (SEQ ID NO: 19),
LIYPDNGDTSYNQKFKG (SEQ ID NO: 20),
LIYPENGDTSYNQKFKD (SEQ ID NO: 21),
LIYPDNGDTSYNQKFTG (SEQ ID NO: 22),
the VH-CDR3 has an amino acid sequence shown in any one of SEQ ID NO: 23-25,
GDFAY (SEQ ID NO: 23),
GGFDY (SEQ ID NO: 24),
GGLDY (SEQ ID NO: 25).

3. The antibody or an antigen-binding fragment thereof of any one of claims 1 or 2, wherein the light chain variable region comprises: VL-CDR1, VL-CDR2 and VL-CDR3,
wherein, the VL-CDR1 has an amino acid sequence shown in any one of SEQ ID NO: 28-38,
the VL-CDR2 has an amino acid sequence shown in any one of SEQ ID NO: 39-46,
the VL-CDR3 has an amino acid sequence shown in any one of SEQ ID NO: 48-57.

4. The antibody or an antigen-binding fragment thereof of any one of claims 1-3, wherein the antibody comprises:
a heavy chain variable region comprising VH-CDR1, VH-CDR2 and VH-CDR3 shown in SEQ ID NO: 2, 15 and 26; or
a heavy chain variable region comprising VH-CDR1, VH-CDR2 and VH-CDR3 shown in SEQ ID NO: 3, 16 and 26; or
a heavy chain variable region comprising VH-CDR1, VH-CDR2 and VH-CDR3 shown in SEQ ID NO: 2, 149 and 26; or
a heavy chain variable region comprising VH-CDR1, VH-CDR2 and VH-CDR3 shown in SEQ ID NO: 7, 19 and 24; or
a heavy chain variable region comprising VH-CDR1, VH-CDR2 and VH-CDR3 shown in SEQ ID NO: 4, 17 and 23; or
a heavy chain variable region comprising VH-CDR1, VH-CDR2 and VH-CDR3 shown in SEQ ID NO: 5, 18 and 24; or
a heavy chain variable region comprising VH-CDR1, VH-CDR2 and VH-CDR3 shown in SEQ ID NO: 6, 19 and 24; or
a heavy chain variable region comprising VH-CDR1, VH-CDR2 and VH-CDR3 shown in SEQ ID NO: 8, 19 and 24; or
a heavy chain variable region comprising VH-CDR1, VH-CDR2 and VH-CDR3 shown in SEQ ID NO: 9, 20 and 24; or
a heavy chain variable region comprising VH-CDR1, VH-CDR2 and VH-CDR3 shown in SEQ ID NO: 10, 19 and 24; or
a heavy chain variable region comprising VH-CDR1, VH-CDR2 and VH-CDR3 shown in SEQ ID NO: 5, 18 and 24; or
a heavy chain variable region comprising VH-CDR1, VH-CDR2 and VH-CDR3 shown in SEQ ID NO: 5, 21 and 24; or
a heavy chain variable region comprising VH-CDR1, VH-CDR2 and VH-CDR3 shown in SEQ ID NO: 11, 19 and 24; or
a heavy chain variable region comprising VH-CDR1, VH-CDR2 and VH-CDR3 shown in SEQ ID NO: 9, 20 and 25; or
a heavy chain variable region comprising VH-CDR1, VH-CDR2 and VH-CDR3 shown in SEQ ID NO: 12, 20 and 25; or
a heavy chain variable region comprising VH-CDR1, VH-CDR2 and VH-CDR3 shown in SEQ ID NO: 13, 22 and 25.

5. The antibody or an antigen-binding fragment thereof of any one of claims 1-4, wherein the antibody comprises:
a light chain variable region comprising VL-CDR1, VL-CDR2 and VL-CDR3 shown in SEQ ID NO: 28, 40 and 48; or
a light chain variable region comprising VL-CDR1, VL-CDR2 and VL-CDR3 shown in SEQ ID NO: 27, 39 and 47; or
a light chain variable region comprising VL-CDR1, VL-CDR2 and VL-CDR3 shown in SEQ ID NO: 32, 42 and 50; or
a light chain variable region comprising VL-CDR1, VL-CDR2 and VL-CDR3 shown in SEQ ID NO: 30, 41 and 49; or
a light chain variable region comprising VL-CDR1, VL-CDR2 and VL-CDR3 shown in SEQ ID NO: 31, 42 and 50; or
a light chain variable region comprising VL-CDR1, VL-CDR2 and VL-CDR3 shown in SEQ ID NO: 32, 42 and 51; or
a light chain variable region comprising VL-CDR1, VL-CDR2 and VL-CDR3 shown in SEQ ID NO: 33, 43 and 52; or
a light chain variable region comprising VL-CDR1, VL-CDR2 and VL-CDR3 shown in SEQ ID NO: 32, 42 and 53; or
a light chain variable region comprising VL-CDR1, VL-CDR2 and VL-CDR3 shown in SEQ ID NO: 34, 39 and 51; or
a light chain variable region comprising VL-CDR1, VL-CDR2 and VL-CDR3 shown in SEQ ID NO: 30, 41 and 54; or
a light chain variable region comprising VL-CDR1, VL-CDR2 and VL-CDR3 shown in SEQ ID NO: 32, 39 and 50; or
a light chain variable region comprising VL-CDR1, VL-CDR2 and VL-CDR3 shown in SEQ ID NO: 30, 44 and 55; or
a light chain variable region comprising VL-CDR1, VL-CDR2 and VL-CDR3 shown in SEQ ID NO: 35, 45 and 56; or
a light chain variable region comprising VL-CDR1, VL-CDR2 and VL-CDR3 shown in SEQ ID NO: 36, 39 and 51; or
a light chain variable region comprising VL-CDR1, VL-CDR2 and VL-CDR3 shown in SEQ ID NO: 37, 46 and 57; or
a light chain variable region comprising VL-CDR1, VL-CDR2 and VL-CDR3 shown in SEQ ID NO: 38, 41 and 49.

6. The antibody or an antigen-binding fragment thereof of any one of claims 1-5, wherein the antibody comprises:
a heavy chain variable region comprising VH-CDR1, VH-CDR2 and VH-CDR3 shown in SEQ ID NO: 2, 15 and 26 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 2, 149 and 26 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 3, 16 and 26 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 7, 19 and 24 and a light chain variable region comprising VL-CDR1, VL-CDR2 and VL-CDR3 shown in SEQ ID NO: 32, 42 and 50; or
a heavy chain variable region comprising VH-CDR1, VH-CDR2 and VH-CDR3 shown in SEQ ID NO: 4, 17 and 23 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 5, 18 and 24 and a light chain variable region comprising VL-CDR1, VL-CDR2 and VL-CDR3 shown in SEQ ID NO: 30, 41 and 49; or
a heavy chain variable region comprising VH-CDR1, VH-CDR2 and VH-CDR3 shown in SEQ ID NO: 6, 19 and 24 and a light chain variable region comprising VL-CDR1, VL-CDR2 and VL-CDR3 shown in SEQ ID NO: 31, 42 and 50; or
a heavy chain variable region comprising VH-CDR1, VH-CDR2 and VH-CDR3 shown in SEQ ID NO: 7, 19 and 24 and a light chain variable region comprising VL-CDR1, VL-CDR2 and VL-CDR3 shown in SEQ ID NO: 32, 42 and 51; or
a heavy chain variable region comprising VH-CDR1, VH-CDR2 and VH-CDR3 shown in SEQ ID NO: 8, 19 and 24 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 8, 19 and 24 and a light chain variable region comprising VL-CDR1, VL-CDR2 and VL-CDR3 shown in SEQ ID NO: 33, 43 and 52; or
a heavy chain variable region comprising VH-CDR1, VH-CDR2 and VH-CDR3 shown in SEQ ID NO: 9, 20 and 24 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 10, 19 and 24 and a light chain variable region comprising VL-CDR1, VL-CDR2 and VL-CDR3 shown in SEQ ID NO: 32, 42 and 53; or
a heavy chain variable region comprising VH-CDR1, VH-CDR2 and VH-CDR3 shown in SEQ ID NO: 5, 18 and 24 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 5, 21 and 24 and a light chain variable region comprising VL-CDR1, VL-CDR2 and VL-CDR3 shown in SEQ ID NO: 32, 42 and 50; or
a heavy chain variable region comprising VH-CDR1, VH-CDR2 and VH-CDR3 shown in SEQ ID NO: 11, 19 and 24 and a light chain variable region comprising VL-CDR1, VL-CDR2 and VL-CDR3 shown in SEQ ID NO: 34, 39 and 51; or
a heavy chain variable region comprising VH-CDR1, VH-CDR2 and VH-CDR3 shown in SEQ ID NO: 9, 20 and 25 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 9, 20 and 25 and a light chain variable region comprising VL-CDR1, VL-CDR2 and VL-CDR3 shown in SEQ ID NO: 30, 41 and 54; or
a heavy chain variable region comprising VH-CDR1, VH-CDR2 and VH-CDR3 shown in SEQ ID NO: 12, 20 and 25 and a light chain variable region comprising VL-CDR1, VL-CDR2 and VL-CDR3 shown in SEQ ID NO: 32, 39 and 50; or
a heavy chain variable region comprising VH-CDR1, VH-CDR2 and VH-CDR3 shown in SEQ ID NO: 13, 22 and 25 and a light chain variable region comprising VL-CDR1, VL-CDR2 and VL-CDR3 shown in SEQ ID NO: 28, 40 and 48; or
a heavy chain variable region comprising VH-CDR1, VH-CDR2 and VH-CDR3 shown in SEQ ID NO: 12, 20 and 25 and a light chain variable region comprising VL-CDR1, VL-CDR2 and VL-CDR3 shown in SEQ ID NO: 30, 44 and 55; or
a heavy chain variable region comprising VH-CDR1, VH-CDR2 and VH-CDR3 shown in SEQ ID NO: 12, 20 and 25 and a light chain variable region comprising VL-CDR1, VL-CDR2 and VL-CDR3 shown in SEQ ID NO: 35, 45 and 56; or
a heavy chain variable region comprising VH-CDR1, VH-CDR2 and VH-CDR3 shown in SEQ ID NO: 9, 20 and 25 and a light chain variable region comprising VL-CDR1, VL-CDR2 and VL-CDR3 shown in SEQ ID NO: 36, 39 and 51; or
a heavy chain variable region comprising VH-CDR1, VH-CDR2 and VH-CDR3 shown in SEQ ID NO: 9, 20 and 25 and a light chain variable region comprising VL-CDR1, VL-CDR2 and VL-CDR3 shown in SEQ ID NO: 37, 46 and 57; or
a heavy chain variable region comprising VH-CDR1, VH-CDR2 and VH-CDR3 shown in SEQ ID NO: 9, 20 and 25 and a light chain variable region comprising VL-CDR1, VL-CDR2 and VL-CDR3 shown in SEQ ID NO: 38, 41 and 49.

7. The antibody or an antigen-binding fragment thereof of any one of claims 1-6, wherein the antibody comprises at least one of a heavy chain framework region sequence and a light chain framework region sequence, and at least a part of at least one of the heavy chain framework region sequence and the light chain framework region sequence is derived from at least one of a murine antibody, a primate antibody, or a mutant thereof.

8. The antibody or an antigen-binding fragment thereof of any one of claims 1-7, wherein the antibody comprises: a heavy chain variable region having an amino acid sequence with at least 85% identical to any one of SEQ ID NO: 64-89.

9. The antibody or an antigen-binding fragment thereof of any one of claims 1-7, wherein the antibody comprises: a light chain variable region having an amino acid sequence with at least 85% identical to any one of SEQ ID NO: 91-116.

10. The antibody or an antigen-binding fragment thereof of any one of claims 1-9, wherein the antibody comprises:
(a) a heavy chain variable region having an amino acid sequence with at least 85% identical to SEQ ID NO: 64, and a light chain variable region having an amino acid sequence with at least 85% identical to SEQ ID NO: 106; or
(b) a heavy chain variable region having an amino acid sequence with at least 85% identical to SEQ ID NO: 65, and a light chain variable region having an amino acid sequence with at least 85% identical to SEQ ID NO: 92; or
(c) a heavy chain variable region having an amino acid sequence with at least 85% identical to SEQ ID NO: 66, and a light chain variable region having an amino acid sequence with at least 85% identical to SEQ ID NO: 93; or
(d) a heavy chain variable region having an amino acid sequence with at least 85% identical to SEQ ID NO: 67, and a light chain variable region having an amino acid sequence with at least 85% identical to SEQ ID NO: 94; or
(e) a heavy chain variable region having an amino acid sequence with at least 85% identical to SEQ ID NO: 68, and a light chain variable region having an amino acid sequence with at least 85% identical to SEQ ID NO: 95; or
(f) a heavy chain variable region having an amino acid sequence with at least 85% identical to SEQ ID NO: 69, and a light chain variable region having an amino acid sequence with at least 85% identical to SEQ ID NO: 96; or
(g) a heavy chain variable region having an amino acid sequence with at least 85% identical to SEQ ID NO: 70, and a light chain variable region having an amino acid sequence with at least 85% identical to SEQ ID NO: 97; or
(h) a heavy chain variable region having an amino acid sequence with at least 85% identical to SEQ ID NO: 71, and a light chain variable region having an amino acid sequence with at least 85% identical to SEQ ID NO: 98; or
(i) a heavy chain variable region having an amino acid sequence with at least 85% identical to SEQ ID NO: 72, and a light chain variable region having an amino acid sequence with at least 85% identical to SEQ ID NO: 99; or
(j) a heavy chain variable region having an amino acid sequence with at least 85% identical to SEQ ID NO: 73, and a light chain variable region having an amino acid sequence with at least 85% identical to SEQ ID NO: 100; or
(k) a heavy chain variable region having an amino acid sequence with at least 85% identical to SEQ ID NO: 74, and a light chain variable region having an amino acid sequence with at least 85% identical to SEQ ID NO: 101; or
(l) a heavy chain variable region having an amino acid sequence with at least 85% identical to SEQ ID NO: 75, and a light chain variable region having an amino acid sequence with at least 85% identical to SEQ ID NO: 102; or
(m) a heavy chain variable region having an amino acid sequence with at least 85% identical to SEQ ID NO: 76, and a light chain variable region having an amino acid sequence with at least 85% identical to SEQ ID NO: 103; or
(n) a heavy chain variable region having an amino acid sequence with at least 85% identical to SEQ ID NO: 77, and a light chain variable region having an amino acid sequence with at least 85% identical to SEQ ID NO: 104; or
(o) a heavy chain variable region having an amino acid sequence with at least 85% identical to SEQ ID NO: 78, and a light chain variable region having an amino acid sequence with at least 85% identical to SEQ ID NO: 105; or
(p) a heavy chain variable region having an amino acid sequence with at least 85% identical to SEQ ID NO: 79, and a light chain variable region having an amino acid sequence with at least 85% identical to SEQ ID NO: 106; or
(q) a heavy chain variable region having an amino acid sequence with at least 85% identical to SEQ ID NO: 80, and a light chain variable region having an amino acid sequence with at least 85% identical to SEQ ID NO: 107; or
(r) a heavy chain variable region having an amino acid sequence with at least 85% identical to SEQ ID NO: 81, and a light chain variable region having an amino acid sequence with at least 85% identical to SEQ ID NO: 108; or
(s) a heavy chain variable region having an amino acid sequence with at least 85% identical to SEQ ID NO: 82, and a light chain variable region having an amino acid sequence with at least 85% identical to SEQ ID NO: 109; or
(t) a heavy chain variable region having an amino acid sequence with at least 85% identical to SEQ ID NO: 83, and a light chain variable region having an amino acid sequence with at least 85% identical to SEQ ID NO: 110; or
(u) a heavy chain variable region having an amino acid sequence with at least 85% identical to SEQ ID NO: 84, and a light chain variable region having an amino acid sequence with at least 85% identical to SEQ ID NO: 111; or
(v) a heavy chain variable region having an amino acid sequence with at least 85% identical to SEQ ID NO: 85, and a light chain variable region having an amino acid sequence with at least 85% identical to SEQ ID NO: 112; or
(w) a heavy chain variable region having an amino acid sequence with at least 85% identical to SEQ ID NO: 86, and a light chain variable region having an amino acid sequence with at least 85% identical to SEQ ID NO: 113; or
(x) a heavy chain variable region having an amino acid sequence with at least 85% identical to SEQ ID NO: 87, and a light chain variable region having an amino acid sequence with at least 85% identical to SEQ ID NO: 114; or
(y) a heavy chain variable region having an amino acid sequence with at least 85% identical to SEQ ID NO: 88, and a light chain variable region having an amino acid sequence with at least 85% identical to SEQ ID NO: 115; or
(z) a heavy chain variable region having an amino acid sequence with at least 85% identical to SEQ ID NO: 89, and a light chain variable region having an amino acid sequence with at least 85% identical to SEQ ID NO: 116.

11. The antibody or an antigen-binding fragment thereof of any one of claims 1-10, wherein the antibody comprises at least one of a heavy chain constant region and a light chain constant region, and at least a part of at least one of the heavy chain constant region and the light chain constant region is derived from at least one of a murine antibody, a primate antibody, or a mutant thereof;
preferably, the heavy chain constant region and light chain constant region of the antibody are both derived from human IgG antibodies or mutants thereof;
more preferably, the heavy chain constant region of the antibody is derived from a human IgG1 or IgG4 antibody or a mutant thereof;
more preferably, the light chain constant region of the antibody is derived from a human IgGκ or IgGλ constant region;
more preferably, the full-length sequence of the heavy chain constant region is shown in SEQ ID NO: 117, the full-length sequence of the light chain constant region is shown in SEQ ID NO: 118.

12. The antibody or an antigen-binding fragment thereof of any one of claims 1-11, wherein the antibody comprises: a heavy chain consisting of a heavy chain variable region having an amino acid sequence with at least 85% identical to any one of SEQ ID NO: 64-89, and a heavy chain constant region.

13. The antibody or an antigen-binding fragment thereof of any one of claims 1-11, wherein the antibody comprises: a light chain consisting of a light chain variable region having an amino acid sequence with at least 85% identical to any one of SEQ ID NO: 91-116, and a light chain constant region.

14. The antibody or an antigen-binding fragment thereof of any one of claims 1-13, wherein the antibody comprises:
(a) a heavy chain consisting of a heavy chain variable region having an amino acid sequence with at least 85% identical to SEQ ID NO: 64, and a heavy chain constant region, and a light chain consisting of a light chain variable region having an amino acid sequence with at least 85% identical to SEQ ID NO: 106, and a light chain constant region; or
(b) a heavy chain consisting of a heavy chain variable region having an amino acid sequence with at least 85% identical to SEQ ID NO: 65, and a heavy chain constant region, and a light chain consisting of a light chain variable region having an amino acid sequence with at least 85% identical to SEQ ID NO: 92, and a light chain constant region; or
(c) a heavy chain consisting of a heavy chain variable region having an amino acid sequence with at least 85% identical to SEQ ID NO: 66, and a heavy chain constant region, and a light chain consisting of a light chain variable region having an amino acid sequence with at least 85% identical to SEQ ID NO: 93, and a light chain constant region; or
(d) a heavy chain consisting of a heavy chain variable region having an amino acid sequence with at least 85% identical to SEQ ID NO: 67, and a heavy chain constant region, and a light chain consisting of a light chain variable region having an amino acid sequence with at least 85% identical to SEQ ID NO: 94, and a light chain constant region; or
(e) a heavy chain consisting of a heavy chain variable region having an amino acid sequence with at least 85% identical to SEQ ID NO: 68, and a heavy chain constant region, and a light chain consisting of a light chain variable region having an amino acid sequence with at least 85% identical to SEQ ID NO: 95, and a light chain constant region; or
(f) a heavy chain consisting of a heavy chain variable region having an amino acid sequence with at least 85% identical to SEQ ID NO: 69, and a heavy chain constant region, and a light chain consisting of a light chain variable region having an amino acid sequence with at least 85% identical to SEQ ID NO: 96, and a light chain constant region; or
(g) a heavy chain consisting of a heavy chain variable region having an amino acid sequence with at least 85% identical to SEQ ID NO: 70, and a heavy chain constant region, and a light chain consisting of a light chain variable region having an amino acid sequence with at least 85% identical to SEQ ID NO: 97, and a light chain constant region; or
(h) a heavy chain consisting of a heavy chain variable region having an amino acid sequence with at least 85% identical to SEQ ID NO: 71, and a heavy chain constant region, and a light chain consisting of a light chain variable region having an amino acid sequence with at least 85% identical to SEQ ID NO: 98, and a light chain constant region; or
(i) a heavy chain consisting of a heavy chain variable region having an amino acid sequence with at least 85% identical to SEQ ID NO: 72, and a heavy chain constant region, and a light chain consisting of a light chain variable region having an amino acid sequence with at least 85% identical to SEQ ID NO: 99, and a light chain constant region; or
(j) a heavy chain consisting of a heavy chain variable region having an amino acid sequence with at least 85% identical to SEQ ID NO: 73, and a heavy chain constant region, and a light chain consisting of a light chain variable region having an amino acid sequence with at least 85% identical to SEQ ID NO: 100, and a light chain constant region; or
(k) a heavy chain consisting of a heavy chain variable region having an amino acid sequence with at least 85% identical to SEQ ID NO: 74, and a heavy chain constant region, and a light chain consisting of a light chain variable region having an amino acid sequence with at least 85% identical to SEQ ID NO: 101, and a light chain constant region; or
(l) a heavy chain consisting of a heavy chain variable region having an amino acid sequence with at least 85% identical to SEQ ID NO: 75, and a heavy chain constant region, and a light chain consisting of a light chain variable region having an amino acid sequence with at least 85% identical to SEQ ID NO: 102, and a light chain constant region; or
(m) a heavy chain consisting of a heavy chain variable region having an amino acid sequence with at least 85% identical to SEQ ID NO: 76, and a heavy chain constant region, and a light chain consisting of a light chain variable region having an amino acid sequence with at least 85% identical to SEQ ID NO: 103, and a light chain constant region; or
(n) a heavy chain consisting of a heavy chain variable region having an amino acid sequence with at least 85% identical to SEQ ID NO: 77, and a heavy chain constant region, and a light chain consisting of a light chain variable region having an amino acid sequence with at least 85% identical to SEQ ID NO: 104, and a light chain constant region; or
(o) a heavy chain consisting of a heavy chain variable region having an amino acid sequence with at least 85% identical to SEQ ID NO: 78, and a heavy chain constant region, and a light chain consisting of a light chain variable region having an amino acid sequence with at least 85% identical to SEQ ID NO: 105, and a light chain constant region; or
(p) a heavy chain consisting of a heavy chain variable region having an amino acid sequence with at least 85% identical to SEQ ID NO: 79, and a heavy chain constant region, and a light chain consisting of a light chain variable region having an amino acid sequence with at least 85% identical to SEQ ID NO: 106, and a light chain constant region; or
(q) a heavy chain consisting of a heavy chain variable region having an amino acid sequence with at least 85% identical to SEQ ID NO: 80, and a heavy chain constant region, and a light chain consisting of a light chain variable region having an amino acid sequence with at least 85% identical to SEQ ID NO: 107, and a light chain constant region; or
(r) a heavy chain consisting of a heavy chain variable region having an amino acid sequence with at least 85% identical to SEQ ID NO: 81, and a heavy chain constant region, and a light chain consisting of a light chain variable region having an amino acid sequence with at least 85% identical to SEQ ID NO: 108, and a light chain constant region; or
(s) a heavy chain consisting of a heavy chain variable region having an amino acid sequence with at least 85% identical to SEQ ID NO: 82, and a heavy chain constant region, and a light chain consisting of a light chain variable region having an amino acid sequence with at least 85% identical to SEQ ID NO: 109, and a light chain constant region; or
(t) a heavy chain consisting of a heavy chain variable region having an amino acid sequence with at least 85% identical to SEQ ID NO: 83, and a heavy chain constant region, and a light chain consisting of a light chain variable region having an amino acid sequence with at least 85% identical to SEQ ID NO: 110, and a light chain constant region; or
(u) a heavy chain consisting of a heavy chain variable region having an amino acid sequence with at least 85% identical to SEQ ID NO: 84, and a heavy chain constant region, and a light chain consisting of a light chain variable region having an amino acid sequence with at least 85% identical to SEQ ID NO: 111, and a light chain constant region; or
(v) a heavy chain consisting of a heavy chain variable region having an amino acid sequence with at least 85% identical to SEQ ID NO: 85, and a heavy chain constant region, and a light chain consisting of a light chain variable region having an amino acid sequence with at least 85% identical to SEQ ID NO: 112, and a light chain constant region; or
(w) a heavy chain consisting of a heavy chain variable region having an amino acid sequence with at least 85% identical to SEQ ID NO: 86, and a heavy chain constant region, and a light chain consisting of a light chain variable region having an amino acid sequence with at least 85% identical to SEQ ID NO: 113, and a light chain constant region; or
(x) a heavy chain consisting of a heavy chain variable region having an amino acid sequence with at least 85% identical to SEQ ID NO: 87, and a heavy chain constant region, and a light chain consisting of a light chain variable region having an amino acid sequence with at least 85% identical to SEQ ID NO: 114, and a light chain constant region; or
(y) a heavy chain consisting of a heavy chain variable region having an amino acid sequence with at least 85% identical to SEQ ID NO: 88, and a heavy chain constant region, and a light chain consisting of a light chain variable region having an amino acid sequence with at least 85% identical to SEQ ID NO: 115, and a light chain constant region; or
(z) a heavy chain consisting of a heavy chain variable region having an amino acid sequence with at least 85% identical to SEQ ID NO: 89, and a heavy chain constant region, and a light chain consisting of a light chain variable region having an amino acid sequence with at least 85% identical to SEQ ID NO: 116, and a light chain constant region.

15. A humanized antibody or an antigen-binding fragment thereof, wherein the humanized antibody or the antigen-binding fragment thereof is derived from the chimeric antibody or the antigen-binding fragment thereof of any one of claims 1-14.

16. The humanized antibody or antigen-binding fragment thereof of claim 15, wherein the antibody comprises a heavy chain variable region and a light chain variable region;
the heavy chain variable region comprises: VH-CDR1, VH-CDR2 and VH-CDR3,
the VH-CDR1 has an amino acid sequence shown in SEQ ID NO: 2,
the VH-CDR2 has an amino acid sequence shown in SEQ ID NO: 149,
the VH-CDR3 has an amino acid sequence shown in SEQ ID NO: 26; and
the light chain variable region comprises: VL-CDR1, VL-CDR2 and VL-CDR3,
the VL-CDR1 has an amino acid sequence shown in X36ASENIYGALN, wherein X36 is selected from G or R,
the VL-CDR2 has an amino acid sequence shown in SEQ ID NO: 40,
the VL-CDR3 has an amino acid sequence shown in SEQ ID NO: 48.

17. The humanized antibody or antigen-binding fragment thereof of claim 15 or 16, wherein the antibody comprises a heavy chain variable region and a light chain variable region;
the heavy chain variable region comprises: VH-CDR1, VH-CDR2 and VH-CDR3,
the VH-CDR1 has an amino acid sequence shown in SEQ ID NO: 2,
the VH-CDR2 has an amino acid sequence shown in SEQ ID NO: 149,
the VH-CDR3 has an amino acid sequence shown in SEQ ID NO: 26; and
the light chain variable region comprises: VL-CDR1, VL-CDR2 and VL-CDR3,
the VL-CDR1 has an amino acid sequence shown in SEQ ID NO: 28 or SEQ ID NO: 150,
the VL-CDR2 has an amino acid sequence shown in SEQ ID NO: 40,
the VL-CDR2 has an amino acid sequence shown in SEQ ID NO: 48.

18. The humanized antibody or antigen-binding fragment thereof of any one of claims 15-17, wherein the antibody comprises a heavy chain variable region and a light chain variable region, the heavy chain variable region comprises a heavy chain framework region, and the light chain variable region comprises a light chain framework region;
the heavy chain framework region comprises: H-FR1, H-FR2, H-FR3 and H-FR4,
the H-FR1 has an amino acid sequence shown in QVQLVQSGAEVKKPGASVKVSCKASGYX37FX38, wherein X37 is selected from T or I, X38 is selected from T or R,
the H-FR2 has an amino acid sequence shown in WVX39QAPGQGLEWMG, wherein X39 is selected from R or K,
the H-FR3 has an amino acid sequence shown in SEQ ID NO: 156,
the H-FR4 has an amino acid sequence shown in SEQ ID NO: 157; and
the light chain framework region comprises: L-FR1, L-FR2, L-FR3 and L-FR4,
the L-FR1 has an amino acid sequence shown in SEQ ID NO: 158,
the L-FR2 has an amino acid sequence shown in WYQX40KPGKX41PKLLIY, wherein X40 is selected from Q or R, X41 is selected from A or S,
the L-FR3 has an amino acid sequence shown in GVPSRFSGSGSGTDX42TLTISSLQPEDX43ATYYC, wherein X42 is selected from F or Y, X43 is selected from F or V,
the L-FR4 has an amino acid sequence shown in FGGGTKX44EIK, wherein X44 is selected from V or L.

19. The humanized antibody or antigen-binding fragment thereof of any one of claims 15-18, wherein the antibody comprises a heavy chain variable region and a light chain variable region, the heavy chain variable region comprises a heavy chain framework region, and the light chain variable region comprises a light chain framework region;
the heavy chain framework region comprises: H-FR1, H-FR2, H-FR3 and H-FR4,
the H-FR1 has an amino acid sequence shown in SEQ ID NO: 151, SEQ ID NO: 152 or SEQ ID NO: 153,
the H-FR2 has an amino acid sequence shown in SEQ ID NO: 154 or SEQ ID NO: 155,
the H-FR3 has an amino acid sequence shown in SEQ ID NO: 156,
the H-FR4 has an amino acid sequence shown in SEQ ID NO: 157; and
the light chain framework region comprises: L-FR1, L-FR2, L-FR3 and L-FR4,
the L-FR1 has an amino acid sequence shown in SEQ ID NO: 158,
the L-FR2 has an amino acid sequence shown in SEQ ID NO: 159, SEQ ID NO: 160 or SEQ ID NO: 161,
the L-FR3 has an amino acid sequence shown in SEQ ID NO: 162, SEQ ID NO: 163 or SEQ ID NO: 164,
the L-FR4 has an amino acid sequence shown in SEQ ID NO: 165 or SEQ ID NO: 166.

20. The humanized antibody or an antigen-binding fragment thereof of any one of claims 15-19, wherein the antibody comprises a heavy chain variable region having an amino acid sequence with at least 85% identical to any one of SEQ ID NO: 167-170.

21. The humanized antibody or an antigen-binding fragment thereof of any one of claims 15-20, wherein the antibody comprises a light chain variable region having an amino acid sequence with at least 85% identical to any one of SEQ ID NO: 171-174.

22. The humanized antibody or an antigen-binding fragment thereof of any one of claims 15-21, wherein the antibody comprises
(a) a heavy chain variable region having an amino acid sequence with at least 85% identical to SEQ ID NO: 167, and a light chain variable region having an amino acid sequence with at least 85% identical to SEQ ID NO: 171; or
(b) a heavy chain variable region having an amino acid sequence with at least 85% identical to SEQ ID NO: 167, and a light chain variable region having an amino acid sequence with at least 85% identical to SEQ ID NO: 172; or
(c) the antibody comprises a heavy chain variable region having an amino acid sequence with at least 85% identical to SEQ ID NO: 167, and a light chain variable region having an amino acid sequence with at least 85% identical to SEQ ID NO: 173; or
(d) the antibody comprises a heavy chain variable region having an amino acid sequence with at least 85% identical to SEQ ID NO: 167, and a light chain variable region having an amino acid sequence with at least 85% identical to SEQ ID NO: 174; or
(e) a heavy chain variable region having an amino acid sequence with at least 85% identical to SEQ ID NO: 168, and a light chain variable region having an amino acid sequence with at least 85% identical to SEQ ID NO: 171; or
(f) a heavy chain variable region having an amino acid sequence with at least 85% identical to SEQ ID NO: 168, and a light chain variable region having an amino acid sequence with at least 85% identical to SEQ ID NO: 172; or
(g) the antibody comprises a heavy chain variable region having an amino acid sequence with at least 85% identical to SEQ ID NO: 168, and a light chain variable region having an amino acid sequence with at least 85% identical to SEQ ID NO: 173; or
(h) the antibody comprises a heavy chain variable region having an amino acid sequence with at least 85% identical to SEQ ID NO: 168, and a light chain variable region having an amino acid sequence with at least 85% identical to SEQ ID NO: 174; or
(i) a heavy chain variable region having an amino acid sequence with at least 85% identical to SEQ ID NO: 169, and a light chain variable region having an amino acid sequence with at least 85% identical to SEQ ID NO: 171; or
(j) a heavy chain variable region having an amino acid sequence with at least 85% identical to SEQ ID NO: 169, and a light chain variable region having an amino acid sequence with at least 85% identical to SEQ ID NO: 172; or
(k) the antibody comprises a heavy chain variable region having an amino acid sequence with at least 85% identical to SEQ ID NO: 169, and a light chain variable region having an amino acid sequence with at least 85% identical to SEQ ID NO: 173; or
(l) the antibody comprises a heavy chain variable region having an amino acid sequence with at least 85% identical to SEQ ID NO: 169, and a light chain variable region having an amino acid sequence with at least 85% identical to SEQ ID NO: 174; or
(m) a heavy chain variable region having an amino acid sequence with at least 85% identical to SEQ ID NO: 170, and a light chain variable region having an amino acid sequence with at least 85% identical to SEQ ID NO: 171; or
(n) a heavy chain variable region having an amino acid sequence with at least 85% identical to SEQ ID NO: 170, and a light chain variable region having an amino acid sequence with at least 85% identical to SEQ ID NO: 172; or
(o) the antibody comprises a heavy chain variable region having an amino acid sequence with at least 85% identical to SEQ ID NO: 170, and a light chain variable region having an amino acid sequence with at least 85% identical to SEQ ID NO: 173; or
(p) the antibody comprises a heavy chain variable region having an amino acid sequence with at least 85% identical to SEQ ID NO: 170, and a light chain variable region having an amino acid sequence with at least 85% identical to SEQ ID NO: 174.

23. The humanized antibody or antigen-binding fragment thereof of claim 15, wherein the antibody comprises a heavy chain variable region and a light chain variable region; the heavy chain variable region comprises: VH-CDR1, VH-CDR2 and VH-CDR3, the light chain variable region comprises: VL-CDR1, VL-CDR2 and VL-CDR3, and the heavy chain variable region comprises a heavy chain framework region, and the light chain variable region comprises a light chain framework region;
the heavy chain framework region comprises: H-FR1, H-FR2, H-FR3 and H-FR4, the light chain framework region comprises: L-FR1, L-FR2, L-FR3 and L-FR4,
wherein, the VH-CDR1, VH-CDR2 and VH-CDR3 have the amino acid sequences shown in SEQ ID NO: 12, SEQ ID NO: 20 and SEQ ID NO: 25, respectively;
the VL-CDR1, VL-CDR2 and VL-CDR3 have the amino acid sequences shown in SEQ ID NO: 30, SEQ ID NO: 44 and SEQ ID NO: 55, respectively;
the H-FR1 has an amino acid sequence shown in QVQLVQSGAEVKKPGASVKVSCKASGYTFX45, wherein, X45 is selected from T or S,
the H-FR2 has an amino acid sequence shown in WVX46QAPGQGLEWX47G, wherein, X46 is selected from R or K, X47 is selected from M or I,
the H-FR3 has an amino acid sequence shown in SEQ ID NO: 180,
the H-FR4 has an amino acid sequence shown in SEQ ID NO: 181,
the L-FR1 has an amino acid sequence shown in SEQ ID NO: 182,
the L-FR2 has an amino acid sequence shown in WYQQKPGKX48PKLLX49Y, wherein X48 is selected from A or S, X49 is selected from I or V,
the L-FR3 has an amino acid sequence shown in GVPSRFSGSGSGTDX50TLTISSLQPEDFATYYC, wherein, X50 is selected from F or Y,
the L-FR4 has an amino acid sequence shown in FGGGTKX51EIK, wherein X51 is selected from V or L.

24. The humanized antibody or an antigen-binding fragment thereof of claim 23, wherein,
the H-FR1 has an amino acid sequence shown in SEQ ID NO: 175 or SEQ ID NO: 176,
the H-FR2 has an amino acid sequence shown in SEQ ID NO: 177, SEQ ID NO: 178 or SEQ ID NO: 179,
the H-FR3 has an amino acid sequence shown in SEQ ID NO: 180,
the H-FR4 has an amino acid sequence shown in SEQ ID NO: 181,
the L-FR1 has an amino acid sequence shown in SEQ ID NO: 182,
the L-FR2 has an amino acid sequence shown in SEQ ID NO: 183, SEQ ID NO: 184 or SEQ ID NO: 185,
the L-FR3 has an amino acid sequence shown in SEQ ID NO: 186 or SEQ ID NO: 187,
the L-FR4 has an amino acid sequence shown in SEQ ID NO: 188 or SEQ ID NO: 189.

25. The humanized antibody or an antigen-binding fragment thereof of claim 23 or 24, wherein the antibody comprises a heavy chain variable region having an amino acid sequence with at least 85% identical to any one of SEQ ID NO: 190-193.

26. The humanized antibody or an antigen-binding fragment thereof of any one of claims 23-25, wherein the antibody comprises a light chain variable region having an amino acid sequence with at least 85% identical to any one of SEQ ID NO: 194-197.

27. The humanized antibody or an antigen-binding fragment thereof of any one of claims 23-26, wherein the antibody comprises
(a) a heavy chain variable region having an amino acid sequence with at least 85% identical to SEQ ID NO: 190, and a light chain variable region having an amino acid sequence with at least 85% identical to SEQ ID NO: 194; or
(b) a heavy chain variable region having an amino acid sequence with at least 85% identical to SEQ ID NO: 190, and a light chain variable region having an amino acid sequence with at least 85% identical to SEQ ID NO: 195; or
(c) the antibody comprises a heavy chain variable region having an amino acid sequence with at least 85% identical to SEQ ID NO: 190, and a light chain variable region having an amino acid sequence with at least 85% identical to SEQ ID NO: 196; or
(d) the antibody comprises a heavy chain variable region having an amino acid sequence with at least 85% identical to SEQ ID NO: 190, and a light chain variable region having an amino acid sequence with at least 85% identical to SEQ ID NO: 197; or
(e) a heavy chain variable region having an amino acid sequence with at least 85% identical to SEQ ID NO: 191, and a light chain variable region having an amino acid sequence with at least 85% identical to SEQ ID NO: 194; or
(f) a heavy chain variable region having an amino acid sequence with at least 85% identical to SEQ ID NO: 191, and a light chain variable region having an amino acid sequence with at least 85% identical to SEQ ID NO: 195; or
(g) the antibody comprises a heavy chain variable region having an amino acid sequence with at least 85% identical to SEQ ID NO: 191, and a light chain variable region having an amino acid sequence with at least 85% identical to SEQ ID NO: 196; or
(h) the antibody comprises a heavy chain variable region having an amino acid sequence with at least 85% identical to SEQ ID NO: 191, and a light chain variable region having an amino acid sequence with at least 85% identical to SEQ ID NO: 197; or
(i) a heavy chain variable region having an amino acid sequence with at least 85% identical to SEQ ID NO: 192, and a light chain variable region having an amino acid sequence with at least 85% identical to SEQ ID NO: 194; or
(j) a heavy chain variable region having an amino acid sequence with at least 85% identical to SEQ ID NO: 192, and a light chain variable region having an amino acid sequence with at least 85% identical to SEQ ID NO: 195; or
(k) the antibody comprises a heavy chain variable region having an amino acid sequence with at least 85% identical to SEQ ID NO: 192, and a light chain variable region having an amino acid sequence with at least 85% identical to SEQ ID NO: 196; or
(l) the antibody comprises a heavy chain variable region having an amino acid sequence with at least 85% identical to SEQ ID NO: 192, and a light chain variable region having an amino acid sequence with at least 85% identical to SEQ ID NO: 197; or
(m) a heavy chain variable region having an amino acid sequence with at least 85% identical to SEQ ID NO: 193, and a light chain variable region having an amino acid sequence with at least 85% identical to SEQ ID NO: 194; or
(n) a heavy chain variable region having an amino acid sequence with at least 85% identical to SEQ ID NO: 193, and a light chain variable region having an amino acid sequence with at least 85% identical to SEQ ID NO: 195; or
(o) the antibody comprises a heavy chain variable region having an amino acid sequence with at least 85% identical to SEQ ID NO: 193, and a light chain variable region having an amino acid sequence with at least 85% identical to SEQ ID NO: 196; or
(p) the antibody comprises a heavy chain variable region having an amino acid sequence with at least 85% identical to SEQ ID NO: 193, and a light chain variable region having an amino acid sequence with at least 85% identical to SEQ ID NO: 197.

28. The humanized antibody or an antigen-binding fragment thereof of any one of claims 15-27, wherein the antibody comprises at least one of a heavy chain constant region and a light chain constant region, and at least a part of at least one of the heavy chain constant region and the light chain constant region is derived from at least one of a human antibody or a mutant thereof;
preferably, the heavy chain constant region and light chain constant region of the antibody are both derived from human IgG antibodies or mutants thereof;
more preferably, the heavy chain constant region of the antibody is derived from a human IgG1 or IgG4 antibody or a mutant thereof;
more preferably, the light chain constant region of the antibody is derived from a human IgGκ or IgGλ constant region;
more preferably, the full-length sequence of the heavy chain constant region is shown in SEQ ID NO: 117, the full-length sequence of the light chain constant region is shown in SEQ ID NO: 118.

29. An isolated nucleic acid molecule, wherein the nucleic acid molecule comprises a nucleotide sequence encoding the antibody or antigen-binding fragment thereof of any one of claims 1-28;
preferably, the nucleic acid molecule is DNA.

30. An expression vector, wherein the expression vector carries the nucleic acid molecule of claim 29;
preferably, the expression vector is a eukaryotic expression vector.

31. A host cell, wherein the host cell contains the nucleic acid molecule of claim 29 or the expression vector of claim 30;
preferably, the host cell is a eukaryotic host cell;
more preferably, the host cell is a mammalian host cell.

32. A pharmaceutical composition, wherein the pharmaceutical composition comprises at least one of the antibody or antigen-binding fragment thereof of any one of claims 1-28, the nucleic acid molecule of claim 29, the expression vector of claim 30, or the host cell of claim 31;
preferably, the pharmaceutical composition further comprises pharmaceutically acceptable carriers.

33. Use of the antibody or antigen-binding fragment thereof of any one of claims 1-28, the nucleic acid molecule of claim 29, the expression vector of claim 30, the host cell of claim 31, or the pharmaceutical composition of claim 32 in the preparation of a medicament for treating an FGFR2b-related disease.

34. The use of claim 33, wherein the FGFR2b-related disease involves a cancer **characterized by** expression or overexpression of FGFR2b;
preferably, the cancer is ovarian cancer, endometrial cancer, breast cancer, lung cancer, bladder cancer, colon cancer, prostate cancer, cervical cancer, colorectal cancer, pancreatic cancer, gastric cancer, esophageal cancer, liver cancer, renal cell carcinoma, head and neck cancer, mesothelioma, melanoma, sarcoma and brain tumor.

35. A method for treating an FGFR2b-related disease in a subject, wherein the method comprises administering to the subject in need thereof a therapeutically effective amount of the antibody or antigen-binding fragment thereof of any one of claims 1-28, the nucleic acid molecule of claim 29, the expression vector of claim 30, the host cell of claim 31, or the pharmaceutical composition of claim 32.

36. The method of claim 35, wherein the FGFR2b-related disease involves a cancer **characterized by** expression or overexpression of FGFR2b;
preferably, the cancer is ovarian cancer, endometrial cancer, breast cancer, lung cancer, bladder cancer, colon cancer, prostate cancer, cervical cancer, colorectal cancer, pancreatic cancer, gastric cancer, esophageal cancer, liver cancer, renal cell carcinoma, head and neck cancer, mesothelioma, melanoma, sarcoma and brain tumor.

37. The method of claim 35, wherein the administration is oral, nasal, intravenous, subcutaneous, sublingual or intramuscular administration.

38. A bispecific or multispecific antibody, wherein the bispecific or multispecific antibody comprises the antibody or antigen-binding fragment thereof of any one of claims 1-28.

39. An antibody-drug conjugate, wherein the antibody-drug conjugate comprises the antibody or antigen-binding fragment thereof of any one of claims 1-28.

40. A kit for detecting FGFR2b, wherein the kit comprises the antibody or antigen-binding fragment thereof of any one of claims 1-28.
